(19) Europäisches Patentamt
European Patent Office
Office européen des brevets

(11) EP 3 805 407 A1

(12) EUROPEAN PATENT APPLICATION
published in accordance with Art. 153(4) EPC

(43) Date of publication:
**14.04.2021 Bulletin 2021/15**

(21) Application number: **19806410.7**

(22) Date of filing: **24.05.2019**

(51) Int Cl.:
***C12Q 1/6851*** *(2018.01)* ***C12Q 1/6813*** *(2018.01)*
***C12N 15/09*** *(2006.01)* ***C12Q 1/689*** *(2018.01)*

(86) International application number:
**PCT/JP2019/020610**

(87) International publication number:
**WO 2019/225732 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.05.2018 JP 2018100475**
**18.02.2019 JP 2019026519**

(71) Applicant: **Mitsubishi Chemical Corporation**
**Chiyoda-ku**
**Tokyo 100-8251 (JP)**

(72) Inventors:
- **MOMOSE, Kazuki**
**Tokyo 100-8251 (JP)**
- **HARA, Ai**
**Tokyo 100-8251 (JP)**
- **TOGAWA, Naoyuki**
**Tokyo 100-8251 (JP)**
- **SATOU, Eiji**
**Tokyo 100-8251 (JP)**

(74) Representative: **Hoffmann Eitle**
**Patent- und Rechtsanwälte PartmbB**
**Arabellastraße 30**
**81925 München (DE)**

# (54) METHOD FOR QUANTIFYING TARGET NUCLEIC ACIDS

(57) The present invention has an object to provide a simple means capable of simultaneously determining absolute amounts of two or more types of target nucleic acids in a sample.

The present inventors provide a method for determining absolute amounts of two or more types of target nucleic acids in a sample,
the method comprising the steps of: (a) mixing a sample with a known amount of a control nucleic acid; (b) co-amplifying all target nucleic acids and a specific nucleic acid group to be collectively amplified with the target nucleic acids in the sample and the control nucleic acid; (c) determining a total amount of all target nucleic acids and the specific nucleic acid group in the sample based on an indicator of a total amount of the amplified all target nucleic acids and the specific nucleic acid group and an indicator of an amount of the amplified control nucleic acid; and (d) calculating an absolute amount of each target nucleic acid in the sample based on an occupancy of each target nucleic acid in the total of all target nucleic acids and the specific nucleic acid group which is calculated from the indicator of the total amount of the amplified all target nucleic acids and the specific nucleic acid group and an indicator of an amount of the amplified target nucleic acid.

EP 3 805 407 A1

SEQUENCE LISTING

<110> Mitsubishi Chemical Corporation

<120> Method for quantitating target nucleic acids

<130> G2063WO

<150> JP 2018-100475
<151> 2018-05-25

<150> JP 2019-026519
<151> 2019-02-18

<160> 199

<170> PatentIn version 3.5

<210> 1
<211> 30
<212> DNA
<213> Artificial

<220>
<223> Synthetic DNA

<400> 1
ctattcgacc agcgatatca ctacgtaggc 30

<210> 2
<211> 23
<212> DNA
<213> Artificial

<220>
<223> Synthetic DNA

<400> 2
cgtattaccg cggctgctgg cac 23

<210> 3
<211> 27
<212> DNA
<213> Artificial

<220>
<223> Synthetic DNA

<400> 3
gtcaatttgg catgctatta acacacc 27

<210> 4
<211> 27
<212> DNA
<213> Artificial

<220>
<223> Synthetic DNA

<400> 4

gtcaagttgg catgctatta acacacc 27

<210> 5
<211> 20
<212> DNA
<213> Artificial

<220>
<223> Synthetic DNA

<400> 5
gtcataattc tttcccaaga 20

<210> 6
<211> 20
<212> DNA
<213> Artificial

<220>
<223> Synthetic DNA

<400> 6
tacacgtaca ccttattctt 20

<210> 7
<211> 20
<212> DNA
<213> Artificial

<220>
<223> Synthetic DNA

<400> 7
tacattccga aaaacgtcat 20

<210> 8
<211> 20
<212> DNA
<213> Artificial

<220>
<223> Synthetic DNA

<400> 8
ttcaatgcaa tactcgtatc 20

<210> 9
<211> 22
<212> DNA
<213> Artificial

<220>
<223> Synthetic DNA

<400> 9
cgaagggtaa atgcaaaggg gc 22

<210> 10
<211> 22
<212> DNA
<213> Artificial

<220>
<223> Synthetic DNA

<400> 10
cgaagggtaa atgcaaaaag gc 22

<210> 11
<211> 20
<212> DNA
<213> Artificial

<220>
<223> Synthetic DNA

<400> 11
ctttattccc acataaaagc 20

<210> 12
<211> 18
<212> DNA
<213> Artificial

<220>
<223> Synthetic DNA

<400> 12
ttcgcattag gcacgttc 18

<210> 13
<211> 18
<212> DNA
<213> Artificial

<220>
<223> Synthetic DNA

<400> 13
aagtaccgtc actgtgtg 18

<210> 14
<211> 19
<212> DNA
<213> Artificial

<220>
<223> Synthetic DNA

<400> 14
cacccgttct tctcttaca 19

<210> 15
<211> 21
<212> DNA

<213> Artificial

<220>
<223> Synthetic DNA

<400> 15
acagtatgaa ctttccattc t 21

<210> 16
<211> 25
<212> DNA
<213> Artificial

<220>
<223> Synthetic DNA

<400> 16
cacgtatctc attttattcc cctgt 25

<210> 17
<211> 25
<212> DNA
<213> Artificial

<220>
<223> Synthetic DNA

<400> 17
cctcttcttc ttattcttca tctgc 25

<210> 18
<211> 17
<212> DNA
<213> Artificial

<220>
<223> Synthetic DNA

<400> 18
tccttctaac tgttcgc 17

<210> 19
<211> 17
<212> DNA
<213> Artificial

<220>
<223> Synthetic DNA

<400> 19
aagtcagccc gtaccca 17

<210> 20
<211> 17
<212> DNA
<213> Artificial

<220>

<223> Synthetic DNA

<400> 20
ccacccacaa ggagcag 17

<210> 21
<211> 20
<212> DNA
<213> Artificial

<220>
<223> Synthetic DNA

<400> 21
gtcaatttgg catgctatta 20

<210> 22
<211> 18
<212> DNA
<213> Artificial

<220>
<223> Synthetic DNA

<400> 22
cccaagcagt tctatggt 18

<210> 23
<211> 18
<212> DNA
<213> Artificial

<220>
<223> Synthetic DNA

<400> 23
gccttcgcaa taggtatt 18

<210> 24
<211> 20
<212> DNA
<213> Artificial

<220>
<223> Synthetic DNA

<400> 24
caatgggtat tcttcttgat 20

<210> 25
<211> 19
<212> DNA
<213> Artificial

<220>
<223> Synthetic DNA

<400> 25

caaccattca agaccaaca 19

<210> 26
<211> 19
<212> DNA
<213> Artificial

<220>
<223> Synthetic DNA

<400> 26
tcaaaggcag ttgcttagt 19

<210> 27
<211> 19
<212> DNA
<213> Artificial

<220>
<223> Synthetic DNA

<400> 27
ctctagctat ccagttcag 19

<210> 28
<211> 20
<212> DNA
<213> Artificial

<220>
<223> Synthetic DNA

<400> 28
tttctttctt cccaactgaa 20

<210> 29
<211> 19
<212> DNA
<213> Artificial

<220>
<223> Synthetic DNA

<400> 29
ccagtactct agttacaca 19

<210> 30
<211> 20
<212> DNA
<213> Artificial

<220>
<223> Synthetic DNA

<400> 30
tagttataca gtttccaacg 20

<210> 31
<211> 19
<212> DNA
<213> Artificial

<220>
<223> Synthetic DNA

<400> 31
tatgcagttt ccaacgcaa 19

<210> 32
<211> 17
<212> DNA
<213> Artificial

<220>
<223> Synthetic DNA

<400> 32
gccgtgactt gctggtt 17

<210> 33
<211> 17
<212> DNA
<213> Artificial

<220>
<223> Synthetic DNA

<400> 33
ctgtcctctt ctgcact 17

<210> 34
<211> 19
<212> DNA
<213> Artificial

<220>
<223> Synthetic DNA

<400> 34
gggtaaatgc aaaaaggca 19

<210> 35
<211> 18
<212> DNA
<213> Artificial

<220>
<223> Synthetic DNA

<400> 35
ttagccgtcc ctttctgg 18

<210> 36
<211> 19
<212> DNA

<213> Artificial

<220>
<223> Synthetic DNA

<400> 36
tcccctcttg cactcaagt 19

<210> 37
<211> 18
<212> DNA
<213> Artificial

<220>
<223> Synthetic DNA

<400> 37
tctcccctct tgcactca 18

<210> 38
<211> 18
<212> DNA
<213> Artificial

<220>
<223> Synthetic DNA

<400> 38
cacacgttct tgacttac 18

<210> 39
<211> 18
<212> DNA
<213> Artificial

<220>
<223> Synthetic DNA

<400> 39
ccgtcactgt gtaagctt 18

<210> 40
<211> 20
<212> DNA
<213> Artificial

<220>
<223> Synthetic DNA

<400> 40
cacgtatctc attttattcc 20

<210> 41
<211> 21
<212> DNA
<213> Artificial

<220>

<223> Synthetic DNA

<400> 41
cctcttcttc ttattcttca t 21

<210> 42
<211> 18
<212> DNA
<213> Artificial

<220>
<223> Synthetic DNA

<400> 42
tattcgcaag aaggcctt 18

<210> 43
<211> 20
<212> DNA
<213> Artificial

<220>
<223> Synthetic DNA

<400> 43
aaaaagcagt gccttgttcc 20

<210> 44
<211> 20
<212> DNA
<213> Artificial

<220>
<223> Synthetic DNA

<400> 44
gcgcttcata acccggctac 20

<210> 45
<211> 17
<212> DNA
<213> Artificial

<220>
<223> Synthetic DNA

<400> 45
aacccaccca caaacga 17

<210> 46
<211> 21
<212> DNA
<213> Artificial

<220>
<223> Synthetic DNA

<400> 46

ttcccaacta aaagcagttt a 21

<210> 47
<211> 22
<212> DNA
<213> Artificial

<220>
<223> Synthetic DNA

<400> 47
tcttaacaaa ggtaccgtca cc 22

<210> 48
<211> 22
<212> DNA
<213> Artificial

<220>
<223> Synthetic DNA

<400> 48
cctacgctta cttaaccacc ta 22

<210> 49
<211> 20
<212> DNA
<213> Artificial

<220>
<223> Synthetic DNA

<400> 49
ccctaggaca gaggcttaca 20

<210> 50
<211> 20
<212> DNA
<213> Artificial

<220>
<223> Synthetic DNA

<400> 50
aaaccctgcg cttaaggtgc 20

<210> 51
<211> 22
<212> DNA
<213> Artificial

<220>
<223> Synthetic DNA

<400> 51
cccctactac agagttttac ga 22

<210> 52
<211> 20
<212> DNA
<213> Artificial

<220>
<223> Synthetic DNA

<400> 52
ccgtctctac tgtatatagt 20

<210> 53
<211> 20
<212> DNA
<213> Artificial

<220>
<223> Synthetic DNA

<400> 53
gtcaaggcgc taacagttac 20

<210> 54
<211> 20
<212> DNA
<213> Artificial

<220>
<223> Synthetic DNA

<400> 54
agttaacgtc aatcacctag 20

<210> 55
<211> 20
<212> DNA
<213> Artificial

<220>
<223> Synthetic DNA

<400> 55
gcgggatagt cagtcaggtg 20

<210> 56
<211> 19
<212> DNA
<213> Artificial

<220>
<223> Synthetic DNA

<400> 56
cagtttccga tgcagttcc 19

<210> 57
<211> 20
<212> DNA

<213> Artificial

<220>
<223> Synthetic DNA

<400> 57
cgtgcagttc cgtccacctc 20

<210> 58
<211> 20
<212> DNA
<213> Artificial

<220>
<223> Synthetic DNA

<400> 58
taaccacaag attattcgtc 20

<210> 59
<211> 21
<212> DNA
<213> Artificial

<220>
<223> Synthetic DNA

<400> 59
agctgtcgat attagcaaca g 21

<210> 60
<211> 20
<212> DNA
<213> Artificial

<220>
<223> Synthetic DNA

<400> 60
gtgcttaatg aggttaagcc 20

<210> 61
<211> 20
<212> DNA
<213> Artificial

<220>
<223> Synthetic DNA

<400> 61
accaccgact tgaaggacca 20

<210> 62
<211> 22
<212> DNA
<213> Artificial

<220>

<223> Synthetic DNA

<400> 62
ggtacattca ctatggtaca cg 22

<210> 63
<211> 22
<212> DNA
<213> Artificial

<220>
<223> Synthetic DNA

<400> 63
tacatgcatc tcagctacac gt 22

<210> 64
<211> 20
<212> DNA
<213> Artificial

<220>
<223> Synthetic DNA

<400> 64
acacgtgact cttgttattc 20

<210> 65
<211> 20
<212> DNA
<213> Artificial

<220>
<223> Synthetic DNA

<400> 65
agtcagacgt tgggcgccta 20

<210> 66
<211> 20
<212> DNA
<213> Artificial

<220>
<223> Synthetic DNA

<400> 66
cacgtgactg actttatccc 20

<210> 67
<211> 20
<212> DNA
<213> Artificial

<220>
<223> Synthetic DNA

<400> 67

cctactttca gcgcactcaa 20

<210> 68
<211> 20
<212> DNA
<213> Artificial

<220>
<223> Synthetic DNA

<400> 68
aatagggaca cgtccctaac 20

<210> 69
<211> 22
<212> DNA
<213> Artificial

<220>
<223> Synthetic DNA

<400> 69
cacgtgcatc aaattattct cg 22

<210> 70
<211> 20
<212> DNA
<213> Artificial

<220>
<223> Synthetic DNA

<400> 70
acgtgggctc ttttatcccc 20

<210> 71
<211> 20
<212> DNA
<213> Artificial

<220>
<223> Synthetic DNA

<400> 71
acacgtgatt gactttatcc 20

<210> 72
<211> 20
<212> DNA
<213> Artificial

<220>
<223> Synthetic DNA

<400> 72
acccactgca aaaccagggt 20

<210> 73
<211> 20
<212> DNA
<213> Artificial

<220>
<223> Synthetic DNA

<400> 73
tctcttcttc cctgctaaca 20

<210> 74
<211> 20
<212> DNA
<213> Artificial

<220>
<223> Synthetic DNA

<400> 74
actattcgca ctaggcacgt 20

<210> 75
<211> 22
<212> DNA
<213> Artificial

<220>
<223> Synthetic DNA

<400> 75
gtaccgtcac ccaaactcaa ta 22

<210> 76
<211> 20
<212> DNA
<213> Artificial

<220>
<223> Synthetic DNA

<400> 76
ccaacaattt aaccacttac 20

<210> 77
<211> 20
<212> DNA
<213> Artificial

<220>
<223> Synthetic DNA

<400> 77
cgtcattcgt cttctgccaa 20

<210> 78
<211> 18
<212> DNA

<213>  Artificial

<220>
<223>  Synthetic DNA

<400>  78
ctggtaagtt accgtcac                                                      18

<210>  79
<211>  20
<212>  DNA
<213>  Artificial

<220>
<223>  Synthetic DNA

<400>  79
cacactcgtt cttgacttac                                                    20

<210>  80
<211>  20
<212>  DNA
<213>  Artificial

<220>
<223>  Synthetic DNA

<400>  80
tacacacgtt cttcccctac                                                    20

<210>  81
<211>  21
<212>  DNA
<213>  Artificial

<220>
<223>  Synthetic DNA

<400>  81
gtcgattacc gtcatcagat g                                                  21

<210>  82
<211>  20
<212>  DNA
<213>  Artificial

<220>
<223>  Synthetic DNA

<400>  82
ttcctccaaa acttattcct                                                    20

<210>  83
<211>  21
<212>  DNA
<213>  Artificial

<220>

<223> Synthetic DNA

<400> 83
cgtcaaatcc tcgcactatt c 21

<210> 84
<211> 22
<212> DNA
<213> Artificial

<220>
<223> Synthetic DNA

<400> 84
accgtcaatt cctctaacta tt 22

<210> 85
<211> 20
<212> DNA
<213> Artificial

<220>
<223> Synthetic DNA

<400> 85
cagtttcgcg aggtattaac 20

<210> 86
<211> 20
<212> DNA
<213> Artificial

<220>
<223> Synthetic DNA

<400> 86
caccagacga agtccgtgct 20

<210> 87
<211> 22
<212> DNA
<213> Artificial

<220>
<223> Synthetic DNA

<400> 87
ctaggtccgg tagcaagcta cc 22

<210> 88
<211> 20
<212> DNA
<213> Artificial

<220>
<223> Synthetic DNA

<400> 88

taccttcccc cgctcgactt 20

<210> 89
<211> 23
<212> DNA
<213> Artificial

<220>
<223> Synthetic DNA

<400> 89
atcagtagca agctacctct ctc 23

<210> 90
<211> 22
<212> DNA
<213> Artificial

<220>
<223> Synthetic DNA

<400> 90
cgctaagatc agtagcaagc ta 22

<210> 91
<211> 21
<212> DNA
<213> Artificial

<220>
<223> Synthetic DNA

<400> 91
gtattaatct cggtagcctc c 21

<210> 92
<211> 24
<212> DNA
<213> Artificial

<220>
<223> Synthetic DNA

<400> 92
ctaggttagg tagcaagcta catt 24

<210> 93
<211> 22
<212> DNA
<213> Artificial

<220>
<223> Synthetic DNA

<400> 93
caccaaaaca ccacaaaagt ga 22

<210> 94
<211> 20
<212> DNA
<213> Artificial

<220>
<223> Synthetic DNA

<400> 94
actaacccac cccgcaaaaa 20

<210> 95
<211> 20
<212> DNA
<213> Artificial

<220>
<223> Synthetic DNA

<400> 95
tccagtatta gcacccattt 20

<210> 96
<211> 22
<212> DNA
<213> Artificial

<220>
<223> Synthetic DNA

<400> 96
atgcgaagat cagtgaatat cc 22

<210> 97
<211> 20
<212> DNA
<213> Artificial

<220>
<223> Synthetic DNA

<400> 97
aagttggagc atcatcgttc 20

<210> 98
<211> 20
<212> DNA
<213> Artificial

<220>
<223> Synthetic DNA

<400> 98
gccaatgagt gaatatccag 20

<210> 99
<211> 21
<212> DNA

<213> Artificial

<220>
<223> Synthetic DNA

<400> 99
ggaagtctcg aagtattcgg t 21

<210> 100
<211> 22
<212> DNA
<213> Artificial

<220>
<223> Synthetic DNA

<400> 100
gagctttctc gagtcttcca tg 22

<210> 101
<211> 22
<212> DNA
<213> Artificial

<220>
<223> Synthetic DNA

<400> 101
cggaatcatt atgctatcgg gt 22

<210> 102
<211> 20
<212> DNA
<213> Artificial

<220>
<223> Synthetic DNA

<400> 102
caagctttct tcctgatgcc 20

<210> 103
<211> 22
<212> DNA
<213> Artificial

<220>
<223> Synthetic DNA

<400> 103
tagagccaag gatttctttc cg 22

<210> 104
<211> 20
<212> DNA
<213> Artificial

<220>

<223> Synthetic DNA

<400> 104
actacaggac atgttccgat 20

<210> 105
<211> 21
<212> DNA
<213> Artificial

<220>
<223> Synthetic DNA

<400> 105
ctctcaagat cccgaaagat c 21

<210> 106
<211> 20
<212> DNA
<213> Artificial

<220>
<223> Synthetic DNA

<400> 106
gtacgccgct ctcaagatcc 20

<210> 107
<211> 20
<212> DNA
<213> Artificial

<220>
<223> Synthetic DNA

<400> 107
taaccacaac gccttcctcc 20

<210> 108
<211> 20
<212> DNA
<213> Artificial

<220>
<223> Synthetic DNA

<400> 108
gtcacccaag gagcaagctc 20

<210> 109
<211> 22
<212> DNA
<213> Artificial

<220>
<223> Synthetic DNA

<400> 109

ctgacactaa acagtggtcc ta 22

<210> 110
<211> 20
<212> DNA
<213> Artificial

<220>
<223> Synthetic DNA

<400> 110
tcgagtaccc gcagcaagct 20

<210> 111
<211> 20
<212> DNA
<213> Artificial

<220>
<223> Synthetic DNA

<400> 111
cggacagtag tgcaagcact 20

<210> 112
<211> 20
<212> DNA
<213> Artificial

<220>
<223> Synthetic DNA

<400> 112
aataacgcga ggtcttgcga 20

<210> 113
<211> 20
<212> DNA
<213> Artificial

<220>
<223> Synthetic DNA

<400> 113
ttaaccttaa cgccttcctc 20

<210> 114
<211> 22
<212> DNA
<213> Artificial

<220>
<223> Synthetic DNA

<400> 114
cctcgctgaa agtactttac aa 22

<210> 115
<211> 20
<212> DNA
<213> Artificial

<220>
<223> Synthetic DNA

<400> 115
cggttattaa ccacaacacc 20

<210> 116
<211> 20
<212> DNA
<213> Artificial

<220>
<223> Synthetic DNA

<400> 116
agctctctgt gctaccgttc 20

<210> 117
<211> 20
<212> DNA
<213> Artificial

<220>
<223> Synthetic DNA

<400> 117
tctctgtgct accgttcgac 20

<210> 118
<211> 20
<212> DNA
<213> Artificial

<220>
<223> Synthetic DNA

<400> 118
tctttggtct tgcgacgtta 20

<210> 119
<211> 20
<212> DNA
<213> Artificial

<220>
<223> Synthetic DNA

<400> 119
ctctcaggtg cggctatgca 20

<210> 120
<211> 20
<212> DNA

<213>  Artificial

<220>
<223>  Synthetic DNA

<400>  120
aggtcccccct ctttgtgctt                                                    20

<210>  121
<211>  20
<212>  DNA
<213>  Artificial

<220>
<223>  Synthetic DNA

<400>  121
cgtcaatgag caaaggtatt                                                    20

<210>  122
<211>  20
<212>  DNA
<213>  Artificial

<220>
<223>  Synthetic DNA

<400>  122
gacatacgta attaccgcgg                                                    20

<210>  123
<211>  20
<212>  DNA
<213>  Artificial

<220>
<223>  Synthetic DNA

<400>  123
gcttgcgaca tacgtaatta                                                    20

<210>  124
<211>  20
<212>  DNA
<213>  Artificial

<220>
<223>  Synthetic DNA

<400>  124
atgttgtccc ggtctaagag                                                    20

<210>  125
<211>  20
<212>  DNA
<213>  Artificial

<220>

<223> Synthetic DNA

<400> 125
cttcaaggca tattcctacg 20

<210> 126
<211> 20
<212> DNA
<213> Artificial

<220>
<223> Synthetic DNA

<400> 126
ttacaaccct caggccttct 20

<210> 127
<211> 21
<212> DNA
<213> Artificial

<220>
<223> Synthetic DNA

<400> 127
ttacaaccct caggccttct t 21

<210> 128
<211> 22
<212> DNA
<213> Artificial

<220>
<223> Synthetic DNA

<400> 128
cctcttcaag gcatattcct ac 22

<210> 129
<211> 20
<212> DNA
<213> Artificial

<220>
<223> Synthetic DNA

<400> 129
cagtattatc tgaccgtccc 20

<210> 130
<211> 22
<212> DNA
<213> Artificial

<220>
<223> Synthetic DNA

<400> 130

aggctaatct taaagcgcca gg 22

<210> 131
<211> 21
<212> DNA
<213> Artificial

<220>
<223> Synthetic DNA

<400> 131
cagctttcgt cctcagacat t 21

<210> 132
<211> 20
<212> DNA
<213> Artificial

<220>
<223> Synthetic DNA

<400> 132
gcttcctaac ttcgttcgac 20

<210> 133
<211> 20
<212> DNA
<213> Artificial

<220>
<223> Synthetic DNA

<400> 133
gtcagggctt atgggtatta 20

<210> 134
<211> 21
<212> DNA
<213> Artificial

<220>
<223> Synthetic DNA

<400> 134
acatctgact caatcaaccg c 21

<210> 135
<211> 22
<212> DNA
<213> Artificial

<220>
<223> Synthetic DNA

<400> 135
tattaacctt gacaccttcc tc 22

<210> 136
<211> 20
<212> DNA
<213> Artificial

<220>
<223> Synthetic DNA

<400> 136
cagaagacat gcgtcttgag 20

<210> 137
<211> 22
<212> DNA
<213> Artificial

<220>
<223> Synthetic DNA

<400> 137
gagtacctcc gaagaggcct tt 22

<210> 138
<211> 20
<212> DNA
<213> Artificial

<220>
<223> Synthetic DNA

<400> 138
attgcccacg tgttactcac 20

<210> 139
<211> 20
<212> DNA
<213> Artificial

<220>
<223> Synthetic DNA

<400> 139
cttcttctcc acctaccgtc 20

<210> 140
<211> 20
<212> DNA
<213> Artificial

<220>
<223> Synthetic DNA

<400> 140
ccactcgagt gtctccgaag 20

<210> 141
<211> 20
<212> DNA

<213>  Artificial

<220>
<223>  Synthetic DNA

<400>  141
aggcttatcc cggtgtgcag                                                    20

<210>  142
<211>  21
<212>  DNA
<213>  Artificial

<220>
<223>  Synthetic DNA

<400>  142
acccaacagc atgcactgaa a                                                  21

<210>  143
<211>  20
<212>  DNA
<213>  Artificial

<220>
<223>  Synthetic DNA

<400>  143
gtcagttact tcccagagac                                                    20

<210>  144
<211>  22
<212>  DNA
<213>  Artificial

<220>
<223>  Synthetic DNA

<400>  144
ccgcttggtt ttgctcagaa tt                                                 22

<210>  145
<211>  22
<212>  DNA
<213>  Artificial

<220>
<223>  Synthetic DNA

<400>  145
actcaatact gagcaaaccc tc                                                 22

<210>  146
<211>  22
<212>  DNA
<213>  Artificial

<220>

<223>  Synthetic DNA

<400>  146
gaggacctcg ttggaataca ta                                                22

<210>  147
<211>  22
<212>  DNA
<213>  Artificial

<220>
<223>  Synthetic DNA

<400>  147
ctgcttgaag gtaggttgga ta                                                22

<210>  148
<211>  21
<212>  DNA
<213>  Artificial

<220>
<223>  Synthetic DNA

<400>  148
ccctaacttt atccccatac a                                                 21

<210>  149
<211>  20
<212>  DNA
<213>  Artificial

<220>
<223>  Synthetic DNA

<400>  149
gtatggtacc tgcaaatagg                                                   20

<210>  150
<211>  21
<212>  DNA
<213>  Artificial

<220>
<223>  Synthetic DNA

<400>  150
ctttaatcgt cgttggatgc c                                                 21

<210>  151
<211>  20
<212>  DNA
<213>  Artificial

<220>
<223>  Synthetic DNA

<400>  151

agacgatgcc atgaggtatt 20

<210> 152
<211> 20
<212> DNA
<213> Artificial

<220>
<223> Synthetic DNA

<400> 152
tctttaccca ttaccgtcac 20

<210> 153
<211> 20
<212> DNA
<213> Artificial

<220>
<223> Synthetic DNA

<400> 153
cccaagatta cacttccgac 20

<210> 154
<211> 20
<212> DNA
<213> Artificial

<220>
<223> Synthetic DNA

<400> 154
gattaacgcc tgcaccctcc 20

<210> 155
<211> 20
<212> DNA
<213> Artificial

<220>
<223> Synthetic DNA

<400> 155
cgcctgcacc ctccgtatta 20

<210> 156
<211> 20
<212> DNA
<213> Artificial

<220>
<223> Synthetic DNA

<400> 156
taccgctcga ttgcatgtgt 20

<210> 157
<211> 20
<212> DNA
<213> Artificial

<220>
<223> Synthetic DNA

<400> 157
aactttatca ccttcctccc 20

<210> 158
<211> 20
<212> DNA
<213> Artificial

<220>
<223> Synthetic DNA

<400> 158
gcggttcaat atattatccg 20

<210> 159
<211> 20
<212> DNA
<213> Artificial

<220>
<223> Synthetic DNA

<400> 159
ataagtgaca gcaaaaccgt 20

<210> 160
<211> 20
<212> DNA
<213> Artificial

<220>
<223> Synthetic DNA

<400> 160
ttagccagct ggatttcttt 20

<210> 161
<211> 20
<212> DNA
<213> Artificial

<220>
<223> Synthetic DNA

<400> 161
cgacagagta gattccgatg 20

<210> 162
<211> 24
<212> DNA

<213> Artificial

<220>
<223> Synthetic DNA

<400> 162
agtctagtgt aaacaccaaa cctc 24

<210> 163
<211> 21
<212> DNA
<213> Artificial

<220>
<223> Synthetic DNA

<400> 163
actcctacca acgttcttct c 21

<210> 164
<211> 21
<212> DNA
<213> Artificial

<220>
<223> Synthetic DNA

<400> 164
catctactag cgatgcaatt g 21

<210> 165
<211> 20
<212> DNA
<213> Artificial

<220>
<223> Synthetic DNA

<400> 165
ttaagtaccg tcacagcatg 20

<210> 166
<211> 20
<212> DNA
<213> Artificial

<220>
<223> Synthetic DNA

<400> 166
acctttcatc cagtctcgat 20

<210> 167
<211> 20
<212> DNA
<213> Artificial

<220>

<223> Synthetic DNA

<400> 167
cgtcaaatcc tcgcactatt 20

<210> 168
<211> 20
<212> DNA
<213> Artificial

<220>
<223> Synthetic DNA

<400> 168
cacacctgac tgactatccc 20

<210> 169
<211> 21
<212> DNA
<213> Artificial

<220>
<223> Synthetic DNA

<400> 169
ccactaatca gcactctagc a 21

<210> 170
<211> 20
<212> DNA
<213> Artificial

<220>
<223> Synthetic DNA

<400> 170
aaactaagag gcacatgacc 20

<210> 171
<211> 21
<212> DNA
<213> Artificial

<220>
<223> Synthetic DNA

<400> 171
gttttaaatg ctgacttggg g 21

<210> 172
<211> 20
<212> DNA
<213> Artificial

<220>
<223> Synthetic DNA

<400> 172

gcataaactc ttcctcaacc 20

<210> 173
<211> 20
<212> DNA
<213> Artificial

<220>
<223> Synthetic DNA

<400> 173
aaccgttatc cccaagttga 20

<210> 174
<211> 20
<212> DNA
<213> Artificial

<220>
<223> Synthetic DNA

<400> 174
tctcagtccc gcactttcat 20

<210> 175
<211> 20
<212> DNA
<213> Artificial

<220>
<223> Synthetic DNA

<400> 175
tattaacctc atcgccttcc 20

<210> 176
<211> 20
<212> DNA
<213> Artificial

<220>
<223> Synthetic DNA

<400> 176
gtaacgtcaa tcgatgaggt 20

<210> 177
<211> 21
<212> DNA
<213> Artificial

<220>
<223> Synthetic DNA

<400> 177
aaatggtaca gtcaaactct a 21

<210> 178
<211> 20
<212> DNA
<213> Artificial

<220>
<223> Synthetic DNA

<400> 178
catgcgaacc aaagttctta 20

<210> 179
<211> 20
<212> DNA
<213> Artificial

<220>
<223> Synthetic DNA

<400> 179
ccggacgtta tcccccacta 20

<210> 180
<211> 20
<212> DNA
<213> Artificial

<220>
<223> Synthetic DNA

<400> 180
gctgaatcca ggagcaagct 20

<210> 181
<211> 21
<212> DNA
<213> Artificial

<220>
<223> Synthetic DNA

<400> 181
gcgagtaacg tcaattgatg a 21

<210> 182
<211> 20
<212> DNA
<213> Artificial

<220>
<223> Synthetic DNA

<400> 182
cccctctacg agactctagc 20

<210> 183
<211> 22
<212> DNA

<213> Artificial

<220>
<223> Synthetic DNA

<400> 183
gtattaagct caccaccttc ct 22

<210> 184
<211> 20
<212> DNA
<213> Artificial

<220>
<223> Synthetic DNA

<400> 184
ctaacatcag agaagcaagc 20

<210> 185
<211> 20
<212> DNA
<213> Artificial

<220>
<223> Synthetic DNA

<400> 185
aagaccgtct ttcacttttg 20

<210> 186
<211> 20
<212> DNA
<213> Artificial

<220>
<223> Synthetic DNA

<400> 186
cttacacata tgttcttccc 20

<210> 187
<211> 20
<212> DNA
<213> Artificial

<220>
<223> Synthetic DNA

<400> 187
atgcaacatc cactcttatg 20

<210> 188
<211> 20
<212> DNA
<213> Artificial

<220>

<223>  Synthetic DNA

<400>  188
caagtgcacc ttttaagcaa                                                    20

<210>  189
<211>  22
<212>  DNA
<213>  Artificial

<220>
<223>  Synthetic DNA

<400>  189
catgcgttag tctctcttat gc                                                 22

<210>  190
<211>  20
<212>  DNA
<213>  Artificial

<220>
<223>  Synthetic DNA

<400>  190
ccatctcata gtggagcaat                                                    20

<210>  191
<211>  21
<212>  DNA
<213>  Artificial

<220>
<223>  Synthetic DNA

<400>  191
cccaccatca ttcttctcta a                                                  21

<210>  192
<211>  15
<212>  DNA
<213>  Artificial

<220>
<223>  Synthetic DNA

<400>  192
tacgggaggc agcag                                                         15

<210>  193
<211>  19
<212>  DNA
<213>  Artificial

<220>
<223>  Synthetic DNA

<400>  193

crgggtatct aatccygtt 19

<210> 194
<211> 25
<212> DNA
<213> Artificial

<220>
<223> Synthetic DNA

<400> 194
gagaagccta cacaaacgta acgtc 25

<210> 195
<211> 23
<212> DNA
<213> Artificial

<220>
<223> Synthetic DNA

<400> 195
ctctaaagac cgctctatct cgg 23

<210> 196
<211> 458
<212> DNA
<213> Artificial

<220>
<223> Synthetic DNA

<400> 196
gtgagaagcc tacacaaacg taacgtcagg gctaagacaa acgctaacgg tacaccctag 60

atgggagctt gtagctagat cgctaagtcc taccgacatg taggcatact cacgaaggca 120

attccctgaa agcctcgtct tatcccgaac ttggcatctg ctgatacgtc aggttgaacg 180

cgtacattta cctgtcatgc gtgggccttc tccgaatagc ctacgtagtg atatcgctgg 240

tcgaataggc ggattgctca taaatgcaca ttggctaagg cccacggaac acgaatcacg 300

tgagatcact tactattcga cggaactact atacgcaccg ggacatgcaa gtagcgtccc 360

acaagcataa ggaactctat actcgccatc tacgcagcta caggggatac acgtatgagc 420

ggttacgaag taaagccgag atagagcggt ctttagag 458

<210> 197
<211> 18
<212> DNA
<213> Artificial

<220>
<223> Synthetic DNA

<220>

<221> misc_feature
<222> (5)..(5)
<223> n is inosine

<400> 197
taccngggta tctaatcc 18

<210> 198
<211> 458
<212> DNA
<213> Artificial

<220>
<223> Synthetic DNA

<400> 198
gttcctacgg gaggcagcag tagggctaag acaaacgcta acggtacacc ctagatggga 60

gcttgtagct agatcgctaa gtcctaccga catgtaggca tactcacgaa ggcaattccc 120

tgaaagcctc gtcttatccc gaacttggca tctgctgata cgtcaggttg aacgcgtaca 180

tttacctgtc atgcgtgggc cttctccgaa tagcctacgt agtgatatcg ctggtcgaat 240

aggcggattg ctcataaatg cacattggct aaggcccacg gaacacgaat cacgtgagat 300

cacttactat tcgacggaac tactatacgc accgggacat gcaagtagcg tcccacaagc 360

ataaggaact ctatactcgc catctacgca gctacagggg atacacgtat gagcggttac 420

gaagtaaagg gtagcaaaca ggattagata ccctgaaa 458

<210> 199
<211> 463
<212> DNA
<213> Artificial

<220>
<223> Synthetic DNA

<400> 199
gttcctacgg gaggcagcag tagggctaag acaaacgcta acggtacacc ctagatggga 60

gcttgtagct agatcgctaa gtcctaccga catgtaggca tactcacgaa ggcaattccc 120

tgaaagcctc gtcttatccc gaacttggca tctgctgata cgtcaggttg aacgcgtaca 180

tttacctgtc atgcgtgggc cttctccgaa tagcctacgt agtgatatcg ctggtcgaat 240

aggcggattg ctcataaatg cacattggct aaggcccacg gaacacgaat cacgtgagat 300

cacttactat tcgacggaac tactatacgc accgggacat gcaagtagcg tcccacaagc 360

ataaggaact ctatactcgc catctacgca gctacagggg atacacgtat gagcggttac 420

gaagtaaagg gtagcaacag gattagatac cctggtagtc cac 463

## Description

[Technical Field]

The present invention relates to a method for quantifying nucleic acids and an analysis kit utilizing the same.

[Background Art]

There are many fields in need of a simultaneous quantification of multiple types of nucleic acids in a sample and one example is bacterial flora analysis.

In recent years, the relationship between human health and oral bacterial florae and intestinal bacterial florae has become evident thereby demanding techniques for analyzing bacterial florae in a simple manner for the purpose of examinations.

Microscopy and culture method have been traditionally used as tools to evaluate bacterial florae but pose a problem of limiting bacterial species applicable to be discriminated and quantified. Instead of these methods, molecular biological techniques based on the specificity of a nucleic acid sequence has been used in recent years. Quantitative PCR (qPCR), competitive PCR, DNA chips, next generation sequencer (NGS) are known as such a molecular biological technique.

qPCR method commonly used enables highly sensitive quantification but requires cumbersome operations and has a limited number of items applicable to examinations as in competitive PCR. DNA chip is a high-throughput screening but the quantification using this is a relative quantification. NGS requires cumbersome operations and is expensive thereby involving challenges to be used for the purpose of routine diagnosis.

At moment, bacterial flora analysis using a DNA chip is incapable of carrying out absolute quantification. When bacterial flora analysis is carried out using a DNA chip or NGS, a nucleic acid group comprising target nucleic acids needs to be amplified by a technique such as PCR method. The measurement result obtained by the technique represents a relative relationship between a plurality of targets by the amplification.

There is competitive PCR as the technique for determining an absolute amount of a nucleic acid in a simple manner. Competitive PCR is a method in which a control nucleic acid in a known concentration and to be co-amplified with a target is added before amplification for determining an absolute amount before amplification from an indicator of an amount after amplification. However, in this competitive PCR, only one type of a nucleic acid can be an object for quantification and it is difficult to quantify a plural type of nucleic acids simultaneously.

Additionally, a technique for bacterial flora analysis in which a control nucleic acid in a known concentration is added before amplification to study an existing amount thereof is already known (Patent Literature 1, Non Patent Literature 1). For example, in Non Patent Literature 1, a control nucleic acid is added and relative amount ratios among samples are compared after PCR amplification using a DNA chip. In a DNA chip, every probe has different hybridization efficiency thereby failing to compare signals among different probes and thus this method cannot determine an absolute amount.

On the other hand, Patent Literature 1 discloses, for the purpose of absolute quantification by NGS, a method in which a plurality of control nucleic acids are added before amplification and a calibration curve is created using only such a plurality of control nucleic acids thereby to determine an amount of each target nucleic acid. This method has a premise that the amplification efficiency of the control nucleic acids and target nucleic acids is identical and Patent Literature 1 proposes sequences artificially designed so that the amplification efficiency becomes identical. This method is a cumbersome method which uses an expensive NGS and further a plurality of control nucleic acids. Additionally, this method involves an extremely difficult problem in that the amplification efficiency between a target nucleic acid and a plurality of control nucleic acids in a sample must be identical.

As described above, a method capable of simultaneously quantifying absolute amounts of a plural type of target nucleic acids in a simple manner has not been known.

[Citation List]

[Patent Literature]

[Patent Literature 1] Japanese Patent Laid-Open No. 2015-204813

[Non Patent Literature]

[Non Patent Literature 1] Treimo, J. et al. J Appl Microbiol, 100, 985-998, 2006

[Summary of Invention]

[Technical Problem]

Thus, the present invention has an object to provide a simple means capable of simultaneously determining absolute amounts of two or more types of target nucleic acids in a sample.

[Solution to Problem]

The present inventors conducted extensive studies to solve the above problems and consequently found that the step of calculating an absolute quantification of the total of all target nucleic acids and a specific nucleic acid group to be collectively amplified with the target nucleic acids in a sample using a control nucleic acid and a breakdown thereof enables the absolute quantification of two or more types of target nucleic acids respectively in the sample, whereby the present invention has been accomplished.

That is, the present invention is as follows.

[1] A method for determining absolute amounts of two or more types of target nucleic acids in a sample, the method comprising the steps of:

(a) mixing a sample with a known amount of a control nucleic acid;
(b) co-amplifying all target nucleic acids and a specific nucleic acid group to be collectively amplified with the target nucleic acids in the sample and the control nucleic acid;
(c) determining a total amount of all target nucleic acids and the specific nucleic acid group in the sample based on an indicator of a total amount of the amplified all target nucleic acids and the specific nucleic acid group and an indicator of an amount of the amplified control nucleic acid; and
(d) calculating an absolute amount of each target nucleic acid in the sample based on an occupancy of each target nucleic acid in the total of all target nucleic acids and the specific nucleic acid group which is calculated from the indicator of the total amount of the amplified all target nucleic acids and the specific nucleic acid group and an indicator of an amount of each amplified target nucleic acid.

[2] The method according to [1], wherein the control nucleic acid is an artificial sequence consisting of a base sequence required for amplification and a base sequence in which a part or all of the bases are in a random combination.
[3] The method according to [1] or [2], wherein the co-amplification of the total of all target nucleic acids and the specific nucleic acid group in the sample and the control nucleic acid is carried out by a technique selected from the group consisting of Polymerase Chain Reaction (PCR) method, Loop-Mediated Isothermal Amplification (LAMP) method, and in vitro transcription.
[4] The method according to [3], wherein the co-amplification of the total of all target nucleic acids and the specific nucleic acid group in the sample and the control nucleic acid is carried out by PCR method.
[5] The method according to any of [1] to [4], wherein the sample is at least one selected from the group consisting of foods and drinks, biological samples, environmental samples, and samples from industrial process.
[6] The method according to [5], wherein the sample is at least one selected from the group consisting of activated sludges, soils, river waters, seawaters, hot spring waters, drinking waters, processed foods, fermenter cultures, and tissues, cells and body fluids collected from eukaryotes.
[7] The method according to [5], wherein the biological sample is at least one selected from the group consisting of saliva, plaque, gingival crevicular fluid (GCF), feces, and skin-derived samples collected from mammals.
[8] The method according to any of [1] to [7], wherein the target nucleic acid is a bacterial ribosomal RNA (rRNA) gene.
[9] The method according to [8], wherein the target nucleic acids are rRNA genes of at least two species of bacteria selected from the group consisting of bacteria belonging to the *Abiotrophia* genus, *Achromobacter* genus, *Acinetobacter* genus, *Actinomyces* genus, *Aerococcus* genus, *Aggregatibacter* genus, *Alloprevotella* genus, *Alloscardovia* genus, *Anaerococcus* genus, *Anaeroglobus* genus, *Arcanobacterium* genus, *Atopobium* genus, *Bacillus* genus, *Bacteroides* genus, *Bifidobacterium* genus, *Bordetella* genus, *Brevundimonas* genus, *Brucella* genus, *Burkholderia* genus, *Campylobacter* genus, *Candidatus Absconditabacteria* (SRI), *Candidatus Saccharibacteria* (TM7), *Capnocytophaga* genus, *Cardiobacterium* genus, *Catonella* genus, *Chlamydia* genus, *Chlamydophila* genus, *Chryseobacterium* genus, *Citrobacter* genus, *Clostridium* genus, *Collinsella* genus, *Corynebacterium* genus, *Coxiella* genus, *Cronobacter* genus, *Cryptobacterium* genus, *Curvibacter* genus, *Dialister* genus, *Eggerthella* genus, *Eikenella* genus, *Elizabethkingia, Enterobacter* genus, *Enterococcus* genus, *Escherichia* genus, *Eubacterium* genus, *Filifactor* genus, *Finegoldia* genus, *Fusobacterium* genus, *Gardnerella* genus, *Gemella* genus, *Granulicatella* genus, *Hae-*

*mophilus* genus, *Helicobacter* genus, *Kingella* genus, *Klebsiella* genus, *Kocuria* genus, *Lachnoanaerobaculum* genus, *Lactobacillus* genus, *Lactococcus* genus, *Lautropia* genus, *Legionella* genus, *Leptotrichia* genus, *Listeria* genus, *Megasphaera* genus, *Methanobrevibacter* genus, *Microbacterium* genus, *Micrococcus* genus, *Mitsuokella* genus, *Mobiluncus* genus, *Mogibacterium* genus, *Moraxella* genus, *Morganella* genus, *Mycobacterium* genus, *Mycoplasma* genus, *Neisseria* genus, *Nocardia* genus, *Olsenella* genus, *Oribacterium* genus, *Paracoccus* genus, *Parascardovia* genus, *Parvimonas* genus, *Peptoniphilus* genus, *Peptostreptococcus* genus, *Porphyromonas* genus, *Prevotella* genus, *Propionibacterium (Cutibacterium)* genus, *Proteus* genus, *Providencia* genus, *Pseudomonas* genus, *Pseudopropionibacterium* genus, *Pseudoramibacter* genus, *Pyramidobacter* genus, *Ralstonia* genus, *Rothia* genus, *Scardovia* genus, *Schlegelella* genus, *Sebaldella* genus, *Selenomonas* genus, *Serratia* genus, *Simonsiella* genus, *Slackia* genus, *Sneathia* genus, *Solobacterium* genus, *Staphylococcus* genus, *Stenotrophomonas* genus, *Stomatobaculum* genus, *Streptococcus* genus, *Tannerella* genus, *Treponema* genus, *Ureaplasma* genus, *Veillonella* genus, and *Fretibacterium* genus.

[10] The method according to [8], wherein the target nucleic acids are rRNA genes of at least two species of bacteria selected from the group consisting of Abiotrophia defectiva, Achromobacter xylosoxidans, Acinetobacter baumannii, Acinetobacter calcoaceticus, Acinetobacter johnsonii, Acinetobacter junii, Acinetobacter lwoffii, Actinomyces dentalis, Actinomyces georgiae, Actinomyces gerencseriae, Actinomyces graevenitzii, Actinomyces israelii, Actinomyces johnsonii, Actinomyces meyeri, Actinomyces naeslundii, Actinomyces odontolyticus, Actinomyces oris, Actinomyces turicensis, Actinomyces viscosus, Aggregatibacter actinomycetemcomitans, Aggregatibacter aphrophilus, Alloprevotella rava, Alloprevotella tannerae, Alloscardovia omnicolens, Anaeroglobus geminatus, Atopobium parvulum, Bacillus cereus, Bacteroides fragilis, Bifidobacterium adolescentis, Bifidobacterium bifidum, Bifidobacterium breve, Bifidobacterium dentium, Bifidobacterium longum, Bordetella pertussis, Brucella abortus, Burkholderia cepacia, Campylobacter concisus, Campylobacter gracilis, Campylobacter rectus, Campylobacter showae, Capnocytophaga gingivalis, Capnocytophaga ochracea, Capnocytophaga sputigena, Cardiobacterium hominis, Catonella morbi, Chlamydia psittaci, Chlamydia trachomatis, Chlamydia pneumoniae, Chryseobacterium indologenes, Citrobacter freundii, Clostridium perfringens, Collinsella aerofaciens, Corynebacterium diphtheriae, Corynebacterium durum, Corynebacterium matruchotii, Corynebacterium pseudodiphtheriticum, Corynebacterium pseudotuberculosis, Corynebacterium renale, Corynebacterium striatum, Corynebacterium xerosis, Coxiella burnetii, Cryptobacterium curtum, Curvibacter delicatus, Dialister invisus, Dialister micraerophilus, Dialister pneumosintes, Eggerthella lenta, Eikenella corrodens, Elizabethkingia meningoseptica, Enterobacter aerogenes, Enterobacter cloacae, Enterococcus avium, Enterococcus faecalis, Enterococcus faecium, Escherichia coli, Eubacterium brachy, Eubacterium infirmum, Eubacterium limosum, Eubacterium minutum, Eubacterium nodatum, Eubacterium saphenum, Eubacterium sulci, Filifactor alocis, Fretibacterium fastidiosum, Fusobacterium necrophorum, Fusobacterium nucleatum, Fusobacterium nucleatum subsp. animalis, Fusobacterium nucleatum subsp. nucleatum, Fusobacterium nucleatum subsp. polymorphum, Fusobacterium nucleatum subsp. vincentii, Fusobacterium periodonticum, Fusobacterium simiae, Gemella haemolysans, Gemella morbillorum, Gemella sanguinis, Granulicatella adiacens, Granulicatella balaenopterae, Granulicatella elegans, Haemophilus ducreyi, Haemophilus haemolyticus, Haemophilus influenzae, Haemophilus parainfluenzae, Helicobacter pylori, Kingella denitrificans, Kingella kingae, Kingella oralis, Klebsiella oxytoca, Klebsiella pneumoniae, Lachnoanaerobaculum orale, Lachnoanaerobaculum saburreum, Lactobacillus acidophilus, Lactobacillus brevis, Lactobacillus buchneri, Lactobacillus casei, Lactobacillus crispatus, Lactobacillus delbrueckii, Lactobacillus fermentum, Lactobacillus gasseri, Lactobacillus paracasei, Lactobacillus plantarum, Lactobacillus reuteri, Lactobacillus rhamnosus, Lactobacillus salivarius, Lautropia mirabilis, Legionella pneumophila, Leptotrichia buccalis, Leptotrichia sp. OT 215, Leptotrichia sp. OT 417, Leptotrichia sp. OT 462, Listeria monocytogenes, Megasphaera micronuciformis, Micrococcus luteus, Mogibacterium diversum, Mogibacterium neglectum, Mogibacterium pumilum, Mogibacterium timidum, Mogibacterium vescum, Moraxella catarrhalis, Moraxella lacunata, Morganella morganii, Mycobacterium abscessus, Mycobacterium avium, Mycobacterium chelonae, Mycobacterium fortuitum, Mycobacterium gordonae, Mycobacterium haemophilum, Mycobacterium intracellulare, Mycobacterium kansasii, Mycobacterium marinum, Mycobacterium szulgai, Mycobacterium tuberculosis, Mycobacterium xenopi, Mycoplasma genitalium, Mycoplasma hominis, Mycoplasma orale, Mycoplasma pneumoniae, Mycoplasma salivarium, Neisseria elongata, Neisseria flava, Neisseria flavescens, Neisseria gonorrhoeae, Neisseria meningitidis, Neisseria mucosa, Neisseria sicca, Neisseria subflava, Nocardia asteroides, Nocardia brasilliensis, Nocardia farcinica, Nocardia nova, Olsenella uli, Oribacterium asaccharolyticum, Oribacterium parvum, Parascardovia denticolens, Parvimonas micra, Peptostreptococcus stomatis, Porphyromonas catoniae, Porphyromonas endodontalis, Porphyromonas gingivalis, Porphyromonas pasteri, Prevotella dentalis, Prevotella denticola, Prevotella histicola, Prevotella intermedia, Prevotella loescheii, Prevotella melaninogenica, Prevotella nigrescens, Prevotella oralis, Prevotella oris, Prevotella pallens, Prevotella salivae, Prevotella shahii, Prevotella veroralis, Propionibacterium acnes (Cutibacterium acnes), Propionibacterium granulosum (Cutibacterium granulosum), Propionibacterium propionicus (Pseudopropionibacterium propionicum), Proteus mirabilis, Proteus vulgaris, Providencia stuartii, Pseudomonas aeruginosa, Pseudomonas fluorescens, Pseudomonas putida, Pseudoramibacter alactolyticus, Ralstonia pickettii, Rothia aeria, Rothia dentocariosa, Rothia mucilaginosa, Scardovia inopinata, Schlegelella aquatica, Sebaldella termitidis, Selenomonas flueggei, Selenomonas noxia, Selenomonas sp. OT 478, Selenomonas sputigena, Serratia marcescens, Simonsiella muelleri, Slackia exigua, Solobacterium moorei, SR1 sp. OT 345, Staphylococcus aureus, Staphylococcus epidermidis, Staphylococcus saccharolyticus, Stenotrophomonas maltophilia, Streptococcus agalactiae, Streptococcus anginosus, Streptococcus australis, Streptococcus constellatus, Streptococcus cristatus, Streptococcus dentisani, Streptococcus gordonii, Streptococcus infantis, Streptococcus intermedius, Streptococcus milleri, Streptococcus mitis, Streptococcus mutans, Streptococcus oralis, Streptococcus parasanguinis, Streptococcus pneumoniae, Streptococcus pyogenes, Streptococcus salivarius, Streptococcus sanguinis, Streptococcus sobrinus, Streptococcus tigurinus, Streptococcus vestibularis, Tannerella forsythia, Treponema denticola, Treponema lecithinolyticum, Treponema medium, Treponema pallidum, Treponema socranskii, Treponema vincentii, Ureaplasma urealyticum, Veillonella atypica, Veillonella dispar, *Veillonella parvula,* and *Veillonella rogosae.*

[11] The method according to any of [1] to [10], wherein Step (c) comprises the steps of:

(c1) creating a calibration curve of relationships, using a part or all of the target nucleic acids and the specific nucleic acid group and the control nucleic acid, between amounts before amplification and indicators of amounts after amplification; and

(c2) calculating a total amount of all target nucleic acids and the specific nucleic acid group in the sample by applying an indicator of a total amount of the amplified all target nucleic acids and the specific nucleic acid group from the sample and an indicator of an amount of the amplified control nucleic acid to the calibration curve created in Step (c1).

[12] The method according to [11], wherein the nucleic acid used in Step (c1) is a part of the target nucleic acids and the specific nucleic acid group.

[13] The method according to [11] or [12], wherein the calibration curve represents a relationship between

a ratio of an indicator of a total amount of a part or all of the target nucleic acids and the specific nucleic acid group to an indicator of an amount of the control nucleic acid after amplification and

a ratio of a total amount of a part or all of the target nucleic acids and the specific nucleic acid group to an amount of the control nucleic acid before amplification.

[14] The method according to any of [1] to [13], wherein, in Steps (c) and (d), an indicator of an amount of each amplified target nucleic acid, an indicator of a total amount of amplified all target nucleic acids and the specific nucleic acid group, and an indicator of an amount of the amplified control nucleic acid are signal intensities obtained by hybridization with the following probes (x) to (z), respectively, loaded on a DNA chip:

(x) a probe hybridizable respectively with amplified products of two or more types of target nucleic acids;

(y) a probe hybridizable in common with both amplified products of all target nucleic acids and the specific nucleic acid group in the sample; and

(z) a probe hybridizable with amplified products of the control nucleic acid.

[15] The method according to [14], wherein Step (d) comprises the steps of:

(d1) hybridizing respective target nucleic acids (each target nucleic acid) of all target nucleic acids in the sample with the DNA chip and calculating a relational expression on signal intensities between the probe (x) and the probe (y) hybridizable with each target nucleic acid,

(d2) converting the signal intensity of the probe (x) obtained by hybridizing products amplified from the sample with the DNA chip to a value equivalent to the signal intensity of the probe (y) hybridizable with each target nucleic acid using the relational expression calculated in Step (d1),

(d3) calculating a ratio of the value equivalent to the signal intensity of the probe (y) calculated in Step (d2) to the signal intensity of the probe (y) obtained by hybridizing products amplified from the sample with the DNA chip; and

(d4) calculating an absolute amount of each target nucleic acid in the sample by multiplying the total amount of all target nucleic acids and the specific nucleic acid group in the sample calculated in Step (c) by the occupancy calculated in Step (d3).

[16] The method according to [14] or [15], wherein Step (b) comprises the step of enriching nucleic acids after amplification for a single chain hybridizable with probes loaded on the DNA chip.

[17] The method according to [16], wherein the step of enriching for a single chain is carried out by asymmetric PCR or λ exonuclease treatment.

[18] The method according to any of [14] to [17], wherein the probe (x) is any of the following sequences:

(I) a sequence selected from the group consisting of the base sequences as set forth in SEQ ID NO: 3 to 191;
(II) a complementary sequence to (I); or
(III) a sequence substantially identical with the sequence (I) or (II).

[19] The method according to any of [14] to [18], wherein the DNA chip is a fiber-type DNA chip.

[20] A kit comprising: one or two pairs of primer sets for co-amplifying all target nucleic acids and the specific nucleic acid group in a sample and a control nucleic acid by PCR method; a control nucleic acid; and a DNA chip on which the following probes (x) to (z) are loaded:

(x) a probe hybridizable respectively with amplified products of two or more types of target nucleic acids;
(y) a probe hybridizable in common with both amplified products of all target nucleic acids and the specific nucleic acid group in the sample; and
(z) a probe hybridizable with amplified products of the control nucleic acid.

[21] The kit according to [20], wherein the probe (x) is any of the following sequences:

(I) a sequence selected from the group consisting of the base sequences as set forth in SEQ ID NO: 3 to 191;
(II) a complementary sequence to (I); or
(III) a sequence substantially identical with the sequence (I) or (II).

[22] A DNA chip on which the following probes (x) to (z) are loaded:

(x) a probe hybridizable respectively with amplified products of two or more types of target nucleic acids;
(y) a probe hybridizable in common with both amplified products of all target nucleic acids and the specific nucleic acid group in the sample; and
(z) a probe hybridizable with amplified products of the control nucleic acid, wherein the probe (x) is any of the following sequences:

(IV) a sequence selected from the group consisting of the base sequences as set forth in SEQ ID NO: 43 to 191;
(V) a complementary sequence to (IV); or
(VI) a sequence substantially identical with the sequence (IV) or (V).

[Advantageous Effects of Invention]

According to the present invention, absolute amounts of two or more types of target nucleic acids in a sample can be simultaneously determined in a simple manner.

[Brief Description of Drawings]

[Figure 1] Figure 1 is a drawing showing the relationship on an amount ratio before amplification and a ratio of indicators of amounts after amplification between nucleic acids for calibration and a control nucleic acid.

[Figure 2] Figure 2 is drawings showing relationships on signal intensities among probes.

[Figure 3] Figure 3 is a drawing showing the relationship on an amount ratio before amplification and a ratio of indicators of amounts after amplification between nucleic acids for calibration and a control nucleic acid.

[Figure 4A] Figure 4A is a schematic drawing showing a principle in reference to an example of the embodiments of the present invention and a drawing illustrating Step (a).

[Figure 4B] Figure 4B is a schematic drawing showing a principle in reference to an example of the embodiments of the present invention and a drawing illustrating Step (b).

[Figure 4C] Figure 4C is a schematic drawing showing a principle in reference to an example of the embodiments of the present invention and a drawing illustrating Step (c).

[Figure 4D] Figure 4D is a schematic drawing showing a principle in reference to an example of the embodiments of the present invention and a drawing illustrating Step (d).

[Figure 5] Figure 5 is a drawing showing the relationship on an amount ratio before amplification and a ratio of indicators of amounts after amplification between nucleic acids for calibration and a control nucleic acid.
[Figure 6] Figure 6 is drawings showing relationships on signal intensities among probes.
[Figure 7] Figure 7 is a drawing showing the relationship on an amount ratio before amplification and a ratio of indicators of amounts after amplification between nucleic acids for calibration and a control nucleic acid.
[Figure 8] Figure 8 is drawings showing relationships on signal intensities among probes.
[Figure 9] Figure 9 is a drawing showing the relationship on an amount ratio before amplification and a ratio of indicators of amounts after amplification between nucleic acids for calibration and a control nucleic acid.
[Figure 10] Figure 10 is drawings showing relationships on signal intensities among probes.

[Description of Embodiments]

Hereinafter, the present invention will be described in detail. The following embodiments are examples to describe the present invention and do not intend to limit the present invention only to these embodiments. The present invention can be carried out in various modifications without departing from the spirit of the present invention. The present description incorporates the contents described in the specification and drawings of a Japanese patent application (Japanese Patent Application No. 2018-100475) filed on May 25, 2018 and a Japanese patent application (Japanese Patent Application No. 2019-026519) filed on February 18, 2019, which serve as a basis of the priority of the present application.

1. Determination of absolute amounts of target nucleic acids

(1) Target nucleic acid

The target nucleic acid in the present invention is a nucleic acid to be quantified. For example, in a sample containing a bacterial genome DNA to be measured, a bacterial genome DNA not to be measured and other DNA, the target nucleic acids are the nucleic acids from bacteria to be measured. In the present invention, even if when a part of the nucleic acids from bacteria not to be measured is collectively amplified with the target nucleic acids, such a part of the amplified nucleic acids is not the target nucleic acid. Such a "the specific nucleic acid group to be collectively amplified with the target nucleic acids" will be described later.
For evaluating bacterial florae, as the target nucleic acids, nucleic acids comprising a consensus sequence conserved among genome DNA of a plurality of bacterial species to be measured can be used. Further, as the target nucleic acids, nucleic acids comprising a base sequence specific to respective bacteria to be measured can be used.
When the target nucleic acids comprise consensus sequences of a plurality of bacterial species, target nucleic acids (all target nucleic acids) of a plurality of bacterial species can be captured using a single type of probe. When the target nucleic acids comprise base sequences specific to respective bacteria to be measured, bacterial target nucleic acids (each target nucleic acid) of interest can be specifically detected.
A target nucleic acid may be one molecule of a nucleic acid or a plurality thereof may be present as a partial sequence in one molecule of a nucleic acid. For example, a bacterial 16SrRNA gene (for example, DNA encoding 16SrRNA) may be present in plurality copies in one molecule of a genome DNA but when these copies are considered as target nucleic acids, each copy can be considered as one molecule of the target nucleic acid, respectively. A nucleic acid may be either DNA or RNA.
In the present invention, the target nucleic acid further includes a nucleic acid mass which can be considered as a single type when obtaining an indicator of an amount of target nucleic acids. Specifically, a mass of nucleic acids comprising identical base sequence regions can be considered as a single type. For example, when a DNA chip is used, for a mass of nucleic acids having a partially common sequence, an indicator of an amount as a signal intensity of a probe hybridizable with such a common sequence can be obtained, and thereby the mass of nucleic acids can be considered as one target nucleic acid. Examples of this specifically include a case when the 16SrRNA genes of bacteria belonging to the *Streptococcus* genus in a sample are considered as target nucleic acids and an indicator of an amount is obtained from a signal intensity of a probe corresponding to the common sequence thereof.
Examples of the target nucleic acid include a genome DNA, a gene of interest in a genome DNA, and mRNA. More specifically, a bacterial genome DNA is preferable, a rRNA gene in a bacterial genome DNA is more preferable, and the 16SrRNA gene in a bacterial genome DNA is particularly preferable.
For evaluating oral bacterial florae, for example, rRNA genes of bacteria belonging to the *Abiotrophia* genus, *Achromobacter* genus, *Acinetobacter* genus, *Actinomyces* genus, *Aerococcus* genus, *Aggregatibacter* genus, *Alloprevotella* genus, *Alloscardovia* genus, *Anaerococcus* genus, *Anaeroglobus* genus, *Arcanobacterium* genus, *Atopobium* genus, *Bacillus* genus, *Bacteroides* genus, *Bifidobacterium* genus, *Bordetella* genus, *Brevundimonas* genus, *Brucella* genus, *Burkholderia* genus, *Campylobacter* genus, *Candidatus Absconditabacteria* (SRI), *Candidatus Saccharibacteria* (TM7), *Capnocytophaga* genus, *Cardiobacterium* genus, *Catonella* genus, *Chlamydia* genus, *Chlamydophila* genus, *Chryseobacterium* genus, *Citrobacter* genus, *Clostridium* genus, *Collinsella* genus, *Corynebacterium* genus, *Coxiella* genus, *Cronobacter* genus, *Cryptobacterium* genus, *Curvibacter* genus, *Dialister* genus, *Eggerthella* genus, *Eikenella* genus, *Elizabethkingia, Enterobacter* genus, *Enterococcus* genus, *Escherichia* genus, *Eubacterium* genus, *Filifactor* genus, *Finegoldia* genus, *Fusobacterium* genus, *Gardnerella* genus, *Gemella* genus, *Granulicatella* genus, *Haemophilus* genus, *Helicobacter* genus, *Kingella* genus, *Klebsiella* genus, *Kocuria* genus, *Lachnoanaerobaculum* genus, *Lactobacillus* genus, *Lactococcus* genus, *Lautropia* genus, *Legionella* genus, *Leptotrichia* genus, *Listeria* genus, *Megasphaera* genus, *Methanobrevibacter* genus, *Microbacterium* genus, *Micrococcus* genus, *Mitsuokella* genus, *Mobiluncus* genus, *Mogibacterium* genus, *Moraxella* genus, *Morganella* genus, *Mycobacterium* genus, *Mycoplasma* genus, *Neisseria* genus, *Nocardia* genus, *Olsenella* genus, *Oribacterium* genus, *Paracoccus* genus, *Parascardovia* genus, *Parvimonas* genus, *Peptoniphilus* genus, *Peptostreptococcus* genus, *Porphyromonas* genus, *Prevotella* genus, *Propionibacterium (Cutibacterium)* genus, *Proteus* genus, *Providencia* genus, *Pseudomonas* genus, *Pseudopropionibacterium* genus, *Pseudoramibacter* genus, *Pyramidobacter* genus, *Ralstonia* genus, *Rothia* genus, *Scardovia* genus, *Schlegelella* genus, *Sebaldella* genus, *Selenomonas* genus, *Serratia* genus, *Simonsiella* genus, *Slackia* genus, *Sneathia* genus, *Solobacterium* genus, *Staphylococcus* genus, *Stenotrophomonas* genus, *Stomatobaculum* genus, *Streptococcus* genus, *Tannerella* genus, *Treponema* genus, *Ureaplasma* genus, *Veillonella* genus and *Fretibacterium* genus can be considered as target nucleic acids, and more preferably rRNA genes of bacteria belonging to the *Actinomyces* genus, *Aggregatibacter* genus, *Alloprevotella* genus, *Campylobacter* genus, *Candidatus Absconditabacteria* (SRI), *Capnocytophaga* genus, *Corynebacterium* genus, *Eikenella* genus, *Eubacterium* genus, *Filifactor* genus, *Fusobacterium* genus, *Gemella* genus, *Granulicatella* genus, *Haemophilus* genus, *Helicobacter* genus, *Lactobacillus* genus, *Leptotrichia* genus, *Megasphaera* genus, *Neisseria* genus, *Parvimonas* genus, *Peptostreptococcus* genus, *Porphyromonas* genus, *Prevotella* genus, *Rothia* genus, *Selenomonas* genus, *Solobacterium* genus, *Streptococcus* genus, *Tannerella* genus, *Treponema* genus and *Veillonella* genus can be considered as target nucleic acids but are not limited thereto.

Further, for the target nucleic acid, rRNA genes of bacteria such as Abiotrophia defectiva, Achromobacter xylosoxidans, Acinetobacter baumannii, Acinetobacter calcoaceticus, Acinetobacter johnsonii, Acinetobacter junii, Acinetobacter lwoffii, Actinomyces dentalis, Actinomyces georgiae, Actinomyces gerencseriae, Actinomyces graevenitzii, Actinomyces israelii, Actinomyces johnsonii, Actinomyces meyeri, Actinomyces naeslundii, Actinomyces odontolyticus, Actinomyces oris, Actinomyces turicensis, Actinomyces viscosus, Aggregatibacter actinomycetemcomitans, Aggregatibacter aphrophilus, Alloprevotella rava, Alloprevotella tannerae, Alloscardovia omnicolens, Anaeroglobus geminatus, Atopobium parvulum, Bacillus cereus, Bacteroides fragilis, Bifidobacterium adolescentis, Bifidobacterium bifidum, Bifidobacterium breve, Bifidobacterium dentium, Bifidobacterium longum, Bordetella pertussis, Brucella abortus, Burkholderia cepacia, Campylobacter concisus, Campylobacter gracilis, Campylobacter rectus, Campylobacter showae, Capnocytophaga gingivalis, Capnocytophaga ochracea, Capnocytophaga sputigena, Cardiobacterium hominis, Catonella morbi, Chlamydia psittaci, Chlamydia trachomatis, Chlamydia pneumoniae, Chryseobacterium indologenes, Citrobacter freundii, Clostridium perfringens, Collinsella aerofaciens, Corynebacterium diphtheriae, Corynebacterium durum, Corynebacterium matruchotii, Corynebacterium pseudodiphtheriticum, Corynebacterium pseudotuberculosis, Corynebacterium renale, Corynebacterium striatum, Corynebacterium xerosis, Coxiella burnetii, Cryptobacterium curtum, Curvibacter delicatus, Dialister invisus, Dialister micraerophilus, Dialister pneumosintes, Eggerthella lenta, Eikenella corrodens, Elizabethkingia meningoseptica, Enterobacter aerogenes, Enterobacter cloacae, Enterococcus avium, Enterococcus faecalis, Enterococcus faecium, Escherichia coli, Eubacterium brachy, Eubacterium infirmum, Eubacterium limosum, Eubacterium minutum, Eubacterium nodatum, Eubacterium saphenum, Eubacterium sulci, Filifactor alocis, Fretibacterium fastidiosum, Fusobacterium necrophorum, Fusobacterium nucleatum, Fusobacterium nucleatum subsp. animalis, Fusobacterium nucleatum subsp. nucleatum, Fusobacterium nucleatum subsp. polymorphum, Fusobacterium nucleatum subsp. vincentii, Fusobacterium periodonticum, Fusobacterium simiae, Gemella haemolysans, Gemella morbillorum, Gemella sanguinis, Granulicatella adiacens, Granulicatella balaenopterae, Granulicatella elegans, Haemophilus ducreyi, Haemophilus haemolyticus, Haemophilus influenzae, Haemophilus parainfluenzae, Helicobacter pylori, Kingella denitrificans, Kingella kingae, Kingella oralis, Klebsiella oxytoca, Klebsiella pneumoniae, Lachnoanaerobaculum orale, Lachnoanaerobaculum saburreum, Lactobacillus acidophilus, Lactobacillus brevis, Lactobacillus buchneri, Lactobacillus casei, Lactobacillus crispatus, Lactobacillus delbrueckii, Lactobacillus fermentum, Lactobacillus gasseri, Lactobacillus paracasei, Lactobacillus plantarum, Lactobacillus reuteri, Lactobacillus rhamnosus, Lactobacillus salivarius, Lautropia mirabilis, Legionella pneumophila, Leptotrichia buccalis, Leptotrichia sp. OT 215, Leptotrichia sp. OT 417, Leptotrichia sp. OT 462, Listeria monocytogenes, Megasphaera micronuciformis, Micrococcus luteus, Mogibacterium diversum, Mogibacterium neglectum, Mogibacterium pumilum, Mogibacterium timidum, Mogibacterium vescum, Moraxella catarrhalis, Moraxella lacunata, Morganella morganii, Mycobacterium abscessus, Mycobacterium avium, Mycobacterium chelonae, Mycobacterium fortuitum, Mycobacterium gordonae, Mycobacterium haemophilum, Mycobacterium intracellulare, Mycobacterium kansasii, Mycobacterium marinum, Mycobacterium szulgai, Mycobacterium tuberculosis, Mycobacterium xenopi, Mycoplasma genitalium, Mycoplasma hominis, Mycoplasma orale, Mycoplasma pneumoniae, Mycoplasma salivarium, Neisseria elongata, Neisseria flava, Neisseria flavescens, Neisseria gonorrhoeae, Neisseria meningitidis, Neisseria mucosa, Neisseria sicca, Neisseria subflava, Nocardia asteroides, Nocardia brasilliensis, Nocardia farcinica, Nocardia nova, Olsenella uli, Oribacterium asaccharolyticum, Oribacterium parvum, Parascardovia denticolens, Parvimonas micra, Peptostreptococcus stomatis, Porphyromonas catoniae, Porphyromonas endodontalis, Porphyromonas gingivalis, Porphyromonas pasteri, Prevotella dentalis, Prevotella denticola, Prevotella histicola, Prevotella intermedia, Prevotella loescheii, Prevotella melaninogenica, Prevotella nigrescens, Prevotella oralis, Prevotella oris, Prevotella pallens, Prevotella salivae, Prevotella shahii, Prevotella veroralis, Propionibacterium acnes (Cutibacterium acnes), Propionibacterium granulosum (Cutibacterium granulosum), Propionibacterium propionicus (Pseudopropionibacterium propionicum), Proteus mirabilis, Proteus vulgaris, Providencia stuartii, Pseudomonas aeruginosa, Pseudomonas fluorescens, Pseudomonas putida, Pseudoramibacter alactolyticus, Ralstonia pickettii, Rothia aeria, Rothia dentocariosa, Rothia mucilaginosa, Scardovia inopinata, Schlegelella aquatica, Sebaldella termitidis, Selenomonas flueggei, Selenomonas noxia, Selenomonas sp. OT 478, Selenomonas sputigena, Serratia marcescens, Simonsiella muelleri, Slackia exigua, Solobacterium moorei, SR1 sp. OT 345, Staphylococcus aureus, Staphylococcus epidermidis, Staphylococcus saccharolyticus, Stenotrophomonas maltophilia, Streptococcus agalactiae, Streptococcus anginosus, Streptococcus australis, Streptococcus constellatus, Streptococcus cristatus, Streptococcus dentisani, Streptococcus gordonii, Streptococcus infantis, Streptococcus intermedius, Streptococcus milleri, Streptococcus mitis, Streptococcus mutans, Streptococcus oralis, Streptococcus parasanguinis, Streptococcus pneumoniae, Streptococcus pyogenes, Streptococcus salivarius, Streptococcus sanguinis, Streptococcus sobrinus, Streptococcus tigurinus, Streptococcus vestibularis, Tannerella forsythia, Treponema denticola, Treponema lecithinolyticum, Treponema medium, Treponema pallidum, Treponema socranskii, Treponema vincentii, Ureaplasma urealyticum, Veillonella atypica, Veillonella dispar, *Veillonella parvula* and *Veillonella rogosae* considered associated with human health are preferably considered as target nucleic acids, and rRNA genes of *Actinomyces graevenitzii,* Actinomyces israelii, Actinomyces naeslundii, Actinomyces odontolyticus, Actinomyces viscosus, Aggregatibacter actinomycetemcomitans, Aggregatibacter actinomycetemcomitans, Alloprevotella rava, Alloprevotella sp. OT 308, Campylobacter concisus, Campylobacter gracilis, Campylobacter rectus, Campylobacter showae, Capnocytophaga gingivalis, Capnocytophaga ochracea, Capnocytophaga sputigena, Corynebacterium matruchotii, Eikenella corrodens, Enterobacter cloacae, Enterococcus faecalis, Eubacterium nodatum, Eubacterium saphenum, Eubacterium sulci, Filifactor alocis, Fusobacterium nucleatum, Fusobacterium nucleatum subsp. animalis, Fusobacterium nucleatum subsp. polymorphum, Fusobacterium nucleatum subsp. vincentii, Fusobacterium periodonticum, Gemella sanguinis, Granulicatella adiacens, Haemophilus parainfluenzae, Helicobacter pylori, Klebsiella pneumoniae, Lactobacillus acidophilus, Lactobacillus casei, Lactobacillus salivarius, Leptotrichia sp. OT 215, Leptotrichia sp. OT 417, Leptotrichia sp. OT 462, Megasphaera micronuciformis, Neisseria flavescens, Parvimonas micra, Peptostreptococcus stomatis, Porphyromonas catoniae, Porphyromonas endodontalis, Porphyromonas gingivalis, Porphyromonas pasteri, Prevotella denticola, Prevotella histicola, Prevotella intermedia, Prevotella loescheii, Prevotella melaninogenica, Prevotella nigrescens, Prevotella pallens, Prevotella shahii, Prevotella sp. OT 306, Prevotella sp. OT 313, Prevotella veroralis, Rothia dentocariosa, Rothia mucilaginosa, Selenomonas noxia, Selenomonas sp. OT 478, Selenomonas sputigena, Solobacterium moorei, SRI sp. OT 345, Staphylococcus aureus, Staphylococcus epidermidis, Streptococcus constellatus, Streptococcus dentisani, Streptococcus gordonii, Streptococcus intermedius, Streptococcus mitis, Streptococcus mutans, Streptococcus parasanguinis, Streptococcus pneumoniae, Streptococcus salivarius, Streptococcus sobrinus, Tannerella forsythia, Treponema denticola, Treponema medium, Treponema socranskii, Veillonella atypica, *Veillonella parvula* and *Veillonella rogosae* are more preferably considered as target nucleic acids but are not limited thereto.

(2) Absolute amount

The absolute amount of a target nucleic acid in the present invention means an amount of substance, the number of copies or a weight of the target nucleic acid. A result of NGS (read length) and a signal intensity of a DNA chip are relative quantitative values whereby respective abundance ratios or magnitudes in the values among samples can be compared but absolute amounts are not indicated.

2. Step (a)

(1) Sample

The method of the present invention comprises the step of (a) mixing a sample with a known amount of a control nucleic acid (Figure 4A).

The sample in the present invention is not particularly restricted as long as nucleic acids are contained. For the sample, for example, foods and drinks, biological samples, environmental samples and samples from industrial process can be used.

Examples of the foods and drinks include naturally occurring foods and drinking waters, processed foods and processed drinks but are not limited thereto.

Examples of the biological sample are not limited and include tissues, cells, body fluids, plaques and feces obtained (for example, collected) from eucaryotes (for example, mammals, birds and plants). Examples of the mammal include mice, rats, rabbits, dogs, monkeys and human but are not limited thereto. The tissue is not limited as long as it can be obtained from various organs and examples include skin tissues, oral tissues, mucosal tissues, intestinal tissue and cancer tissues. The cell is not limited as long as it is a cell from these tissues and examples include cells from oral tissues, cells from mucosal tissues and cells from skin tissues. The body fluid is not limited and examples include saliva, gingival crevicular fluid (GCF), blood, urine, breast milk and amniotic fluid.

Examples of the environmental sample include soils, river waters, seawaters and hot spring waters.

Examples of the sample from the industrial process include active sludges.

Example of other samples include cultures of microorganisms artificially grown such as fermenter cultures.

In an embodiment, the sample of the present invention is at least one selected from the group consisting of active sludges, soils, river waters, seawaters, hot spring waters, drinking waters, processed foods, fermenter cultures, and tissues, cells and body fluids collected from eukaryotes.

In another embodiment, the sample of the present invention is at least one selected from the group consisting of saliva, plaque, gingival crevicular fluid (GCF), feces and skin-derived samples collected from mammals.

For the "sample" in the present invention, nucleic acids extracted from the samples described in the above examples and compositions containing extracted nucleic acids can be used in addition to the samples described in the above examples.

The method for extracting nucleic acids from a sample in the present invention is not limited and a known method can be used. For examples, a method of utilizing a commercial nucleic acid extraction kit and a method of phenol·chloroform extraction after treatment with proteinase K are included. When bacterial DNA is extracted, bacteriolysis treatment is included. Examples of the bacteriolysis treatment include enzyme treatment, heat treatment and bead crushing. A suspension of a sample can also be used directly for an amplification reaction.

A sample in the present invention can contain two or more types of target nucleic acids. The number of types of target nucleic acids contained in a sample has no particular upper limit and can be suitably selected in accordance with the purpose of evaluation.

(2) <u>Control nucleic acid</u>

The control nucleic acid in the present invention is a nucleic acid added in a known amount to an amplification reaction solution before the reaction thereby to be a control when quantifying target nucleic acids and the like.

The addition of a control nucleic acid enables the determination of amounts before amplification from indicators of amounts after amplification of all target nucleic acids and the specific nucleic acid group to be collectively amplified with the target nucleic acids (hereinafter, "the specific nucleic acid group to be collectively amplified with the target nucleic acids" is also referred to as "the specific nucleic acid group"). For example, when the amplification by PCR method is used, an indicator of an amount dependent on an amount before amplification is obtained based on the principle of competitive PCR method.

The control nucleic acid is desirably a sequence which does not affect obtaining an indicator of an amount of a target nucleic acid and an indicator of the total amount of the target nucleic acid and a specific nucleic acid group and, for example, when a technique for obtaining an indicator of an amount is a DNA chip, a sequence which is not non-specifically hybridizable with a target nucleic acid or a probe for the target nucleic acid and the specific nucleic acid group is desirable, and more specifically, a specific sequence which has low identity with and similarity to a complementary sequence to a target nucleic acid and a probe for the target nucleic acid and a specific nucleic acid group is desirable.

The control nucleic acid needs to comprise a site required for amplification. For example, when the amplification by PCR method is used, the "site required for amplification" is a primer binding site. The base sequence of a primer binding site may be identical with or different from the base sequence of a primer binding site of a target nucleic acid.

It is difficult for the control nucleic acid to achieve the same amplification efficiency as those of a target nucleic acid and a specific nucleic acid group. However, on the other hand, it is desirable to bring the amplification efficiencies of the both closer as extremely different amplification efficiencies are presumed to affect the quantification. For achieving closer amplification efficiencies of a control nucleic acid, a target nucleic acid and the specific nucleic acid group, it is desirable that the base length of a control nucleic acid be designed so that there are no significant difference from base lengths at the sites to be amplified in a target nucleic acid and the specific nucleic acid group. For example, when amplified products from a target nucleic acid and the specific nucleic acid group are about 500 bp, amplified products of a control nucleic acid ranges desirably from about 300 bp to 1000 bp.

Examples of the design method for a base sequence of a control nucleic acid include a method in which a base sequence is randomly produced and a method of using a sequence having a partial homology with base sequences of a target nucleic acid and the specific nucleic acid group. The method of randomly producing a base sequence is particularly preferable in the present invention. This is because random designing is more likely to suppress the impact of the non-specific hybridization on quantification in the case of, for example, a DNA chip, in a sample in which the diversity of base sequences of target nucleic acids is high and the similarity thereof to each other is also high, such as bacterial florae in a human-derived sample.

Designing a control nucleic acid can be achieved by using, for example, RANDBETWEEN function in a Microsoft software "EXCEL" by which integers from 1 to 4 are randomly created in the X numbers (X is any number), linked to form X-digit numerical values composed only of the numerical values from 1 to 4 and are substituted with A for 1, T for 2, C for 3, and G for 4 thereby obtaining a large number of random sequences by X bases of ATGC. In these sequences, only the sequences having the same number of the sum of G and C as the sum of A and T are extracted, the extracted sequences are blast-searched on database such as NCBI GenBank, those having fewer analogous sequences are selected and, for example, in the case of amplifying by PCR method, sites required for amplification are added to both ends of the sequences thereby enabling the design. The sites required for amplification to be added at this time may be, for example, a sequence designed randomly as in the above or may be a sequence identical with the sequence at the primer binding site of a target nucleic acid. Additionally, the designed sequence can also be elongated by a suitable linkage or shortened by a partial removal.

The number of types of the control nucleic acid used in the present invention is not limited but the fewer the number thereof, the preferable from the viewpoint of simplicity, and is, for example, a single type.

(3) Mixing

The "mixing" of a sample with a control nucleic acid in Step (a) includes the addition of the control nucleic acid to the sample or the addition of the sample to a composition containing the control nucleic acid.

3. Step (b)

(1) Specific nucleic acid group to be collectively amplified with target nucleic acids

The method of the present invention comprises the step of co-amplifying all target nucleic acids and the specific nucleic acid group to be collectively amplified with the target nucleic acids in a sample and a control nucleic acid (Step (b)) (Figure 4B).

The "all target nucleic acids" in the present invention means all of the plural type of all target nucleic acids contained in a sample or amplified products.

In the present invention, a sample can contain a genome DNA of bacterial species to be measured, a genome DNA of bacterial species not to be measured and/or other nucleic acids (Figure 4A). In the case where the genome DNA of bacterial species not to be measured contained in the sample comprise a common base sequence with a part of the base sequences of all target nucleic acids (for example, the 16SrRNA genes) (consensus sequence), when all target nucleic acids (for example, the 16SrRNA genes) in the sample or extracted nucleic acids are amplified, the 16SrRNA genes in the genome DNA of bacterial species not to be measured are also collectively amplified (Figure 4B). Thus, a nucleic acid group other than target nucleic acids to be collectively (together with) amplified with all target nucleic acids are, in the present invention, referred to as "the specific nucleic acid group to be collectively amplified with target nucleic acids" or "the specific nucleic acid group".

In a sample containing mRNA to be measured and mRNA not to be measured, for example, when the mRNA to be measured is considered as a target nucleic acid, in vitro transcription after reverse transcription utilizing a poly A sequence can be selected as the amplification method. In this case, the mRNA not to be measured are also collectively obtained but this mRNA also applies to "the specific nucleic acid group to be collectively amplified with target nucleic acids" or "the specific nucleic acid group".

That is, "the specific nucleic acid group to be collectively amplified with target nucleic acids" (also referred to as "the specific nucleic acid group") in the present invention refers to, other than target nucleic acids, a group of nucleic acids to be collectively amplified with the amplification of all target nucleic acids in a sample.

All target nucleic acids and the specific nucleic acid group in a sample are the nucleic acids to be collectively amplified all together as described above. Thus, the "all target nucleic acids and the specific nucleic acid group" in the present invention can be understood as a single collective group of nucleic acids comprising a common sequence. Additionally, in the present invention, a collective amount of all target nucleic acids and the specific nucleic acid group may refer to "a total of all target nucleic acids and the specific nucleic acid group."

(2) Co-amplification

The "co-amplification" in the present invention means to amplify together in the same nucleic acid amplification reaction system.

The amplification technique of nucleic acids is not limited in the present invention and a known method can be used. When a target is DNA, examples include PCR method and LAMP (Loop-Mediated Isothermal Amplification) method, and when a target is RNA, examples include in vitro transcription after reverse transcription and PCR method. PCR method is particularly preferably utilized. When a DNA chip is used, for example, primers are fluorescence-labelled thereby to obtain an indicator of an amount as a signal intensity.

For example, when a bacterial 16SrRNA gene is amplified by PCR method, the amplification can be carried out under the conditions: an extracted DNA amount from a sample in the reaction solution of 1 pg to 10 ng, a control nucleic acid concentration of 0.0001 to 1 pM, respective primer concentrations of 0.01 to 10 μM, and a total solution volume of 1 to 100 μL. More preferably, the amplification can be carried out under the conditions: an extracted DNA amount from a sample in the reaction solution of 50 pg to 200 pg, a control nucleic acid concentration of 0.01 to 0.05 pM, respective primer concentrations of 0.1 to 2 μM, and a total solution volume of 20 μL. In this case, for example, the amplification can be carried out with a cycle number of 10 to 50. More preferably, a cycle number can be 15 to 40. The DNA polymerase used herein is not particularly restricted and a commercial heat-resistant DNA polymerase can be utilized. Similarly, the temperature, time, and the composition of a buffer solution are not particularly restricted and can be suitably set considering the DNA polymerase to be used and the size of amplification region of a nucleic acid of interest. When a commercial DNA polymerase is used in the quantification method of the present invention, the reaction time of the DNA polymerase is preferably set to be a sufficient reaction time of the recommended time or more described in an instruction manual. Specifically, the amplification reaction time of each step is preferably 1.2 times or more and 3 times or less the time recommended in an instruction manual as the reaction time of the DNA polymerase to be used. Further, in the final extension step, a so-called final extension time of 5 to 15 minutes can be set as needed. Thus, the complete length of a target nucleic acid and the like is homogeneously amplified.

When rRNA genes are quantified for the purpose of measuring the number of bacteria, the quantitative value can be divided (division) by the number of copies in 1 genome to obtain the number of bacteria as the rRNA genes are present in a plurality of copies in 1 genome. The number of copies of rRNA genes in 1 genome varies depending on bacterial species and known information (for example, Genbank) can be used for such a value.

When a DNA chip is used in the present invention, the step of co-amplification can comprise the step of enriching nucleic acids after amplification for single chains (single strands) hybridizable with probes loaded on a DNA chip. The present inventors found in the study for the first time that the hybridization of a single strand DNA rather than a double strand DNA enhances not only the sensitivity but also the accuracy thereof in the quantification method of the present invention.

The step of enriching for a single chain may be, for example, a step using the amplification reaction conditions for enriching for a single chain as the reaction conditions for co-amplification in the method of the present invention or a step further comprising treatment for enriching for a single chain after the reaction of co-amplification.

The amplification reaction conditions for enriching for a single chain include, for example, conditions to be so-called asymmetric PCR. Conditions to be asymmetric PCR can employ, specifically, conditions such as varied concentrations, different melting points, or different base lengths between a pair of primers used for PCR, singly or in combination. Further, in the PCR reaction composition, a concentration of a compound which affects a melting point of a nucleic acid (for example, a salt, dNTP, oligo, denaturating agent) is adjusted to make the condition more effective for enriching for a single chain. Additionally, in the asymmetric PCR, one primer of the pair can further be added during the PCR reaction or annealing conditions (temperatures or compound concentrations in the reaction solution) can be changed.

Examples of the treatment for enriching for a single chain after the reaction of co-amplification include a method for physically separating a double strand DNA after co-amplification to a single strand DNA (for example, denaturing high performance liquid chromatography) and a chemical method (for example, λ exonuclease treatment). Such a method is not particularly limited in the present invention but, from the viewpoint of operability, a method by λ exonuclease (Enzyme Commission numbers: EC 3.1.11.3) treatment is preferable.

In the case of carrying out this treatment step, one primer of a primer set used in the co-amplification of Step (b) phosphorylated at the 5'-end must be used. During the λ exonuclease reaction, the reaction conditions such as the reaction solution composition containing amplified fragments, buffer, and enzymes, reaction temperature, reaction time, and enzyme inactivation conditions are not particularly limited and, for example, the reaction conditions can be suitably set in accordance with the recommended condition of a commercial λ exonuclease. The λ exonuclease treatment can also be carried out directly without a purification step of the product after the co-amplification reaction in Step (b).

4. Step (c)

The present invention comprises the step of determining the total amount of all target nucleic acids and the specific nucleic acid group in a sample based on an indicator of the total amount of the amplified all target nucleic acids and the specific nucleic acid group and an indicator of an amount of the amplified control nucleic acid (Step (c)) (Figure 4C). The "amplified all target nucleic acids and the specific nucleic acid group" and the "amplified control nucleic acid" herein respectively refer to, of the amplified products co-amplified as described above, the amplified products from all target nucleic acids and the specific nucleic acid group and the amplified products from the control nucleic acid.

This step preferably comprises the step of creating in advance a calibration curve of relationships, using a part or all of the target nucleic acids and the specific nucleic acid group and the control nucleic acid, between amounts before amplification and indicators of amounts after amplification of the total of a part or all of the target nucleic acids and the specific nucleic acid group and the control nucleic acid (Step (c1)). This is because the relationship between the total amount of all target nucleic acids and the specific nucleic acid group and the amount of the control nucleic acid changes before and after the amplification when the amplification efficiencies of the both vary.

In Patent Literature 1, for nucleic acids equivalent to a bacterial 16SrRNA gene and a control nucleic acid, an analysis is carried out in which a ratio of indicators of amounts after amplification is construed as a ratio of amounts before amplification or a value proportional thereto. However, such a construe is applicable only when amplification efficiencies of the both are the same. Additionally, in the control nucleic acid, an artificially designed sequence is used and it is difficult to bring its amplification efficiency to be the same as amplification efficiencies of all target nucleic acids and thus the presumption that a ratio of indicators of amounts after amplification is equivalent to a ratio of amounts before amplification is not most likely established.

In the present invention, a calibration curve is obtained in advance, and based on the calibration curve, the total amount of all target nucleic acids and the specific nucleic acid group is determined thereby enabling more accurate quantification.

The indicator of an amount herein refers to, for example, a signal intensity of a corresponding probe in the case of a DNA chip and, for example, a read length of a corresponding sequence in the case of NGS.

For nucleic acids used for creating a calibration curve in the present invention, a part or all of target nucleic acids and the specific nucleic acid group can be used. Hereinafter, the "a part or all of target nucleic acids and the specific nucleic acid group" used for creating a calibration curve is called "the nucleic acids for calibration."

For the nucleic acids for calibration in the present invention, for example, when target nucleic acids are the 16SrRNA genes of two or more species of specific bacteria and the specific nucleic acid group is the bacterial 16SrRNA gene, a 16SrRNA gene comprised as a partial sequence in a genome DNA of a certain single bacterial species or a 16SrRNA gene comprised as a partial sequence in a mixture of genome DNA of a plurality of bacterial species can be used. The known sequence information on the full length of these bacterial 16SrRNA genes or a partial sequence comprising a site to be amplified is obtained and synthetic nucleic acids of these sequences can also be used as nucleic acids for calibration. The bacteria herein can be selected from bacteria having the 16SrRNA gene to be the target nucleic acid or can be selected from bacteria other than those. Alternatively, such a bacterium can be selected from both of them. That is, in the present invention, all of the nucleic acids to be target nucleic acids do not need to be used for creating a calibration curve and a part of the types of target nucleic acids and/or the specific nucleic acid group can be used. Thus, when the target nucleic acid contained in a sample are multiple types, a workload to create a calibration curve using all of these can be obviated thereby making the method simple.

Creation of a calibration curve is desirably carried out in a range wherein the concentration condition of the nucleic acids for calibration includes the total amount of all target nucleic acids and the specific nucleic group supposedly contained in a sample. For example, in the measurement of the 16SrRNA genes in saliva, when a sample is measured in such a way that 100 pg of the DNA extracted from the saliva is contained in an amplification reaction solution, an amount of substance of the 16SrRNA genes when the full 100 pg is supposedly bacterial genome DNA can be estimated. For example, given that the bacteria in saliva have an average genome molecular weight of $2.2 \times 10^6$ and an average number of 16SrRNA copies in 1 genome of 4.5, an amount of substance of the 16SrRNA gene in 100 pg of the bacterial genome DNA is estimated about 0.31 amol. The average genome molecular weight of the bacteria in saliva and the average number of 16SrRNA copies in 1 genome used herein for the above estimation are values estimated by the present inventors from the average of data on the bacterial composition in saliva of healthy person reported in Oral Dis. 21, 748-54 (2015) and the information of each bacterium.

Additionally, according to Microbiome 6, 42 (2018) (https://doi.org/10.1186/s40168-018-0426-3), about 86% to 97% of the DNA extracted from saliva is human genome DNA, in other words, bacteria-derived DNA is estimated about 3% to 14%. Thus, the concentration condition of the nucleic acids for calibration in the reaction solution, when creating a calibration curve, can be determined so that the estimated concentration of bacteria-derived DNA is included. The calibration curve creation needs to be carried out under at least two or more concentration conditions. The maximum concentration condition in this case can be, for example, a concentration condition selected from 0.050 pM to 500 pM when an amplification reaction solution volume is 20 μL. It is more preferably a concentration condition selected from 0.050 pM to 50 pM. The minimum concentration condition can be, for example, a concentration condition selected from 0.10 aM to 0.10 fM when an amplification reaction solution volume is 20 μL. It is more preferably a concentration condition selected from 0.010 fM to 0.10 fM.

It is particularly preferable for the calibration curve to represent the relationships between [a ratio of (an indicator of the total amount of a part or all target nucleic acids and a specific nucleic acid group) to (an indicator of an amount of a control nucleic acid after amplification)] and [a ratio of (the total amount of a part or all target nucleic acids and the specific nucleic acid group) to (an amount of the control nucleic acid before amplification)]. These relationships are regression-analyzed and usable as a calibration curve. The simplest method is linear regression but other non-linear regression using non-linear models can also be used. When the amplification efficiencies of a control nucleic acid are the same as those of the total of a part or all target nucleic acids and the specific nucleic acid group are supposedly the same, this relational expression is represented by a linear expression. However, amplification efficiencies are often different in reality. Such a case plots a curved relationship but, for example, can be approximated by a linear expression when double logarithm is applied.

In other words, when an indicator of the total amount of amplified all target nucleic acids and the specific nucleic acid group is represented by $I_T$, an indicator of an amount of a control nucleic acid is represented by Ic, the total amount of all target nucleic acids and the specific nucleic acid group before amplification is represented by $C_T$, and an amount of the control nucleic acid before amplification is represented by Cc, the relationship of these values can be approximated by the expression below.

$$\log_{10}(I_T \div I_C) = a \times \log_{10}(C_T \div C_C) + b \cdots \text{(Expression 1)}$$

The coefficients a and b can be determined from experiment data for creating a calibration curve by using, for example, LINEST function in a Microsoft software "EXCEL".

Alternatively, a sigmoidal non-linear regression calibration curve can also be created. For example, when PCR method is used as the amplification reaction, both of the mechanism: an individual mechanism occurring partially between the total of all target nucleic acids and the specific nucleic acid group and a control nucleic acid and a mechanism occurring to the entire nucleic acids of the system, contribute to a saturation of amplification. Due to the combination of these mechanisms, [a ratio of (an indicator of the total amount of a part or all target nucleic acids and the specific nucleic acid group) to (an indicator of an amount of a control nucleic acid after amplification)] to changes of [a ratio of (the total amount of a part or all target nucleic acids and the specific nucleic acid group) to (an amount of the control nucleic acid before amplification)] changes in a non-monotonous manner. In this case, the use of a non-linear calibration curve enables highly accurate quantification in a wide concentration range.

The function is not particularly restricted as long as it gives a common sigmoidal curve and, for example, when an indicator of the total amount of amplified all target nucleic acids and the specific nucleic acid group is represented by $I_T$, an indicator of an amount of a control nucleic acid is represented by $I_C$, the total amount of all target nucleic acids and the specific nucleic acid group before amplification is represented by $C_T$, and an amount of the control nucleic acid before amplification is represented by Cc, the relationship of these values can be approximated by the expression below.

$$\log10(I_T \div I_C) = b \times (\log10(C_T \div C_C) - c) \div (a - |\log10(C_T \div C_C) - c|) \cdots \text{(Expression 2)}$$

The coefficients a, b, and c can be determined from experiment data for creating a calibration curve by regression using, for example, a tool called "SOLVER" in a Microsoft software "EXCEL".

Descriptions of the symbols used in the present description are shown below. $I_T$: Indicator of the total amount of amplified all target nucleic acids and the specific nucleic acid group (Intensity, Total bacteria)

$I_C$: Indicator of an amount of the amplified control nucleic acid (Intensity, Control)
$C_T$: Total amount of all target nucleic acids and the specific nucleic acid group before amplification (Concentration, Total bacteria)
Cc: An amount of a control nucleic acid before amplification (Concentration, Control)
$C_{TM}$: Measured value of the total amount of all target nucleic acids and the specific nucleic acid group before amplification (Concentration, Total bacteria, Measurement)
$I_{TM}$: Measured value of an indicator of the total amount of amplified all target nucleic acids and the specific nucleic acid group (Intensity, Total bacteria, Measurement)
$I_{CM}$: Measured value of an indicator of an amount of the amplified control nucleic acid (Intensity, Control, Measurement)
Ius: Signal intensity of probe (x) from each target nucleic acid S (Intensity, Unique, Species) (S = A, B, ...)
$I_{TS}$: Signal intensity of probe (y) from each target nucleic acid S (Intensity, Total bacteria, Species) (S = A, B, ...)
Es: Coefficient in each target nucleic acid S (Efficiency, Species) (S = A, B, ···)

$I_{TSM}$: Measured value of a value equivalent to a signal intensity of probe (y) from each target nucleic acid S (Intensity, Total bacteria, Species, Measurement) (S = A, B, ···)

$I_{USM}$: Measured value of a signal intensity of probe (x) corresponding to each target nucleic acid S (Intensity, Unique, Species, Measurement) (S = A, B, ···)

Rs: Occupancy of each target nucleic acid S (Rate, Species) (S = A, B, ···)

Step (c) in the present invention preferably comprises the step (Step (c2)) of calculating a total amount of all target nucleic acids and the specific nucleic acid group in a sample by applying an indicator of the total amount of the amplified all target nucleic acids and the specific nucleic acid group to be collectively amplified with the target nucleic acids from the sample and an indicator of an amount of the control nucleic acid to the calibration curve created in the previous step.

In this step, for example, when an indicator of the total amount of amplified all target nucleic acids and the specific nucleic acid group from the sample is represented by $I_{TM}$ and an indicator of an amount of a control nucleic acid is represented by $I_{CM}$ and are applied to the calibration curve created in the above Expression 1, a total amount of all target nucleic acids and the specific nucleic acid group before amplification $C_{TM}$ (that is, in a sample) can be calculated using the expression below.

$$C_{TM} = C_C \times 10 \text{ \^{} } ((\log_{10}(I_{TM} \div I_{CM}) - b) \div a) \cdots \text{(Expression 3)}$$

A sigmoidal calibration curve can also be calculated in the same manner when the indicators are applied to an approximate expression thereof. For example, when an indicator of the total amount of amplified all target nucleic acids and the specific nucleic acid group is represented by $I_{TM}$ and an indicator of an amount of a control nucleic acid is represented by $I_{CM}$ and are applied to the calibration curve created in the above Expression 2, a total amount of all target nucleic acids and the specific nucleic acid group before amplification $C_{TM}$ (that is, in a sample) can be calculated using the expression below.

$$C_{TM} = C_C \times 10 \text{ \^{} } (a \times \log10(I_{TM} \div I_{CM}) \div (b + |\log10\ (I_{TM} \div I_{CM})|) + c) \cdots \text{(Expression 4)}$$

5. <u>Step (d)</u>

The present invention comprises the step of calculating an occupancy of each target nucleic acid in the total of all target nucleic acids and the specific nucleic acid group based on an indicator of the total amount of amplified all target nucleic acids and the specific nucleic acid group and an indicator of an amount of each amplified target nucleic acid thereby calculating an absolute amount of each target nucleic acid in a sample based on this occupancy (Step (d)) (Figure 4D).

The "each target nucleic acid" in the present invention means a respective plurality types of target nucleic acids contained in a sample or amplified products.

In this step, an absolute amount of the total of all target nucleic acids and the specific nucleic acid group obtained in Step (c) is multiplied by an occupancy thereby to determine an absolute amount of each target nucleic acid in a sample.

The present invention comprises separately the step of determining the total amount of all target nucleic acids and the specific nucleic acid group from the step of determining an occupancy of each target nucleic acid. In the present invention, the occupancy of each target nucleic acid in the total of all target nucleic acids and the specific nucleic acid group is, for example, when the indicator of an amount is a read length of NGS, a ratio of a read length of the amplified product of each target nucleic acid to a read length of amplified products of all target nucleic acids and the specific nucleic acid group.

When a DNA chip is used in the present invention, the indicators of amounts are signal intensities of the following probes (x) to (z):

(x) a probe hybridizable respectively with amplified products of two or more types of target nucleic acids;

(y) a probe hybridizable in common with both amplified products of all target nucleic acids and a specific nucleic acid group in a sample; and

(z) a probe hybridizable with amplified products of the control nucleic acid.

As the probe (x) herein, a probe that is hybridizable specifically with respective amplified products of target nucleic acids is preferable.

Detailed description of a DNA chip will be described later.

In Step (d), the calculation of an occupancy is preferably carried out by the following Steps (d1) to (d3):

(d1) hybridizing two or more types of respective target nucleic acids (each target nucleic acid) with a DNA chip in advance and calculating a relational expression on signal intensities between the probe (x) and the probe (y) hybridizable with each target nucleic acid;

(d2) converting the signal intensity of the probe (x) obtained by hybridizing products amplified from a sample with the DNA chip to a value equivalent to the signal intensity of the probe (y) hybridizable with each target nucleic acid using the relational expression calculated in the above (d1); and

(d3) calculating a ratio of the value equivalent to the signal intensity of the probe (y) calculated in the above (d2) to the signal intensity of the probe (y) obtained by hybridizing products amplified from the sample with the DNA chip.

The target nucleic acid used herein in Step (d1) can be, for example, artificially synthesized nucleic acids or biological nucleic acids such as those extracted from an isolated bacterium as long as it is bacterial genome DNA.

A DNA chip, even with the same amount of nucleic acids to be hybridized, could cause different signal intensities depending on hybridization efficiencies when the type of probe varies or different various conditions. That is, the determination of an occupancy of each target nucleic acid in the total of all target nucleic acids and the specific nucleic acid group from the signal intensities of the probe (x) and the probe (y) requires the conversion of the signal intensity of the probe (x) to a value equivalent to the signal intensity of the probe (y) (Step (d2)). For such an achievement, a relational expression on the signal intensities between the probe (x) and the probe (y) needs to be obtained in advance in the above step of (d1). This relational expression can be suitably determined from experiments and can be, for example, approximated by a proportional relationship.

In other words, when a signal intensity of the probe (x) hybridizable with a single target nucleic acid S (S is an index of the type of each target nucleic acid) is represented by Ius and a signal intensity of the probe (y) is represented by $I_{TS}$, the relationship between $I_{US}$ and $I_{TS}$ can be approximated by, for example, the expression below.

$$I_{TS} = E_S \times I_{US} \cdots \text{(Expression 5)}$$

This coefficient Es can be determined from the signals of the respective probes obtained in Step (d1) using, for example, LINEST function in a Microsoft software "EXCEL".

Here, when the signal intensity of the probe (x) corresponding to each target nucleic acid S obtained by hybridizing the products amplified from the sample with a DNA chip is represented by $I_{USM}$ and the signal intensity of the probe (y) is represented by $I_{TM}$, the value equivalent to the signal intensity of the probe (y) from each target nucleic acid $I_{TSM}$ to be determined in Step (d2) is represented by the expression below.

$$I_{TSM} = E_S \times I_{USM} \cdots \text{(Expression 6)}$$

Further, here, the occupancy Rs of each target nucleic acid S can be calculated using the expression below (Step (d3)).

$$R_S = I_{TSM} \div I_{TM} \cdots \text{(Expression 7)}$$

In the present invention, Step (d) further comprises the step (Step (d4)) of calculating an absolute amount of each target nucleic acid in the sample by multiplying the total amount of all target nucleic acids and the specific nucleic acid group in the sample calculated in Step (c) by the occupancy calculated in Step (d3).

In this step, an absolute amount of each target nucleic acid in the sample can be calculated by multiplying the amount of the nucleic acid group calculated in Step (c) by the occupancy calculated in Step (d3).

6. DNA chip

In the present invention, the DNA chip is not limited to any device as long as it is immobilized in such a way that the probes are independent and the position can be specified, and, as a wider dynamic range is demanded for bacterial florae analysis, an array having a large amount of probe immobilization is more suitable than a chip with probes immobilized simply on a planar substrate. Examples of such a DNA chip particularly include a fiber-type DNA chip on which probes are three-dimensionally immobilized through a gel.

The form of a support of a DNA chip is not particularly limited and any forms such as flat plates, rods, and beads can be used. When a flat plate is used as the support, predetermined probes by the type can be immobilized on a flat plate at a predetermined interval (spotting method and the like; see Science 270, 467-470 (1995) and the like). Additionally, predetermined probes can be successively synthesized by the type at a specific position on the flat plate (photolithography method and the like; see Science 251, 767-773 (1991) and the like).

Examples of other preferable form of a support include those using hollow fibers. When a hollow fiber is used as the support, a preferable example includes a device obtained by immobilizing oligonucleotide probes by the type on each hollow fiber, bundling and immobilizing all hollow fibers and subsequently repeating cutting of the fibers in a longitudinal direction. This device can be described as the type of chip in which oligonucleotide probes are immobilized on a through-hole substrate (see Figure 1 and the like of Japanese Patent No. 3510882).

The method for immobilizing probes on the substrate is not limited and can be any binding mode. Further, the method is not limited to direct immobilization on the support and, for example, a support is coated in advance with a polymer such as polylysine and probes can also be immobilized on the treated support. Furthermore, when a tubular body such as hollow fibers is used as the support, a gel-like substance is retained in the tubular body and probes can also be immobilized on the gel-like substance.

7. Probe

A probe captures a nucleic acid through hybridization and DNA hybridizable with a sequence specific to the nucleic acid to be captured can be used.

Typically, when a DNA chip collectively containing multiple types of probes is used, it is desirable to arrange the hybridization efficiencies among the probes as much as possible so that the multiple types of probes are subjected to the hybridization reaction under the same conditions. For this purpose, when designing probes, GC contents and sequence lengths must be adjusted and Tm values need to be arranged to be constant within a possible range. The type and number of probes loaded on a DNA chip are not particularly limited.

In the present invention, the sequence of a probe is preferably a sequence consisting of a complementary sequence to a specific base sequence to be captured.

In the present invention, the probe (the above probe (x)) hybridizable with respective amplified products of two or more types of target nucleic acids is preferably, for example, when capturing the 16SrRNA genes of Achromobacter xylosoxidans, Acinetobacter baumannii, Acinetobacter calcoaceticus, Acinetobacter lwoffii, Actinomyces graevenitzii, Actinomyces israelii, Actinomyces meyeri, Actinomyces naeslundii, Actinomyces odontolyticus, Actinomyces turicensis, Actinomyces viscosus, Aggregatibacter actinomycetemcomitans, Alloprevotella rava, Alloprevotella sp.OT 308, Atopobium parvulum, Bacteroides fragilis, Burkholderia cepacia, Campylobacter concisus, Campylobacter gracilis, Campylobacter rectus, Campylobacter showae, Capnocytophaga gingivalis, Capnocytophaga ochracea, Capnocytophaga sputigena, Chlamydophila (Chlamydia) pneumoniae, Chryseobacterium indologenes, Citrobacter freundii, Corynebacterium matruchotii, Corynebacterium striatum, Eikenella corrodens, Enterobacter aerogenes, Enterobacter cloacae, Enterobacter cloacae subsp. cloacae, Enterobacter cloacae subsp. dissolvens, Enterococcus faecium, Escherichia coli, Eubacterium nodatum, Eubacterium saphenum, Eubacterium sulci, Filifactor alocis, Fusobacterium necrophorum subsp. funduliforme, Fusobacterium necrophorum subsp. necrophorum, Fusobacterium nucleatum, Fusobacterium nucleatum subsp. animalis, Fusobacterium nucleatum subsp. polymorphum, Fusobacterium nucleatum subsp. vincentii, Fusobacterium periodonticum, Gemella sanguinis, Granulicatella adiacens, Haemophilus influenzae, Haemophilus parainfluenzae, Helicobacter pylori, Klebsiella pneumoniae subsp. pneumoniae, Lactobacillus acidophilus, Lactobacillus casei, Lactobacillus salivarius, Legionella pneumophila, Legionella pneumophila subsp. pneumophila str. Philadelphia, Leptotrichia sp. OT 215, Leptotrichia sp. OT 417, Leptotrichia sp. OT 462, Megasphaera micronuciformis, Moraxella catarrhalis, Morganella morganii, Mycobacterium avium, Mycobacterium intracellulare, Mycobacterium kansasii, Mycoplasma pneumoniae, Neisseria flavescens, Nocardia brasilliensis, Parvimonas micra, Peptostreptococcus stomatis, Porphyromonas catoniae, Porphyromonas endodontalis, Porphyromonas gingivalis, Porphyromonas pasteri, Prevotella dentalis, Prevotella denticola, Prevotella histicola, Prevotella intermedia, Prevotella loescheii, Prevotella melaninogenica, Prevotella nigrescens, Prevotella oris, Prevotella pallens, Prevotella shahii, Prevotella veroralis, Propionibacterium acnes (Cutibacterium acnes), Proteus mirabilis, Proteus vulgaris, Pseudomonas aeruginosa, Rothia dentocariosa, Rothia mucilaginosa, Selenomonas noxia, Selenomonas sp. OT 478, Selenomonas sputigena, Serratia marcescens, Serratia marcescens subsp. marcescens, Solobacterium moorei, SRI sp. OT 345, Staphylococcus aureus, Staphylococcus epidermidis, Stenotrophomonas maltophilia, Streptococcus agalactiae, Streptococcus anginosus, Streptococcus constellatus, Streptococcus dentisani, Streptococcus gordonii, Streptococcus intermedius, Streptococcus mitis, Streptococcus mutans, Streptococcus parasanguinis, Streptococcus pneumoniae, Streptococcus pyogenes, Streptococcus salivarius, Streptococcus sobrinus, Tannerella forsythia, Treponema denticola, Treponema medium, Treponema socranskii, Veillonella atypica, Veillonella parvula, *Veillonella rogosae,* and the genera of *Streptococcus,* a sequence selected from the group consisting of the base sequences as set forth in SEQ ID NO: 3 to 191. In this case, such a probe can be a complementary sequence to the sequence selected from the group consisting of the base sequences as set forth in SEQ ID NO: 3 to 191 or can be a sequence substantially identical with the sequence selected from the group consisting of the base sequences as set forth in SEQ ID NO: 3 to 191 or a complementary sequence thereto.

The substantially identical sequence herein refers to a sequence hybridizable under stringent condition with the sequence selected from the group consisting of the base sequences as set forth in SEQ ID NO: 3 to 191 or a complementary sequence thereto.

The length of probe is, for example, a sequence of 13 bases or more, preferably 15 bases or more, and more preferably 17 bases or more. The stringent condition herein means a condition under which a specific hybrid is formed and a non-specific hybrid is not formed. That is, the stringent condition refers to a condition under which a pair of polynucleotides having a high homology (a homology or identity of 95% or more, preferably 96% or more, more preferably 97% or more, further preferably 98% or more, and most preferably 99% or more) is hybridized.

The stringent condition in the present invention can be any of a low stringent condition, a moderate stringent condition, and a high stringent condition. The "low stringent condition" is a condition of, for example, 5 × SSC, 5 × Denhardt's solution, 0.5% SDS, 50% formamide, and 32°C. The "moderate stringent condition" is a condition of, for example, 5 × SSC, 5 × Denhardt's solution, 0.5% SDS, 50% formamide, and 42°C. The "high stringent condition" is a condition of, for example, 5 × SSC, 5 × Denhardt's solution, 0.5% SDS, 50% formamide, and 50°C. Under these conditions, at a higher temperature, it can be expected to efficiently capture a DNA having a higher homology. It is conceivable that a plurality of elements affect the stringency of hybridization such as temperature, probe concentration, probe length, ion intensity, time, and salt concentration but a person skilled in the art can achieve the equivalent stringency by suitably selecting these elements. Detailed procedures of hybridization method can be referred to "Molecular Cloning, A Laboratory Manual (4th edition)" (Cold Spring Harbor Laboratory Press (2012)) and the like.

Further, for the stringent condition, conditions employed by well-known routine techniques in the filed such as Northern blotting method, dot blot method, colony hybridization method, plaque hybridization method, or Southern blotting hybridization method can be set.

In the present invention, the probe (the above probe (y)) hybridizable in common with both amplified products of all target nucleic acids and the specific nucleic acid group is preferably a complementary sequence to a sequence comprised commonly in the amplified products of the sequences comprised in all target nucleic acids and the specific nucleic acid group. For example, the probe, when capturing bacterial 16SrRNA genes, can be a part of a complementary strand of a consensus sequence of the bacterial 16SrRNA genes. Examples of the probe in this case include a probe comprising the base sequence as set forth in SEQ ID NO: 2.

In the present invention, the probe (the above probe (z)) hybridizable with amplified products of the control nucleic acid is preferably a part of a complementary sequence of amplified products of the control nucleic acid. For example, when the base sequence as set forth in SEQ ID NO: 196 is used as the sequence of a control nucleic acid, examples of the probe include a probe comprising the base sequence as set forth in SEQ ID NO: 1.

8. Kit

The present invention provides a kit comprising, for achieving the above quantitative technique, one or two pairs of primer sets for co-amplifying all target nucleic acids and the specific nucleic acid group and a control nucleic acid by PCR method, a control nucleic acid, and a DNA chip on which the above probes (x) to (z) are loaded.

When the target nucleic acids are the 16SrRNA genes of, for example, Achromobacter xylosoxidans, Acinetobacter baumannii, Acinetobacter calcoaceticus, Acinetobacter lwoffii, Actinomyces graevenitzii, Actinomyces israelii, Actinomyces meyeri, Actinomyces naeslundii, Actinomyces odontolyticus, Actinomyces turicensis, Actinomyces viscosus, Aggregatibacter actinomycetemcomitans, Alloprevotella rava, Alloprevotella sp.OT 308, Atopobium parvulum, Bacteroides fragilis, Burkholderia cepacia, Campylobacter concisus, Campylobacter gracilis, Campylobacter rectus, Campylobacter showae, Capnocytophaga gingivalis, Capnocytophaga ochracea, Capnocytophaga sputigena, Chlamydophila (Chlamydia) pneumoniae, Chryseobacterium indologenes, Citrobacter freundii, Corynebacterium matruchotii, Corynebacterium striatum, Eikenella corrodens, Enterobacter aerogenes, Enterobacter cloacae, Enterobacter cloacae subsp. cloacae, Enterobacter cloacae subsp. dissolvens, Enterococcus faecium, Escherichia coli, Eubacterium nodatum, Eubacterium saphenum, Eubacterium sulci, Filifactor alocis, Fusobacterium necrophorum subsp. funduliforme, Fusobacterium necrophorum subsp. necrophorum, Fusobacterium nucleatum, Fusobacterium nucleatum subsp. animalis, Fusobacterium nucleatum subsp. polymorphum, Fusobacterium nucleatum subsp. vincentii, Fusobacterium periodonticum, Gemella sanguinis, Granulicatella adiacens, Haemophilus influenzae, Haemophilus parainfluenzae, Helicobacter pylori, Klebsiella pneumoniae subsp. pneumoniae, Lactobacillus acidophilus, Lactobacillus casei, Lactobacillus salivarius, Legionella pneumophila, Legionella pneumophila subsp. pneumophila str. Philadelphia, Leptotrichia sp. OT 215, Leptotrichia sp. OT 417, Leptotrichia sp. OT 462, Megasphaera micronuciformis, Moraxella catarrhalis, Morganella morganii, Mycobacterium avium, Mycobacterium intracellulare, Mycobacterium kansasii, Mycoplasma pneumoniae, Neisseria flavescens, Nocardia brasilliensis, Parvimonas micra, Peptostreptococcus stomatis, Porphyromonas catoniae, Porphyromonas endodontalis, Porphyromonas gingivalis, Porphyromonas pasteri, Prevotella dentalis, Prevotella denticola, Prevotella histicola, Prevotella intermedia, Prevotella loescheii, Prevotella melaninogenica, Prevotella nigrescens, Prevotella oris, Prevotella pallens, Prevotella shahii, Prevotella veroralis, Propionibacterium acnes (Cutibacterium acnes), Proteus mirabilis, Proteus vulgaris, Pseudomonas aeruginosa, Rothia dentocariosa, Rothia mucilaginosa, Selenomonas noxia, Selenomonas sp. OT 478, Selenomonas sputigena, Serratia marcescens, Serratia marcescens subsp. marcescens, Solobacterium moorei, SR1 sp. OT 345, Staphylococcus aureus, Staphylococcus epidermidis, Stenotrophomonas maltophilia, Streptococcus agalactiae, Streptococcus anginosus, Streptococcus constellatus, Streptococcus dentisani, Streptococcus gordonii, Streptococcus intermedius, Streptococcus mitis, Streptococcus mutans, Streptococcus parasanguinis, Streptococcus pneumoniae, Streptococcus pyogenes, Streptococcus salivarius, Streptococcus sobrinus, Tannerella forsythia, Treponema denticola, Treponema medium, Treponema socranskii, Veillonella atypica, Veillonella parvula, Veillonella rogosae, and the genera of *Streptococcus,* the probe (x) is a sequence selected from the group consisting of the base sequences as set forth in SEQ ID NO: 3 to 191. In this case, the probe can be a complementary sequence to the sequence selected from the group consisting of the base sequences as set forth in SEQ ID NO: 3 to 191 or can be a sequence substantially identical with the sequence selected from the group consisting of the base sequences as set forth in SEQ ID NO: 3 to 191 or a complementary sequence thereto. The "sequence substantially identical with" is as defined as described above.

Additionally, the present invention provides a DNA chip on which the above probes (x) to (z) are loaded, wherein the probe (x) is a sequence selected from the group consisting of the base sequences as set forth in SEQ ID NO: 43 to 191, a complementary sequence to the sequence selected from the group consisting of the base sequences as set forth in SEQ ID NO: 43 to 191, or a sequence substantially identical with the sequence selected from the group consisting of the base sequences as set forth in SEQ ID NO: 43 to 191 or a complementary sequence thereof.

The probes used in the present invention are shown in Tables 1 below.

Table 1

[Table 1-1]

| SEQ ID NO | Name | Probe sequence |
|---|---|---|
| 1 | Probe for control nucleic acid | CTATTCGACCAGCGATATCACTACGTAGGC |
| 2 | Probe for total 16SrRNA genes | CGTATTACCGCGGCTGCTGGCAC |
| 3 | Probe for *Aggregatibacter actinomycetemcomitans* | GTCAATTTGGCATGCTATTAACACACC |
| 4 | Probe *for Aggregatibacter actinomycetemcomitans* | GTCAAGTTGGCATGCTATTAACACACC |
| 5 | Probe for *Campylobacter rectus* | GTCATAATTCTTTCCCAAGA |
| 6 | Probe for *Capnocvtophaaa gingivalis* | TACACGTACACCTTATTCTT |
| 7 | Probe for *Fusobacterium nucleatum* | TACATTCCGAAAAACGTCAT |
| 8 | Probe for *Porphyromonas gingivalis* | TTCAATGCAATACTCGTATC |
| 9 | Probe for *Prevotella intermedia* | CGAAGGGTAAATGCAAAGGGGC |
| 10 | Probe for *Prevotella intermedia* | CGAAGGGTAAATGCAAAAAGGC |
| 11 | Probe for *Prevotella niarescens* | CTTTATTCCCACATAAAAGC |
| 12 | Probe for *Selenomonas noxia* | TTCGCATTAGGCACGTTC |
| 13 | Probe for *Streptococcus constellatus* | AAGTACCGTCACTGTGTG |
| 14 | Probe for *Streptococcus gordonii* | CACCCGTTCTTCTCTTACA |
| 15 | Probe for *Streptococcus intermedius* | ACAGTATGAACTTTCCATTCT |
| 16 | Probe for *Tannerella forsythia* | CACGTATCTCATTTTATTCCCCTGT |
| 17 | Probe for *Treponema denticola* | CCTCTTCTTCTTATTCTTCATCTGC |
| 18 | Probe for *Veillonella parvula* | TCCTTCTAACTGTTCGC |

(continued)

| SEQ ID NO | Name | Probe sequence |
|---|---|---|
| 19 | Probe for *Actinomyces odontolyticus* | AAGTCAGCCCGTACCCA |
| 20 | Probe for *Actinomyces viscosus* | CCACCCACAAGGAGCAG |
| 21 | Probe for *Aggregatibacter actinomycetemcomitans* | |
| 22 | Probe for *Campylobacter concisus* | CCCAAGCAGTTCTATGGT |
| 23 | Probe for *Campylobacter gracilis* | GCCTTCGCAATAGGTATT |
| 24 | Probe for *Campylobacter showae* | CAATGGGTATTCTTCTTGAT |
| 25 | Probe for *Capnocytophaga ochracea* | CAACCATTCAAGACCAACA |
| 26 | Probe for *Capnocytophaga sputigena* | TCAAAGGCAGTTGCTTAGT |
| 27 | Probe *for Eikenella corrodens* | CTCTAGCTATCCAGTTCAG |
| 28 | Probe for *Fusobacterium nucleatum subsp. animalis* | TTTCTTTCTTCCCAACTGAA |
| 29 | Probe for *Fusobacterium nucleatum subsp. polymorphum* | CCAGTACTCTAGTTACACA |
| 30 | Probe for *Fusobacterium nucleatum subsp. vincentii* | TAGTTATACAGTTTCCAACG |
| 31 | Probe for *Fusobacterium periodonticum* | TATGCAGTTTCCAACGCAA |
| 32 | Probe for *Lactobacillus salivarius* | GCCGTGACTTGCTGGTT |
| 33 | Probe for *Lactobacillus casei* | CTGTCCTCTTCTGCACT |
| 34 | Probe for *Prevotella intermedia* | GGGTAAATGCAAAAAGGCA |
| 35 | Probe for all *Streptococcus* | TTAGCCGTCCCTTTCTGG |
| 36 | Probe for *Streptococcus dentisani* | TCCCCTCTTGCACTCAAGT |
| 37 | Probe for *Streptococcus mitis* | TCTCCCCTCTTGCACTCA |
| 38 | Probe for *Streptococcus mutans* | CACACGTTCTTGACTTAC |
| 39 | Probe for *Streptococcus sobrinus* | CCGTCACTGTGTAAGCTT |
| 40 | Probe for *Tannerella forsythia* | CACGTATCTCATTTTATTCC |
| 41 | Probe *for Treponema denticola* | CCTCTTCTTCTTATTCTTCAT |
| 42 | Probe for *Veillonella parvula* | TATTCGCAAGAAGGCCTT |
| 43 | Probe for *Actinomyces graevenitzii* | AAAAAGCAGTGCCTTGTTCC |
| 44 | Probe for *Actinomyces israelii* | GCGCTTCATAACCCGGCTAC |
| 45 | Probe for *Actinomyces naeslundii* | AACCCACCCACAAACGA |
| 46 | Probe for *Alloprevotella rava, Alloprevotella sp.OT 308* | TTCCCAACTAAAAGCAGTTTA |
| 47 | Probe for *Corynebacterium matruchotii* | TCTTAACAAAGGTACCGTCACC |
| 48 | Probe for *Eubacterium nodatum* | CCTACGCTTACTTAACCACCTA |
| 49 | Probe for *Eubacterium saphenum* | CCCTAGGACAGAGGCTTACA |
| 5C | Probe for *Eubacterium sulci* | AAACCCTGCGCTTAAGGTGC |
| 51 | Probe for *Filifactor alocis* | CCCCTACTACAGAGTTTTACGA |
| 52 | Probe for *Gemella sanguinis* | CCGTCTCTACTGTATATAGT |
| 53 | Probe for *Granulicatella adiacens* | GTCAAGGCGCTAACAGTTAC |
| 54 | Probe for *Haemophilus parainfluenzae* | AGTTAACGTCAATCACCTAG |

(continued)

| SEQ ID NO | Name | Probe sequence |
|---|---|---|
| 55 | Probe for *Helicobacter pylori* | GCGGGATAGTCAGTCAGGTG |
| 56 | Probe for *Lactobacillus acidophilus* | CAGTTTCCGATGCAGTTCC |
| 57 | Probe for *Leptotrichia spp. (OT 215,417,462)* | CGTGCAGTTCCGTCCACCTC |
| 58 | Probe for *Megasphaera micronuciformis* | TAACCACAAGATTATTCGTC |
| 59 | Probe for *Neisseria flavescens* | AGCTGTCGATATTAGCAACAG |
| 60 | Probe for *Parvimonas micra* | GTGCTTAATGAGGTTAAGCC |
| 61 | Probe for *Peptostreptococcus stomatis* | ACCACCGACTTGAAGGACCA |
| 62 | Probe for *Porphyromonas catoniae* | GGTACATTCACTATGGTACACG |
| 63 | Probe for *Porphyromonas endodontalis* | TACATGCATCTCAGCTACACGT |
| 64 | Probe for *Porphyromonas pasteri* | ACACGTGACTCTTGTTATTC |
| 65 | Probe for *Prevotella denticola* | AGTCAGACGTTGGGCGCCTA |

[Table 1-2]

| | | |
|---|---|---|
| 66 | Probe for *Prevotella histicola* | CACGTGACTGACTTTATCCC |
| 67 | Probe for *Prevotella loescheii* | CCTACTTTCAGCGCACTCAA |
| 68 | Probe for *Prevotella melaninogenica* | AATAGGGACACGTCCCTAAC |
| 69 | Probe for *Prevotella pallens* | CACGTGCATCAAATTATTCTCG |
| 70 | Probe for *Prevotella shahii* | ACGTGGGCTCTTTTATCCCC |
| 71 | Probe for *Prevotella veroralis* | ACACGTGATTGACTTTATCC |
| 72 | Probe for *Rothia dentocariosa* | ACCCACTGCAAAACCAGGGT |
| 73 | Probe for *Rothia mucilaainosa* | TCTCTTCTTCCCTGCTAACA |
| 74 | Probe for *Selenomonas sp. OT 478* | ACTATTCGCACTAGGCACGT |
| 75 | Probe for *Selenomonas sputigena* | GTACCGTCACCCAAACTCAATA |
| 76 | Probe for *Solobacterium moorei* | CCAACAATTTAACCACTTAC |
| 77 | Probe for *SR1 sp. OT* 345 | CGTCATTCGTCTTCTGCCAA |
| 78 | Probe for *Streptococcus parasanauinis* | CTGGTAAGTTACCGTCAC |
| 79 | Probe for *Streptococcus salivarius* | CACACTCGTTCTTGACTTAC |
| 80 | Probe for *Streptococcus sobrinus* | TACACACGTTCTTCCCCTAC |
| 81 | Probe for *Treponema medium* | GTCGATTACCGTCATCAGATG |
| 82 | Probe for *Treponema socranskii* | TTCCTCCAAAACTTATTCCT |
| 83 | Probe for *Veillonella atypica* | CGTCAAATCCTCGCACTATTC |
| 84 | Probe for *Veillonella rogosae* | ACCGTCAATTCCTCTAACTATT |
| 85 | Probe for *Achromobacter xylosoxidans* | CAGTTTCGCGAGGTATTAAC |
| 86 | Probe for *Achromobacter xylosoxidans* | CACCAGACGAAGTCCGTGCT |
| 87 | Probe for *Acinetobacter baumannii* | CTAGGTCCGGTAGCAAGCTACC |
| 88 | Probe for *Acinetobacter baumannii* | TACCTTCCCCCGCTCGACTT |
| 89 | Probe for *Acinetobacter calcoaceticus* | ATCAGTAGCAAGCTACCTCTCTC |

(continued)

| | | |
|---|---|---|
| 90 | Probe for *Acinetobacter calcoaceticus* | CGCTAAGATCAGTAGCAAGCTA |
| 91 | Probe for *Acinetobacter lwoffii* | GTATTAATCTCGGTAGCCTCC |
| 92 | Probe for *Acinetobacter lwoffii* | CTAGGTTAGGTAGCAAGCTACATT |
| 93 | Probe for *Actinomyces israelii* | CACCAAAACACCACAAAAGTGA |
| 94 | Probe for *Actinomyces israelii* | ACTAACCCACCCCGCAAAAA |
| 95 | Probe for *Actinomyces meyeri* | TCCAGTATTAGCACCCATTT |
| 96 | Probe for *Actinomyces odontolyticus* | ATGCGAAGATCAGTGAATATCC |
| 97 | Probe for *Actinomyces turicensis* | AAGTTGGAGCATCATCGTTC |
| 98 | Probe for *Actinomyces turicensis* | GCCAATGAGTGAATATCCAG |
| 99 | Probe for *Atopobium parvulum* | GGAAGTCTCGAAGTATTCGGT |
| 100 | Probe for *Atopobium parvulum* | GAGCTTTCTCGAGTCTTCCATG |
| 101 | Probe for *Bacteroides fraailis* | CGGAATCATTATGCTATCGGGT |
| 102 | Probe for *Bacteroides fragilis* | CAAGCTTTCTTCCTGATGCC |
| 103 | Probe for *Burkholderia cepacia* | TAGAGCCAAGGATTTCTTTCCG |
| 104 | Probe for *Burkholderia cepacia* | ACTACAGGACATGTTCCGAT |
| 105 | Probe for *Chryseobacterium indologenes* | CTCTCAAGATCCCGAAAGATC |
| 106 | Probe for *Chryseobacterium indologenes* | GTACGCCGCTCTCAAGATCC |
| 107 | Probe for *Citrobacter freundii* | TAACCACAACGCCTTCCTCC |
| 108 | Probe for *Citrobacter freundii* | GTCACCCAAGGAGCAAGCTC |
| 109 | Probe for *Corynebacterium striatum* | CTGACACTAAACAGTGGTCCTA |
| 110 | Probe for *Corynebacterium striatum* | TCGAGTACCCGCAGCAAGCT |
| 111 | Probe for *Eikenella corrodens* | CGGACAGTAGTGCAAGCACT |
| 112 | Probe for *Eikenella corrodens* | AATAACGCGAGGTCTTGCGA |
| 113 | Probe for *Enterobacter aerogenes* | TTAACCTTAACGCCTTCCTC |
| 114 | Probe for *Enterobacter aerogenes* | CCTCGCTGAAAGTACTTTACAA |
| 115 | Probe for *Enterobacter cloacae subsp. cloacae* | CGGTTATTAACCACAACACC |
| 116 | Probe for *Enterobacter cloacae* | AGCTCTCTGTGCTACCGTTC |
| 117 | Probe for *Enterobacter cloacae* | TCTCTGTGCTACCGTTCGAC |
| 118 | Probe for *Enterobacter cloacae subsp. dissolvens* | TCTTTGGTCTTGCGACGTTA |
| 119 | Probe for *Enterococcus faecium* | CTCTCAGGTGCGGCTATGCA |
| 120 | Probe for *Escherichia coli* | AGGTCCCCCTCTTTGTGCTT |
| 121 | Probe for *Escherichia coli* | CGTCAATGAGCAAAGGTATT |
| 122 | Probe for *Fusobacterium necrophorum subsp.rum* | GACATACGTAATTACCGCGG |
| 123 | Probe for *Fusobacterium necrophorum subsp.rum* | GCTTGCGACATACGTAATTA |
| 124 | Probe for *Fusobacterium subsp.ne* | ATGTTGTCCCGGTCTAAGAG |
| 125 | *necrophorum* Probe for *Leaionella pneumophila* | CTTCAAGGCATATTCCTACG |
| 126 | Probe for *Legionella pneumophila* | TTACAACCCTCAGGCCTTCT |
| 127 | Probe for *Leaionella pneumophila* | TTACAACCCTCAGGCCTTCTT |
| 128 | Probe for *Legionella pneumophila* | CCTCTTCAAGGCATATTCCTAC |

(continued)

| | | |
|---|---|---|
| 129 | Probe for *Leglionella pneumophila* | CAGTATTATCTGACCGTCCC |
| 130 | Probe for *Legionella pneumophila* | AGGCTAATCTTAAAGCGCCAGG |

[Table 1-3]

| | | |
|---|---|---|
| 131 | Probe for *Leaionella pneumophila subsp. pneumophila str. Philadelphia* | CAGCTTTCGTCCTCAGACATT |
| 132 | Probe for *Moraxella catarrhalis* | GCTTCCTAACTTCGTTCGAC |
| 133 | Probe for *Moraxella catarrhalis* | GTCAGGGCTTATGGGTATTA |
| 134 | Probe for *Morganella moraanii* | ACATCTGACTCAATCAACCGC |
| 135 | Probe for *Moraanella moraanii* | TATTAACCTTGACACCTTCCTC |
| 136 | Probe for *Mvcobacterium avium* | CAGAAGACATGCGTCTTGAG |
| 137 | Probe for *Mycobacterium avium* | GAGTACCTCCGAAGAGGCCTTT |
| 138 | Probe for *Mycobacterium intracellulare* | ATTGCCCACGTGTTACTCAC |
| 139 | Probe for *Mycobacterium intracellulare* | CTTCTTCTCCACCTACCGTC |
| 140 | Probe for *Mycobacterium kansasii* | CCACTCGAGTGTCTCCGAAG |
| 141 | Probe for *Mycobacterium kansasii* | AGGCTTATCCCGGTGTGCAG |
| 142 | Probe for *Nocardia brasilliensis* | ACCCAACAGCATGCACTGAAA |
| 143 | Probe for *Nocardia brasilliensis* | GTCAGTTACTTCCCAGAGAC |
| 144 | Probe for *Peptostreptococcus stomatis* | CCGCTTGGTTTTGCTCAGAATT |
| 145 | Probe for *Peptostreptococcus stomatis* | ACTCAATACTGAGCAAACCCTC |
| 146 | Probe for *Prevotella dentalis* | GAGGACCTCGTTGGAATACATA |
| 147 | Probe for *Prevotella dentalis* | CTGCTTGAAGGTAGGTTGGATA |
| 148 | Probe for *Prevotella melaninogenica* | CCCTAACTTTATCCCCATACA |
| 149 | Probe for *Prevotella melaninogenica* | GTATGGTACCTGCAAATAGG |
| 150 | Probe for *Prevotella oris* | CTTTAATCGTCGTTGGATGCC |
| 151 | Probe for *Prevotella oris* | AGACGATGCCATGAGGTATT |
| 152 | Probe for *Propionibacterium acnes* (*Cutibacterium acnes*) | TCTTTACCCATTACCGTCAC |
| 153 | Probe for *Propionibacterium acnes* (*Cutibacterium acnes*) | CCCAAGATTACACTTCCGAC |
| 154 | Probe for *Proteus mirabilis* | GATTAACGCCTGCACCCTCC |
| 155 | Probe for *Proteus mirabilis* | CGCCTGCACCCTCCGTATTA |
| 156 | Probe for *Proteus vulgaris* | TACCGCTCGATTGCATGTGT |
| 157 | Probe for *Proteus vulgaris* | AACTTTATCACCTTCCTCCC |
| 158 | Probe for *Staphylococcus epidermidis* | GCGGTTCAATATATTATCCG |
| 159 | Probe for *Staphylococcus epidermidis* | ATAAGTGACAGCAAAACCGT |
| 160 | Probe for *Stenotrophomonas maltophilia* | TTAGCCAGCTGGATTTCTTT |
| 161 | Probe for *Stenotrophomonas maltophilia* | CGACAGAGTAGATTCCGATG |
| 162 | Probe *Streptococcus agalactiae* | AGTCTAGTGTAAACACCAAACCTC |
| 163 | Probe for *Streptococcus agalactiae* | ACTCCTACCAACGTTCTTCTC |
| 164 | Probe for *Streptococcus anginosus* | CATCTACTAGCGATGCAATTG |

(continued)

| | | |
|---|---|---|
| 165 | Probe for *Streptococcus anainosus* | TTAAGTACCGTCACAGCATG |
| 166 | Probe for *Veillonella atypica* | ACCTTTCATCCAGTCTCGAT |
| 167 | Probe for *Veillonella atypica* | CGTCAAATCCTCGCACTATT |
| 168 | Probe for *Helicobacter pylori* | CACACCTGACTGACTATCCC |
| 169 | Probe for *Helicobacter pylori* | CCACTAATCAGCACTCTAGCA |
| 170 | Probe for *Helicobacter pylori* | AAACTAAGAGGCACATGACC |
| 171 | Probe for *Chlamydophila* (*Chlamydia*) *pneumoniae* | GTTTTAAATGCTGACTTGGGG |
| 172 | Probe for *Chlamydophila* (*Chlamydia*) *pneumoniae* | GCATAAACTCTTCCTCAACC |
| 173 | Probe for *Chlamydophila* (*Chlamydia*) *pneumoniae* | AACCGTTATCCCCAAGTTGA |
| 174 | Probe for *Haemophilus influenzae* | TCTCAGTCCCGCACTTTCAT |
| 175 | Probe for *Klebsiella pneumoniae subsp. pneumoniae* | TATTAACCTCATCGCCTTCC |
| 176 | Probe for *Klebsiella pneumoniae subsp. pneumoniae* | GTAACGTCAATCGATGAGGT |
| 177 | Probe for *Mvcoplasma pneumoniae* | AAATGGTACAGTCAAACTCTA |
| 178 | Probe for *Mycoplasma pneumoniae* | CATGCGAACCAAAGTTCTTA |
| 179 | Probe for *Pseudomonas aeruginosa* | CCGGACGTTATCCCCCACTA |
| 180 | Probe for *Pseudomonas aeruginosa* | GCTGAATCCAGGAGCAAGCT |
| 181 | Probe for *Serratia marcescens* | GCGAGTAACGTCAATTGATGA |
| 182 | Probe for *Serratia marcescens* | CCCCTCTACGAGACTCTAGC |
| 183 | Probe for *Serratia marcescens subsp. marcescens* | GTATTAAGCTCACCACCTTCCT |
| 184 | Probe for *Staphylococcus aureus* | CTAACATCAGAGAAGCAAGC |
| 185 | Probe for *Staphylococcus aureus* | AAGACCGTCTTTCACTTTTG |
| 186 | Probe for *Staphylococcus aureus* | CTTACACATATGTTCTTCCC |
| 187 | Probe for *Streptococcus pneumoniae* | ATGCAACATCCACTCTTATG |
| 188 | Probe for *Streptococcus pneumoniae* | CAAGTGCACCTTTTAAGCAA |
| 189 | Probe for *Streptococcus pyogenes* | CATGCGTTAGTCTCTCTTATGC |
| 190 | Probe for *Streptococcus pyogenes* | CCATCTCATAGTGGAGCAAT |
| 191 | Probe for *Streptococcus pyogenes* | CCCACCATCATTCTTCTCTAA |

Hereinafter, the present invention is described in more details in reference to examples but the present invention is not limited to these examples.

[Example 1]

In the present example, for multiple bacterial species contained in a sample, an absolute amount of the target nucleic acids (each target nucleic acid) from each bacterium was determined. Specifically, as described below, the creation of a calibration curve, calculation of a relational expression on signal intensities among the probes, and determination of an absolute amount of a target nucleic acid in the sample were carried out.

1. <u>Creation of calibration curve</u>

A calibration curve was created on a relationship between amounts before amplification and indicators of amounts after amplification of the total of a part of the target nucleic acids and the specific nucleic acid group (nucleic acids for calibration) and a control nucleic acid. Specifically, a calibration curve was created on the relationship between [a ratio of (an indicator of the total amount of a part of the target nucleic acids and the specific nucleic acid group) to (an indicator of an amount of a control nucleic acid after amplification)] and [a ratio of (the total amount of a part of the target nucleic acids and the specific nucleic acid group) to (an amount of the control nucleic acid before amplification)]. The amount of the control nucleic acid before amplification is known, whereby the total amount of all target nucleic acids and the specific nucleic acid group before amplification can be determined based on this calibration curve.

For the creation of the calibration curve, a mixed solution of genome DNA of 11 bacterial species was used as the nucleic acids for calibration. The composition of the mixed solution used is as shown in Table 2. For each genome DNA used, those with ATCC numbers shown in Table 2 were purchased from ATCC (registered trademark) (American Type Culture Collection).

The 16SrRNA gene was used as the target nucleic acids.

[Table 2]

Table 2

| Strain used | ATCC No. | Concentration of 16SrRNA gene (pM) |
|---|---|---|
| Aggregatibacter actinomycetemcomitans | 700685D-5 | 11. 2 |
| Capnocytophaga gingivalis | 33624D-5 | 9.41 |
| Fusobacterium nucleatum subsp. Nucleatum | 25586D-5 | 12.4 |
| Porphyromonas gingivalis | 33277D-5 | 10.0 |
| Prevotella intermedia | 25611D-5 | 7.38 |
| Streptococcus gordonii | 35105D-5 | 10.0 |
| Tannerella forsythia | 43037D-5 | 10.0 |
| Treponema denticola | 35405D-5 | 10.1 |
| Veillonella parvula | 10790D-5 | 9.98 |
| Streptococcus mutans | 700610D-5 | 9.99 |
| Campylobacter rectus | 33238D-5 | 3.32 |
| Total | - | 104 |

The above mixed solution (104 pM) was added to a PCR reaction solution so that the total of concentrations of the 16SrRNA genes in the PCR reaction solution was 0.24 pM and $0.25^n$ times (n = 1, 2, ..., 9) thereof. Other compositions of the PCR reaction solution are as shown in Table 3. The total solution volume was adjusted to be 20 μL. Two reactions for each condition were carried out.

[Table 3]

Table 3

| | Final concentration |
|---|---|
| Nuclease free water | - |
| F Primer (for 16SrRNA gene) | 0. 20 μ M |
| R Primer (for 16SrRNA gene) | 0. 20 μ M |
| F Primer (for control nucleic acid) | 0. 20 μ M |
| R Primer (for control nucleic acid) | 0. 20 μ M |
| Control nucleic acid | 0. 012pM |
| Premix Ex Taq HS | 1x |

The sequences of each of the primers and the control nucleic acid herein are as shown in Tables 4 and 5, respectively, and the F (Forward) primers are Cy5-modified at the 5'-end. R and Y in the sequences shown in Table 4 represent mixed bases wherein R represents A and G and Y represents C and T. Each of the primers are designed in such a way as to bind to a consensus sequence in the 16SrRNA gene of each bacterium. The control nucleic acid is a nucleic acid comprising an artificial sequence consisting of a base sequence required for the amplification and a base sequence of a random combination of bases (Table 5).

[Table 4]

Table 4

| SEQ ID NO | Name | Sequence |
|---|---|---|
| 192 | F Primer (for 16SrRNA gene) | TACGGGAGGCAGCAG |
| 193 | R Primer (for 16SrRNA gene) | CRGGGTATCTAATCCYGTT |
| 194 | F Primer (for control nucleic acid) | GAGAAGCCTACACAAACGTAACGT<br>C |
| 195 | R Primer (for control nucleic acid) | CTCTAAAGACCGCTCTATCTCGG |

[Table 5]

Table 5

| SEQ ID NO | Name | Sequence |
|---|---|---|
| 196 | Control nucleic acid | GTGAGAAGCCTACACAAACGTAACGTCAGGGCTAAGACAAACGC<br>TAACGGTACACCCTAGATGGGAGCTTGTAGCTAGATCGCTAAGT<br>CCTACCGACATGTAGGCATACTCACGAAGGCAATTCCCTGAAAG<br>CCTCGTCTTATCCCGAACTTGGCATCTGCTGATACGTCAGGTTG<br>AACGCGTACATTTACCTGTCATGCGTGGGCCTTCTCCGAATAGC<br>CTACGTAGTGATATCGCTGGTCGAATAGGCGGATTGCTCATAAA<br>TGCACATTGGCTAAGGCCCACGGAACACGAATCACGTGAGATCA<br>CTTACTATTCGACGGAACTACTATACGCACCGGGACATGCAAGT<br>AGCGTCCCACAAGCATAAGGAACTCTATACTCGCCATCTACGCA<br>GCTACAGGGGATACACGTATGAGCGGTTACGAAGTAAAGCCGAG<br>ATAGAGCGGTCTTTAGAG |

PCR enzyme used was Premix Ex Taq (registered trademark) Hot Start Version (TaKaRa), and the reaction was carried out by adjusting 2x concentration including the enzyme and a buffer as provided in such a way as to be 1x concentration.

The PCR reaction was carried out in a thermal cycler under the condition shown in Table 6.

[Table 6]

Table 6

| | | | | |
|---|---|---|---|---|
| 95°C | 98°C | 55°C | 72°C | 4°C |

(continued)

| | | | | |
|---|---|---|---|---|
| 1min | 10sec | 30sec | 20sec | ∞ |
| | 40 Cycles | | | |

After the completion of PCR, a hybridization reaction solution (1M Tris-HCl 48 μL, 1M NaCl 48 μL, 0.5% Tween20 20 μL, Nuclease free water 64 μL) was added to the respective reaction solutions (the entire volume of 20 μL). Hybridization was carried out using this solution and a DNA chip in an automated hybridization cleaning apparatus (Mitsubishi Chemical Corporation) at 50°c for 16 hours. Subsequently, cleaning was carried out.

The DNA chip used in the present example was Genopal (registered trademark), a DNA chip manufactured by Mitsubishi Chemical Corporation. The probe (probe x) loaded on this DNA chip and hybridizable with a specific sequence of each bacterium is the base sequences as set forth in SEQ ID NO: 3 to 18 (Table 7), and for the probe (probe (y)) hybridizable in common with both amplified products of all target nucleic acids and the specific nucleic acid group and for the probe (probe (z)) hybridizable with amplified products of the control nucleic acid, the base sequences as set forth in SEQ ID NO: 2 and 1 were used, respectively. For the detection of nucleic acids from *Aggregatibacter actinomycetemcomitans* and *Prevotella intermedia,* a mixture of nucleotides having the base sequences as set forth in SEQ ID NO: 3 and 4 and a mixture of nucleotides having the base sequences as set forth in SEQ ID NO: 9 and 10 were used as the probes, respectively. The 5'-end vinylated oligo-DNA of these sequences were arrayed using Genopal platform (Japanese Patent No. 6299660).

Genopal reader (Mitsubishi Chemical Corporation) was used for detection. Numerical data converted based on the detected fluorescent signals were used for analysis. Those having a condition with the total concentration of the 16SrRNA genes of $0.25^n$ times (n = 8, 9) of 0.24 pM did not change in the signal intensity ratio even with different concentrations and thus were decided as below the lower limit for quantification thereby not used for creating a calibration curve.

The amplified products of the mixed solution of genome DNA of 11 bacterial species and the control nucleic acid contain the amplified products of all of the 16SrRNA genes from the 11 bacterial species (total 16SrRNA genes) and the control nucleic acid. The signal intensity of the probe (SEQ ID NO: 2) hybridizable with total 16SrRNA genes (the indicator showing the amount of the 16SrRNA genes after amplification) was represented as $I_T$, the signal intensity of the probe (SEQ ID NO: 1) hybridizable with the control nucleic acid was represented as Ic, the amount of the 16SrRNA genes before amplification was represented as $C_T$, and the amount of the control nucleic acid before amplification was represented as Cc, and these were approximated using [Expression 1: $(\log_{10}(I_T \div I_C) = a \times \log_{10}(C_T \div C_C) + b)$] (Figure 1). The slope and intercept of the approximate straight line were determined using LINEST function in a Microsoft software "EXCEL" and the parameters in Expression 1 were determined as a = 0.389 and b = 0.581.

This calibration curve enabled the determination of the total amount of all target nucleic acids and the specific nucleic acid group before amplification. This "the total amount of all target nucleic acids and the specific nucleic acid group before amplification" was needed when finally determining an absolute amount of the target nucleic acids (each target nucleic acid) from each bacterium.

2. Calculation of relational expression on signal intensities among probes

In a later test, for determining an absolute amount of each target nucleic acid based on an occupancy of target nucleic acids (each target nucleic acid) from each bacterium in the "total of all target nucleic acids and the specific nucleic acid group", the calculation must be carried out by applying the signal intensity of each probe (x) hybridizable with each target nucleic acid and the signal intensity of the probe (y) hybridizable with the "all target nucleic acids and the specific nucleic acid group" to the same expression. On the other hand, as the probe (X) and the probe (y) are likely to be different in the absolute values of the signal intensities, applying both of them to the same expression for the calculation requires to obtain a relational expression on the signal intensities between both of them and convert the signal intensity of the probe (x) to a value equivalent to the signal intensity of the probe (y).

For this reason, in the present example, relational expressions between the signal intensities of probes (probe (x)) (SEQ ID NO: 3 to 18) hybridizable respectively with specific sequences of the 14 bacterial species (Table 7) and the signal intensity of the probe (probe (y)) (SEQ ID NO: 2) hybridizable with all of the 16SrRNA genes (total 16SrRNA genes) from the 14 bacterial species were calculated by the bacterial species.

For each genome DNA used, those with the numbers shown in Table 7 were purchased from ATCC (registered trademark) (American Type Culture Collection) or DSMZ (Deutsche Sammlung Von Mikroorganismen Und Zellkulturen).

[Table 7]

Table 7

| SEQ ID NO of probe | Name of bacteria | Number |
|---|---|---|
| 3, 4 | Aggregatibacter actinomycetemcomitans | ATCC 700685D-5 |
| 5 | Campylobacter rectus | ATCC 33238D-5 |
| 6 | Capnocytophaga gingivalis | ATCC 33624D-5 |
| 7 | Fusobacterium nucleatum | ATCC 25586D-5 |
| 8 | Porphyromonas gingivalis | ATCC 33277D-5 |
| 9,10 | Prevotella intermedia | ATCC 25611D-5 |
| 11 | Prevotella nigrescens | DSMZ N0.13386 |
| 12 | Selenomonas noxia | DSMZ N0.19578 |
| 13 | Streptococcus constellatus | DSMZ N0.25819 |
| 14 | Streptococcus gordonii | ATCC 35105D-5 |
| 15 | Streptococcus intermedius | DSMZ N0.20573 |
| 16 | Tannerella forsythia | ATCC 43037D-5 |
| 17 | Treponema denticola | ATCC 35405D-5 |
| 18 | Veillonella parvula | ATCC 10790D-5 |

The following experiment was carried out for each genome DNA.

The PCR reaction solution was first prepared in such a way that weights of the genome DNA contained in the solution were 1 pg, 0.5 pg, and 0.1 g. Other compositions and the solution volume were the same as in the above "1. Creation of calibration curve."

The PCR reaction, hybridization and detection were carried out in the same manner as in the above " 1. Creation of calibration curve" and numerical data converted based on the detected fluorescent signals were used for analysis. Specifically, DNA of all 16SrRNA genes from the above 14 bacterial species were amplified by PCR reaction and hybridized with the probes loaded on the DNA chip. The signal intensity of each probe (probe (x)) hybridizable respectively with specific base sequence of each bacterium was represented as Ius and the signal intensity of the probe (probe (y)) hybridizable with total 16SrRNA genes was represented as $I_{TS}$, and these were approximated using [Expression 5: $I_{TS} = E_S \times I_{US}$] for each of the bacterial species. This relational expression, that is, the relational expression between the signal intensity of each probe (x) and the signal intensity of the probe (y), was determined for all of the 14 bacterial species.

The results on the genome DNA of *Aggregatibacter actinomycetemcomitans* and *Campylobacter rectus* are shown as representative in Figure 2. As a result of determining the slope of the approximate straight line using an option to force an intercept of 0 with LINEST function in a Microsoft software "EXCEL", the parameter Es in Expression 5 was determined as shown in Table 8.

[Table 8]

Table 8

| Probe No. | Name of bacteria | $E_s$ |
|---|---|---|
| 3, 4 | Aggregatibacter actinomycetemcomitans | 0.526 |
| 5 | Campylobacter rectus | 0.708 |
| 6 | Capnocytophaga gingivalis | 0. 447 |
| 7 | Fusobacterium nucleatum | 0.572 |

(continued)

| Probe No. | Name of bacteria | $E_s$ |
|---|---|---|
| 8 | Porphyromonas gingivalis | 1. 15 |
| 9,10 | Prevotella intermedia | 0.804 |
| 11 | Prevotella nigrescens | 2.62 |
| 12 | Selenomonas noxia | 0.925 |
| 13 | Streptococcus constellatus | 2. 78 |
| 14 | Streptococcus gordonii | 0.488 |
| 15 | Streptococcus intermedius | 0.427 |
| 16 | Tannerella forsythensis | 0.258 |
| 17 | Treponema denticola | 0.175 |
| 18 | Veillonella parvula | 2.95 |

3. Determination of absolute amounts of target nucleic acids in a sample

A saliva evaluation test was carried out with the cooperation of 1 healthy adult (subject).

Saliva was collected at the time of waking up, crushed with bead, treated with proteinase K (QUIAGEN), and extracted DNA using QIAamp 96 DNA Blood Kit (QIAGEN). DNA extracted from the saliva contained genome DNA of the above 14 bacterial species to be measured, genome DNA of the bacterial species other than the bacterial species to be measured, and other DNA (Figure 4A).

A concentration of the extracted DNA was measured using NanoDrop (trademark) (Thermo Fisher Scientific) and a PCR reaction solution was prepared based on the measured concentration in such a way that a weight of the extracted DNA contained in the solution was 100 pg. Other composition and the solution volume were the same as in the above "1. Creation of calibration curve."

The PCR reaction, hybridization and detection were carried out in the same manner as in the above "1. Creation of calibration curve" and numerical data converted based on the detected fluorescent signals were used for analysis.

The amplified products of the DNA extracted from the saliva sample and the control nucleic acid contained (i) the 16SrRNA genes (target nucleic acids) from the above 14 bacterial species to be measured, (ii) the 16SrRNA genes from bacterial species other than the bacterial species to be measured, which are the specific nucleic acid group to be collectively amplified with all target nucleic acids by amplification (specific nucleic acid group), and (iii) the amplified control nucleic acid (Figure 4B).

The total of the above (i) and (ii) is applicable to the above "the total of all target nucleic acids and the specific nucleic acid group" or "total 16SrRNA genes."

An amount of substance (mol) $C_{TM}$ of total 16SrRNA genes added to the PCR reaction solution was calculated using the signal intensities $I_{TM}$ of probes of total 16SrRNA genes and the parameters determined in "1. Creation of calibration curve" in accordance with Expression 3 [$C_{TM} = C_C \times 10$^(($\log_{10}(I_{TM} \div I_{CM})$- 0.581) ÷ 0.389)] (Figure 4C) and the number of copies was calculated by multiplying $C_{TM}$ by Avogadro's number. The number of copies of total 16SrRNA gene in 1 mL of the saliva was calculated based on the dilution ratios at the extraction step and the PCR reaction solution preparation step.

Subsequently, the number of 16SrRNA gene copies of each bacterium was calculated from the signal intensity $I_{US}$ of each probe (x) specifically hybridizable with each bacterium, the parameters determined in "2. Calculation of relational expression on signal intensities among probes" and the number of copies of total 16SrRNA genes per mL of the above saliva in accordance with Expression 6 ($I_{TSM} = E_S \times I_{USM}$) and Expression 7($R_S = I_{TSM} \div I_{TM}$) (Figure 4D).

The calculated number of copies of each 16SrRNA gene per mL of the saliva are as shown in Table 9.

[Table 9]

Table 9

| SEQ ID NO of probe | Name | Number of copies |
|---|---|---|
| 2 | Total 16SrRNA genes | 4.3.E+07 |
| 3, 4 | Aggregatibacter actinomycetemcomitans | 4.6.E+04 |
| 5 | Campylobacter rectus | 7. 4. E+04 |
| 6 | Capnocytophaga gingivalis | 6.4.E+04 |
| 7 | Fusobacterium nucleatum | 5.0.E+05 |
| 8 | Porphyromonas gingivalis | 1. 4. E+05 |
| 9,10 | Prevotella intermedia | 7. 1. E+04 |
| 11 | Prevotella nigrescens | 1. 1. E+05 |
| 12 | Selenomonas noxia | 2.2.E+04 |
| 13 | Streptococcus constellatus | 1. 9. E+04 |
| 14 | Streptococcus gordonii | 2. 8. E+05 |
| 15 | Streptococcus intermedius | 1.4. E+05 |
| 16 | Tannerella forsythia | 1.6. E+04 |
| 17 | Treponema denticola | 4. 1. E+04 |
| 18 | Veillonella parvula | 3. 1. E+05 |

[Example 2]

In the present example, a calibration curve was created under different conditions from Example 1.

The composition of a mixed solution used as the nucleic acids for calibration was the same as in Example 1.

The above mixed solution was added to a PCR reaction solution so that the total of concentrations of the 16SrRNA genes in the PCR reaction solution was 24 pM and $(1/16)^n$ times (n = 1, 2, ···, 5) thereof. Other compositions of the PCR reaction solution are as shown in Table 10. The total solution volume was adjusted to be 20 μL. One reaction was carried out for each condition.

[Table 10]

Table 10

| | Final concentration |
|---|---|
| Nuclease free water | - |
| F Primer (for 16SrRNA gene) | 1.0 μ M |
| R Primer | 1.0 μ M |
| Control nucleic acid | 0.025pM |
| Premix Ex Taq HS | 1x |

The sequences of each of the primers and the control nucleic acid herein are as shown in Tables 11 and 12, respectively, and the F primers are Cy5-modified at the 5'-end. "I" in the sequence of Table 11 represents inosine.

[Table 11]

Table 11

| SEQ ID NO | Name | Sequence |
|---|---|---|
| 192 | F Primer (for 16SrRNA gene) | TACGGGAGGCAGCAG |
| 197 | R Primer | TACCIGGGTATCTAATCC |

[Table 12]

Table 12

| SEQ ID NO | Name | Sequence |
|---|---|---|
| 198 | Control nucleic acid | GTTCCTACGGGAGGCAGCAGTAGGGCTAAGACAAACGCTAACGG TACACCCTAGATGGGAGCTTGTAGCTAGATCGCTAAGTCCTACC GACATGTAGGCATACTCACGAAGGCAATTCCCTGAAAGCCTCGT CTTATCCCGAACTTGGCATCTGCTGATACGTCAGGTTGAACGCG TACATTTACCTGTCATGCGTGGGCCTTCTCCGAATAGCCTACGT AGTGATATCGCTGGTCGAATAGGCGGATTGCTCATAAATGCACA TTGGCTAAGGCCCACGGAACACGAATCACGTGAGATCACTTACT ATTCGACGGAACTACTATACGCACCGGGACATGCAAGTAGCGTC CCACAAGCATAAGGAACTCTATACTCGCCATCTACGCAGCTACA GGGGATACACGTATGAGCGGTTACGAAGTAAAGGGTAGCAAACA GGATTAGATACCCTGAAA |

The PCR reaction was carried out in a thermal cycler under the condition shown in Table 13.

[Table 13]

Table 13

| | | | | |
|---|---|---|---|---|
| 95°C | 98°C | 55°C | 72°C | 4°C |
| 1min | 10sec | 30sec | 20sec | ∞ |
| | 25 Cycles | | | |

The hybridization and detection were carried out in the same manner as in "1. Creation of calibration curve" of Example 1 and numerical data converted based on the detected fluorescent signals were used for analysis.

The signal intensity of the probe (SEQ ID NO: 2) hybridizable with total 16SrRNA genes is represented as $I_T$, the signal intensity of the probe (SEQ ID NO: 1) hybridizable with the control nucleic acid was represented as Ic, the amount of the 16SrRNA genes before amplification was represented as $C_T$, and the amount of the control nucleic acid before amplification was represented as Cc, and these were approximated by [Expression 1: $(\log10(I_T \div I_C) = a \times \log10(C_T \div C_C) + b)$] (Figure 3). The slope and intercept of the approximate straight line were determined using LINEST function in a Microsoft software "EXCEL" and the parameters in Expression 1 were determined as a = 0.730 and b = 1.85.

Examples 1 and 2 showed that the method of the present invention can determine an absolute amount of multiple types of target nucleic acids contained in a sample simultaneously and in a simple manner. Additionally, the method of the present invention showed that only a single type of a nucleic acid needs to be used as the control nucleic acid and a plural type of control nucleic acids do not need to be used.

[Example 3]

In the present example, for bacteria contained in a sample, an absolute amount of target nucleic acids (each target nucleic acid) from each bacterium was determined under different conditions from Example 1. Standard samples having a known composition were used as a sample, and measured values were compared with theoretical values.

1. Creation of calibration curve

As nucleic acids for calibration for creating a calibration curve, the total of three types of solutions, commercial 20 Strain Even Mix Genomic Material (ATCC (registered trademark) MSA-1002™), 20 Strain Staggerd Mix Genomic Material (ATCC (registered trademark) MSA-1003™) and, in addition, a mixed solution of genome DNA of seven bacterial species having the composition shown in Table 14 were used as the standard samples.

[Table 14]

Table 14

| Strain used | ATCC No. | Concentration of 16SrRNA gene (pM) |
|---|---|---|
| Aggregatibacter actinomycetemcomitans | 700685D-5 | 14.3 |
| Fusobacterium nucleatum subsp. nucleatum | 25586D-5 | 14.3 |
| Porphyromonas gingivalis | 33277D-5 | 13.6 |
| Prevotella intermedia | 25611D-5 | 14.3 |
| Streptococcus gordonii | 35105D-5 | 14.3 |
| Streptococcus mutans | 700610D-5 | 14.3 |
| Veillonella parvula | 10790D-5 | 14.3 |
| Total | - | 99.4 |

The above standard samples were subjected to PCR reaction under the conditions (template concentration, repeat count (N count)) shown in Table 15 and the obtained PCR products were measured using the DNA chip. The composition of the PCR reaction solution other than the nucleic acids for calibration was as shown in Table 16.

[Table 15]

Table 15

| Nucleic acids for calibration | Concentration of 16SrRNA gene (pM) | Repeat count (N count) |
|---|---|---|
| MSA1003 Staggerd Mix | 9.39×0.25^n (n=1, 2 ···9) | 3 Each |
| | 8. 79 | 3 |
| | 9.50 | 2 |
| MSA1002 Even Mix | 2.43 | 3 |
| | 2. 79 | 2 |
| 0C93 MIX | 24.8 × 0. 0625^n (n=1, 2, ···, 6) | 2 Each (n = 1, 4) or 4 each (n = 2, 3, 5, 6) |

[Table 16]

Table 16

| Component | Final concentration |
|---|---|
| Nuclease free water | - |
| F Primer (for 16SrRNA gene) | 1. 0 $\mu$M |
| R Primer | 1. 0 $\mu$M |
| Control nucleic acid | 0. 025pM |
| Premix Ex Taq HS | 1x |

The primers used are the same as in Example 2 and the base sequence of the control nucleic acid is as shown in Table 17.

[Table 17]

Table 17

| SEQ ID NO | Name | Sequence |
|---|---|---|
| 199 | Control nucleic acid | GTTCCTACGGGAGGCAGCAGTAGGGCTAAGACAAACGCTAA<br>CGGTACACCCTAGATGGGAGCTTGTAGCTAGATCGCTAAGT<br>CCTACCGACATGTAGGCATACTCACGAAGGCAATTCCCTGA<br>AAGCCTCGTCTTATCCCGAACTTGGCATCTGCTGATACGTC<br>AGGTTGAACGCGTACATTTACCTGTCATGCGTGGGCCTTCT<br>CCGAATAGCCTACGTAGTGATATCGCTGGTCGAATAGGCGG<br>ATTGCTCATAAATGCACATTGGCTAAGGCCCACGGAACACG<br>AATCACGTGAGATCACTTACTATTCGACGGAACTACTATAC<br>GCACCGGGACATGCAAGTAGCGTCCCACAAGCATAAGGAAC<br>TCTATACTCGCCATCTACGCAGCTACAGGGGATACACGTAT<br>GAGCGGTTACGAAGTAAAGGGTAGCAACAGGATTAGATACC<br>CTGGTAGTCCAC |

The PCR reaction was carried out in a thermal cycler under the same conditions as in "1. Creation of calibration curve" of Example 1. The DNA chip used in the present example has the same structure as in Example 1 but a new DNA chip on which the probe for *Actinomyces odontolyticus* (SEQ ID NO: 19) and the probe for *Streptococcus mutans* (SEQ ID NO: 38) were additionally loaded in addition to the probe (probe (x)) hybridizable with the specific sequence of each bacterium was prepared.

The hybridization and detection were carried out in the same manner as in "1. Creation of calibration curve" of Example 1 and numerical data converted based on the detected fluorescent signals were used for analysis.

The signal intensity of the probe (SEQ ID NO: 2) hybridizable with total 16SrRNA genes was represented as $I_T$, the signal intensity of the probe (SEQ ID NO: 1) hybridizable with the control nucleic acid was represented as Ic, the amount of the 16SrRNA genes before amplification was represented as $C_T$, and the amount of the control nucleic acid before amplification was represented as Cc, and these were approximated using [Expression 2: $\log10(I_T \div I_C) = b \times (\log10(C_T \div C_C) - c) \div (a - |\log10(C_T \div C_C) - c|)$] (Figure 5). An approximate expression was created using the function called "SOLVER" in a Microsoft software "EXCEL" and the parameters in Expression 2 were determined as a = 8.07, b = 3.43, and c = 0.119. Additionally, the signal intensities obtained were confirmed to have been plotted on the substantially same curved line regardless the type of nucleic acids for calibration used.

2. Calculation of relational expression on signal intensities among probes

A relational expression between the signal intensities of the probes (probe (x)) (SEQ ID NO: 8, 19,38) hybridizable respectively with specific base sequences of three bacterial species (Table 18) and the signal intensity of the probe (probe (y)) (SEQ ID NO: 2) hybridizable with all of the 16SrRNA genes (total 16SrRNA genes) from the three bacterial species was herein calculated by the bacterial species.
The DNA used are synthetic nucleic acids of the base sequences described in Table 18.

[Table 18]

Table 18

| SEQ ID NO of probe | Name of bacteria | Base sequence of synthetic nucleic acid used |
|---|---|---|
| 8 | Porphyromonas gingivalis | Sequence of ID: AB035459.1 of GenBank |
| 19 | Actinomyces odontolyticus | Sequence from $1281646^{th}$ base to $1499^{th}$ base of sequence of ID: AAYI02000004.1 of GenBank |
| 38 | Streptococcus mutans | Sequence of ID: AY188348 of GenBank |

The following experiment was carried out for each DNA of the three bacterial species.
The PCR reaction solution was first prepared in such a way that amounts of substance of DNA contained in the solution were 0.5 amol, 0.05 amol, and 0.005 amol. Other compositions and the solution volume are the same as in the above "1. Creation of calibration curve" of the present example.
The PCR reaction, hybridization and detection were carried out in the same manner as in the above "1. Creation of calibration curve" of the present example and numerical data converted based on the detected fluorescent signals were used for analysis.
Specifically, the above three types of DNA were amplified by PCR reaction and hybridized with the probes loaded on the DNA chip. The signal intensity of each probe (probe (x)) hybridizable respectively with specific base sequence of each bacterium was represented as $I_{US}$ and the signal intensity of the probe (probe (y)) hybridizable with total 16SrRNA genes was represented as $I_{TS}$, and these were approximated using [Expression 5: $I_{TS} = E_S \times I_{US}$] by the bacterial species. The relational expression, that is, the relational expression between the signal intensity of each probe (x) and the signal intensity of the probe (y) was determined for all three bacterial species.
The results on the three types of DNA are shown in Figure 6. As a result of determining the slope of the approximate straight line using an option to force an intercept of 0 with LINEST function in a Microsoft software "EXCEL", the parameter Es in Expression 5 was determined as shown in Table 19.

[Table 19]

Table 19

| Name of bacteria | $E_S$ |
|---|---|
| Porphyromonas gingivalis | 1.45 |
| Actinomyces odontolyticus | 3.30 |
| Streptococcus mutans | 1.15 |

3. Determination of absolute amounts of target nucleic acids in a sample

Quantitativity was verified using 20 Strain Even Mix Genomic Material (ATCC (registered trademark) MSA-1002™) as a sample having a known concentration. The number of copies of genome DNA of the 3 bacteria shown in the above Table 18 contained in this sample was already known to be 5% of the total, respectively. That is, a theoretical value of the number of copies of genome DNA of each bacterium per weight of this sample can be calculated based on this composition information of the genome DNA and the information for the number of copies of 16SrRNA in 1 genome of each bacterial genome DNA and genome size.

The PCR reaction solution was prepared in such a way that a weight of the sample DNA contained in the solution was 211 pg. Other compositions and the solution volume were the same as in the above "1. Creation of calibration curve."

The PCR reaction, hybridization and detection were carried out in the same manner as in the above " 1. Creation of calibration curve" and numerical data converted based on the detected fluorescent signals were used for analysis.

An amount of substance (mol) $C_{TM}$ of total 16SrRNA genes added to the PCR reaction solution was calculated using the signal intensities $I_{TM}$ of probes of total 16SrRNA genes and the parameters determined in "1. Creation of calibration curve" in accordance with Expression 4 [CTM = $C_C \times 10$^(8.43 $\times$log10($I_{TM} \div I_{CM}$) $\div$(3.43 + |log10($I_{TM} \div I_{CM}$)|)+ 0.119)] (Figure 4C) and the number of copies was calculated by multiplying $C_{TM}$ by Avogadro's number.

Subsequently, the number of copies of 16SrRNA gene of each bacterium was calculated from the signal intensity of each probe (x) specifically hybridizable with each bacterium Ius, the parameters determined in "2. Calculation of relational expression on signal intensities among probes" and the number of total 16SrRNA gene copies calculated in the above in accordance with Expression 6 ($I_{TSM}$= Es $\times I_{USM}$) and Expression 7 ($R_S$ = $I_{TSM} \div I_{TM}$) (Figure 4D).

Further, the number of copies of genome DNA of each bacterium was calculated based on the number of copies of 16SrRNA gene of the 3 bacteria calculated and the number of copies of 16SrRNA in 1 genome of respective bacteria (see annotation information on sequences respectively with Genbank ID of AP009380.1, NZ_DS264586.1, and AE014133.2). The number of copies of total genome DNA was calculated using 5.25, which was a weighted average value of the bacterial composition of 20 Strain Even Mix Genomic Material (ATCC (registered trademark) MSA-1002™) used as the sample.

The measured values of the number of copies of each genome DNA added to the PCR reaction solution and theoretical values are shown in Table 20. The differences between the measured values and the theoretical values are matched with each other within an error range of about 2 times, with the exception of *Actinomyces odontolyticus.*

[Table 20]

Table 20

| Name of bacteria | Number of copies (measured value) | Number of copies (theoretical value) | Measured value/ theoretical value |
|---|---|---|---|
| Total genome DNA | 8. 65. E+04 | 5.74.E+04 | 1.51 |
| Porphyromonas gingi valis | 4. 96.E+03 | 2. 87. E+03 | 1.73 |
| Actinomyces odontolyticus | 5. 55.E+02 | 2. 87. E+03 | 0. 193 |
| Streptococcus mutans | 6. 69. E+03 | 2. 87. E+03 | 2.33 |

The above results showed that the method of the present invention can determine an absolute amount of two or more types of target nucleic acids in a sample with a high accuracy.

[Example 4]

In the present example, λ exonuclease treatment was added after the PCR amplification to determine an absolute amount, which was compared with theoretical values.

1. Creation of calibration curve

For creation of a calibration curve, a commercial 20 Strain Even Mix Genomic Material (ATCC (registered trademark) MSA-1002™) was used as the nucleic acids for calibration. The above solution was added to a PCR reaction solution so that the total of concentrations of the 16SrRNA genes in the PCR reaction solution was 1.60 pM and 0.25n times (n = 1, 2, ..., 8) thereof. Other compositions of the PCR reaction solution are the same as in Example 3. The total solution volume was adjusted to be 20 μL. One reaction for each condition was carried out.

The F primers and the control nucleic acid used herein are the same as in Example 3. On the other hand, the R primers used had the same sequence as in Example 3 and were phosphorylated at the 5'-end.

The PCR reaction was carried out in the same manner as in Example 3, and subsequently the λ exonuclease-treated reaction solution was prepared to have a total solution volume of 50 μL using the entire volume of the PCR reaction solution with the composition shown in Table 21. The λ exonuclease-treated reaction was carried out in a thermal cycler under the conditions shown in Table 22. The λ exonuclease and the buffer used are manufactured by NEW ENGLAND BioLabs, Inc.

[Table 21]

Table 21

| Component | Final concentration |
|---|---|
| Nuclease free water | - |
| PCR Reaction solution | - |
| Lambda Exonuclease Reaction Buffer | 1X |
| λ Exonuclease | 0. 3Unit/ μ 1 |

[Table 22]

Table 22

| 37°C | 75°C | 4°C |
|---|---|---|
| 30min | 10min | ∞ |

After the completion of reaction by λ exonuclease, a hybridization reaction solution (1M Tris-HCl 48 μL, 1M NaCl 48 μL, 0.5% Tween20 20 μL, Nuclease free water 34 μL) was added to the respective reaction solutions (the entire volume of 50 μL). Hybridization was carried out using this solution and a DNA chip in an automated hybridization cleaning apparatus (Mitsubishi Chemical Corporation) at 50°C for 16 hours. Subsequently, cleaning was carried out. The DNA chip used was the same as in Example 3.

The detection was carried out in the same manner as in "1. Creation of calibration curve" of Example 3 and numerical data converted based on the detected fluorescent signals were used for analysis. The approximation using Expression 2 was also carried out in the same manner as in "1. Creation of calibration curve" of Example 3 (Figure 7) and the parameters were determined as a = 7.29, b = 2.94, and c = 0.0746.

2. Calculation of relational expression on signal intensities among probes

A relational expression between the signal intensities of the probes (probe (x)) (SEQ ID NO: 8, 19, 38) hybridizable respectively with specific sequences of three bacterial species (Table 18) quantified in Example 3 and the signal intensity of the probe (probe (y)) (SEQ ID NO: 2) hybridizable with all of the 16SrRNA genes (total 16SrRNA genes) from the three bacterial species was herein calculated by the bacterial species. That is, the λ exonuclease treatment after the PCR reaction and the addition of the hybridization reaction solution were carried out in the same manner as in the above "1. Creation of calibration curve" and others were carried out in the same manner as in "2. Calculation of relational expression on signal intensities among probes" of Example 3 to determine the relational expression between the signal intensity of each probe (x) and the signal intensity of the probe (y) for all of the three bacterial species.

The results on the DNA of the three bacterial species are shown in Figure 8. The parameter Es in Expression 5 was determined as shown in Table 23.

[Table 23]

Table 23

| SEQ ID NO of probe | Name of bacteria | $E_S$ |
|---|---|---|
| 8 | Porphyromonas gingivalis | 1.82 |
| 19 | Actinomyces odontolyticus | 1.20 |
| 38 | Streptococcus mutans | 1.74 |

3. Determination of absolute amounts of target nucleic acids in a sample

A quantification experiment was carried out using a commercial 20 Strain Even Mix Genomic Material (ATCC (registered trademark) MSA-1002™) as the standard sample.

The λ exonuclease treatment after the PCR reaction and the addition of the hybridization reaction solution were carried out in the same manner as in the above "1. Creation of calibration curve" and others were carried out in the same manner as in "3. Determination of absolute amounts of target nucleic acids in a sample" of Example 3 to calculate the number of copies of genome DNA of each bacterium for all of the three bacterial species.

The measured values of the calculated number of copies of each genome DNA in the PCR reaction solution and theoretical values are as shown in Table 24.

[Table 24]

Table 24

| Name of bacteria | Number of copies (measured value) | Number of copies (theoretical value) | Measured value/ theoretical value |
|---|---|---|---|
| All genome DNA | 5. 02. E+04 | 5.74. E+04 | 0.874 |
| Porphyromonas gingivalis | 2. 56. E+03 | 2. 87. E+03 | 0.892 |
| Actinomyces odontolyticus | 2. 34.E+03 | 2. 87. E+03 | 0.814 |
| Streptococcus mutans | 5. 75.E+03 | 2.87.E+03 | 2.00 |

In the above results, the smaller the difference between the measured value and the theoretical value in the number of copies (measured value/theoretical value is close to 1), the higher the accuracy of the absolute amount measurement is shown.

The above results showed that the method of the present invention comprising the step of enriching nucleic acids after amplification for a single chain (a single strand) was able to determine absolute amounts of two or more types of target nucleic acids in a sample with a further higher accuracy than the results of Example 3 (Table 20). Particularly, in the determination of absolute amounts of the target nucleic acids of *Actinomyces odontolyticus* in Example 4, the notable enhancement in the measurement accuracy of absolute amounts was recognized when compared with the results of Example 3 (Table 20).

[Example 5]

In the present example, a part of the PCR reaction conditions was altered and the comparison with the theoretical value was carried out in the same manner as in Example 3.

1. Creation of calibration curve

The nucleic acids for calibration used for creating a calibration curve, the composition of the PCR reaction solution, the primers and the control nucleic acid used are the same as in Example 4.

The PCR reaction was carried out in a thermal cycler under the condition shown in Table 25, subsequently the hybridization and detection were carried out in the same manner as in "1. Creation of calibration curve" of Example 1 and numerical data converted based on the detected fluorescent signals were used for analysis. The DNA chip used was the same as in Example 3.

[Table 25]

Table 25

| | | | | | |
|---|---|---|---|---|---|
| 95°C | 98°C | 55°C | 72°C | 72°C | 4°C |
| 1min | 10sec | 30sec | 1min | 10min | ∞ |
| | 40 Cyclles | | | | |

The approximation using Expression 2 was carried out in the same manner as in "1. Creation of calibration curve" of Example 3 (Figure 9) and the parameters were determined as a = 4.74, b = 0.834, and c = -0.593.

2. Calculation of relational expression on signal intensities among probes

A relational expression between the signal intensities of the probes (probe (x)) (SEQ ID NO: 19 and 38) hybridizable respectively with specific sequences of two bacterial species, *Actinomyces odontolyticus* and *Streptococcus mutans,* of the three bacterial species quantified in Example 3 (Table 18) and the signal intensity of the probe (probe (y)) (SEQ ID NO: 2) hybridizable with all of the 16SrRNA genes (total 16SrRNA genes) from the two bacterial species was herein calculated by the bacterial species. That is, the PCR reaction was carried out in the same manner as in the above "1. Creation of calibration curve" and others were carried out in the same manner as in "2. Calculation of relational expression on signal intensities among probes" of Example 3 to determine the relational expression between the signal intensity of each probe (x) and the signal intensity of the probe (y) for the two bacterial species.

The results of two types of DNA are shown in Figure 10. The parameter $E_S$ in Expression 5 was determined as shown in Table 26.

[Table 26]

Table 26

| SEQ ID NO of probe | Name of bacteria | $E_S$ |
|---|---|---|
| 19 | Actinomyces odontolyticus | 1.79 |
| 38 | Streptococcus mutans | 1.21 |

3. Determination of absolute amounts of target nucleic acids in a sample

A quantification experiment was carried out using a commercial 20 Strain Even Mix Genomic Material (ATCC (registered trademark) MSA-1002™) as the standard sample.

The PCR reaction was carried out in the same manner as in the above "1. Creation of calibration curve" and others were carried out in the same manner as in "3. Determination of absolute amounts of target nucleic acids in a sample" of Example 3 to calculate the number of copies of genome DNA of each bacterium for the two bacterial species.

The measured values of the calculated number of copies of each genome DNA in the PCR reaction solution and theoretical values are as shown in Table 27.

[Table 27]

Table 27

| Name of bacteria | Number of copies (measured value) | Number of copies (theoretical value) | Measured value/ theoretical value |
|---|---|---|---|
| Total genome DNA | 8. 74. E+04 | 5. 74. E+04 | 1.52 |
| Actinomyces odontolyticus | 2. 83.E+03 | 2.87. E+03 | 0.986 |
| Streptococcus mutans | 4. 42.E+03 | 2.87. E+03 | 1.54 |

The above results showed that the method of the present invention was able to determine absolute amounts of two or more types of target nucleic acids in a sample with a further higher accuracy than the results of Example 3 (Table 20). Particularly, in the determination of absolute amounts of the target nucleic acids of *Actinomyces odontolyticus* in Example 5, the significant enhancement in the measurement accuracy of absolute amounts was recognized when compared with the results of Example 3 (Table 20).

[Industrial Applicability]

According to the present invention, an absolute quantification of multiple types of target nucleic acids respectively (each target nucleic acid) is enabled in a simple manner.

SEQ ID NOS: 1 to 199: Synthetic DNA

## Claims

1. A method for determining absolute amounts of two or more types of target nucleic acids in a sample, the method comprising the steps of:

   (a) mixing a sample with a known amount of a control nucleic acid;
   (b) co-amplifying all target nucleic acids and a specific nucleic acid group to be collectively amplified with the target nucleic acids in the sample and the control nucleic acid;
   (c) determining a total amount of all target nucleic acids and the specific nucleic acid group in the sample based on an indicator of a total amount of the amplified all target nucleic acids and the specific nucleic acid group and an indicator of an amount of the amplified control nucleic acid; and
   (d) calculating an absolute amount of each target nucleic acid in the sample based on an occupancy of each target nucleic acid in the total of all target nucleic acids and the specific nucleic acid group which is calculated from the indicator of the total amount of the amplified all target nucleic acids and the specific nucleic acid group and an indicator of an amount of each amplified target nucleic acid.

2. The method according to claim 1, wherein the control nucleic acid is an artificial sequence consisting of a base sequence required for amplification and a base sequence in which a part or all of the bases are in a random combination.

3. The method according to claim 1 or 2, wherein the co-amplification of the total of all target nucleic acids and the specific nucleic acid group in the sample and the control nucleic acid is carried out by a technique selected from the group consisting of Polymerase Chain Reaction (PCR) method, Loop-Mediated Isothermal Amplification (LAMP) method, and in vitro transcription.

4. The method according to claim 3, wherein the co-amplification of the total of all target nucleic acids and the specific nucleic acid group in the sample and the control nucleic acid is carried out by PCR method.

5. The method according to any one of claims 1 to 4, wherein the sample is at least one selected from the group consisting of foods and drinks, biological samples, environmental samples, and samples from industrial process.

6. The method according to claim 5, wherein the sample is at least one selected from the group consisting of activated sludges, soils, river waters, seawaters, hot spring waters, drinking waters, processed foods, fermenter cultures, and tissues, cells and body fluids collected from eukaryotes.

7. The method according to claim 5, wherein the biological sample is at least one selected from the group consisting of saliva, plaque, gingival crevicular fluid (GCF), feces, and skin-derived samples collected from mammals.

8. The method according to any one of claims 1 to 7, wherein the target nucleic acid is a bacterial ribosomal RNA (rRNA) gene.

9. The method according to claim 8, wherein the target nucleic acids are rRNA genes of at least two species of bacteria selected from the group consisting of bacteria belonging to the *Abiotrophia* genus, *Achromobacter* genus, *Acinetobacter* genus, *Actinomyces* genus, *Aerococcus* genus, *Aggregatibacter* genus, *Alloprevotella* genus, *Alloscardovia* genus, *Anaerococcus* genus, *Anaeroglobus* genus, *Arcanobacterium* genus, *Atopobium* genus, *Bacillus* genus, *Bacteroides* genus, *Bifidobacterium* genus, *Bordetella* genus, *Brevundimonas* genus, *Brucella* genus, *Burkholderia* genus, *Campylobacter* genus, *Candidatus Absconditabacteria* (SR1), *Candidatus Saccharibacteria* (TM7), *Capnocytophaga* genus, *Cardiobacterium* genus, *Catonella* genus, *Chlamydia* genus, *Chlamydophila* genus, *Chryseobacterium* genus, *Citrobacter* genus, *Clostridium* genus, *Collinsella* genus, *Corynebacterium* genus, *Coxiella* genus, *Cronobacter* genus, *Cryptobacterium* genus, *Curvibacter* genus, *Dialister* genus, *Eggerthella* genus, *Eikenella* genus, *Elizabethkingia, Enterobacter* genus, *Enterococcus* genus, *Escherichia* genus, *Eubacterium* genus, *Filifactor* genus, *Finegoldia* genus, *Fusobacterium* genus, *Gardnerella* genus, *Gemella* genus, *Granulicatella* genus, *Haemophilus* genus, *Helicobacter* genus, *Kingella* genus, *Klebsiella* genus, *Kocuria* genus, *Lachnoanaerobaculum* genus, *Lactobacillus* genus, *Lactococcus* genus, *Lautropia* genus, *Legionella* genus, *Leptotrichia* genus, *Listeria* genus, *Megasphaera* genus, *Methanobrevibacter* genus, *Microbacterium* genus, *Micrococcus* genus, *Mitsuokella* genus, *Mobiluncus* genus, *Mogibacterium* genus, *Moraxella* genus, *Morganella* genus, *Mycobacterium* genus, *Mycoplasma* genus, *Neisseria* genus, *Nocardia* genus, *Olsenella* genus, *Oribacterium* genus, *Paracoccus* genus, *Parascardovia* genus, *Parvimonas* genus, *Peptoniphilus* genus, *Peptostreptococcus* genus, *Porphyromonas* genus, *Prevotella* genus, *Propionibacterium (Cutibacterium)* genus, *Proteus* genus, *Providencia* genus, *Pseudomonas* genus, *Pseudopropionibacterium* genus, *Pseudoramibacter* genus, *Pyramidobacter* genus, *Ralstonia* genus, *Rothia* genus, *Scardovia* genus, *Schlegelella* genus, *Sebaldella* genus, *Selenomonas* genus, *Serratia* genus, *Simonsiella* genus, *Slackia* genus, *Sneathia* genus, *Solobacterium* genus, *Staphylococcus* genus, *Stenotrophomonas* genus, *Stomatobaculum* genus, *Streptococcus* genus, *Tannerella* genus, *Treponema* genus, *Ureaplasma* genus, *Veillonella* genus, and *Fretibacterium* genus.

**10.** The method according to claim 8, wherein the target nucleic acids are rRNA genes of at least two species of bacteria selected from the group consisting of Abiotrophia defectiva, Achromobacter xylosoxidans, Acinetobacter baumannii, Acinetobacter calcoaceticus, Acinetobacter johnsonii, Acinetobacter junii, Acinetobacter lwoffii, Actinomyces dentalis, Actinomyces georgiae, Actinomyces gerencseriae, Actinomyces graevenitzii, Actinomyces israelii, Actinomyces johnsonii, Actinomyces meyeri, Actinomyces naeslundii, Actinomyces odontolyticus, Actinomyces oris, Actinomyces turicensis, Actinomyces viscosus, Aggregatibacter actinomycetemcomitans, Aggregatibacter aphrophilus, Alloprevotella rava, Alloprevotella tannerae, Alloscardovia omnicolens, Anaeroglobus geminatus, Atopobium parvulum, Bacillus cereus, Bacteroides fragilis, Bifidobacterium adolescentis, Bifidobacterium bifidum, Bifidobacterium breve, Bifidobacterium dentium, Bifidobacterium longum, Bordetella pertussis, Brucella abortus, Burkholderia cepacia, Campylobacter concisus, Campylobacter gracilis, Campylobacter rectus, Campylobacter showae, Capnocytophaga gingivalis, Capnocytophaga ochracea, Capnocytophaga sputigena, Cardiobacterium hominis, Catonella morbi, Chlamydia psittaci, Chlamydia trachomatis, Chlamydia pneumoniae, Chryseobacterium indologenes, Citrobacter freundii, Clostridium perfringens, Collinsella aerofaciens, Corynebacterium diphtheriae, Corynebacterium durum, Corynebacterium matruchotii, Corynebacterium pseudodiphtheriticum, Corynebacterium pseudotuberculosis, Corynebacterium renale, Corynebacterium striatum, Corynebacterium xerosis, Coxiella burnetii, Cryptobacterium curtum, Curvibacter delicatus, Dialister invisus, Dialister micraerophilus, Dialister pneumosintes, Eggerthella lenta, Eikenella corrodens, Elizabethkingia meningoseptica, Enterobacter aerogenes, Enterobacter cloacae, Enterococcus avium, Enterococcus faecalis, Enterococcus faecium, Escherichia coli, Eubacterium brachy, Eubacterium infirmum, Eubacterium limosum, Eubacterium minutum, Eubacterium nodatum, Eubacterium saphenum, Eubacterium sulci, Filifactor alocis, Fretibacterium fastidiosum, Fusobacterium necrophorum, Fusobacterium nucleatum, Fusobacterium nucleatum subsp. animalis, Fusobacterium nucleatum subsp. nucleatum, Fusobacterium nucleatum subsp. polymorphum, Fusobacterium nucleatum subsp. vincentii, Fusobacterium periodonticum, Fusobacterium simiae, Gemella haemolysans, Gemella morbillorum, Gemella sanguinis, Granulicatella adiacens, Granulicatella balaenopterae, Granulicatella elegans, Haemophilus ducreyi, Haemophilus haemolyticus, Haemophilus influenzae, Haemophilus parainfluenzae, Helicobacter pylori, Kingella denitrificans, Kingella kingae, Kingella oralis, Klebsiella oxytoca, Klebsiella pneumoniae, Lachnoanaerobaculum orale, Lachnoanaerobaculum saburreum, Lactobacillus acidophilus, Lactobacillus brevis, Lactobacillus buchneri, Lactobacillus casei, Lactobacillus crispatus, Lactobacillus delbrueckii, Lactobacillus fermentum, Lactobacillus gasseri, Lactobacillus paracasei, Lactobacillus plantarum, Lactobacillus reuteri, Lactobacillus rhamnosus, Lactobacillus salivarius, Lautropia mirabilis, Legionella pneumophila, Leptotrichia buccalis, Leptotrichia sp. OT 215, Leptotrichia sp. OT 417, Leptotrichia sp. OT 462, Listeria monocytogenes, Megasphaera micronuciformis, Micrococcus luteus, Mogibacterium diversum, Mogibacterium neglectum, Mogibacterium pumilum, Mogibacterium timidum, Mogibacterium vescum, Moraxella catarrhalis, Moraxella lacunata, Morganella morganii, Mycobacterium abscessus, Mycobacterium avium, Mycobacterium chelonae, Mycobacterium fortuitum, Mycobacterium gordonae, Mycobacterium haemophilum, Mycobacterium intracellulare, Mycobacterium kansasii, Mycobacterium marinum, Mycobacterium szulgai, Mycobacterium tuberculosis, Mycobacterium xenopi, Mycoplasma genitalium, Mycoplasma hominis, Mycoplasma orale, Mycoplasma pneumoniae, Mycoplasma salivarium, Neisseria elongata, Neisseria flava, Neisseria flavescens, Neisseria gonorrhoeae, Neisseria meningitidis, Neisseria mucosa, Neisseria sicca, Neisseria subflava, Nocardia asteroides, Nocardia brasilliensis, Nocardia farcinica, Nocardia nova, Olsenella uli, Oribacterium asaccharolyticum, Oribacterium parvum, Parascardovia denticolens, Parvimonas micra, Peptostreptococcus stomatis, Porphyromonas catoniae, Porphyromonas endodontalis, Porphyromonas gingivalis, Porphyromonas pasteri, Prevotella dentalis, Prevotella denticola, Prevotella histicola, Prevotella intermedia, Prevotella loescheii, Prevotella melaninogenica, Prevotella nigrescens, Prevotella oralis, Prevotella oris, Prevotella pallens, Prevotella salivae, Prevotella shahii, Prevotella veroralis, Propionibacterium acnes (Cutibacterium acnes), Propionibacterium granulosum (Cutibacterium granulosum), Propionibacterium propionicus (Pseudopropionibacterium propionicum), Proteus mirabilis, Proteus vulgaris, Providencia stuartii, Pseudomonas aeruginosa, Pseudomonas fluorescens, Pseudomonas putida, Pseudoramibacter alactolyticus, Ralstonia pickettii, Rothia aeria, Rothia dentocariosa, Rothia mucilaginosa, Scardovia inopinata, Schlegelella aquatica, Sebaldella termitidis, Selenomonas flueggei, Selenomonas noxia, Selenomonas sp. OT 478, Selenomonas sputigena, Serratia marcescens, Simonsiella muelleri, Slackia exigua, Solobacterium moorei, SRI sp. OT 345, Staphylococcus aureus, Staphylococcus epidermidis, Staphylococcus saccharolyticus, Stenotrophomonas maltophilia, Streptococcus agalactiae, Streptococcus anginosus, Streptococcus australis, Streptococcus constellatus, Streptococcus cristatus, Streptococcus dentisani, Streptococcus gordonii, Streptococcus infantis, Streptococcus intermedius, Streptococcus milleri, Streptococcus mitis, Streptococcus mutans, Streptococcus oralis, Streptococcus parasanguinis, Streptococcus pneumoniae, Streptococcus pyogenes, Streptococcus salivarius, Streptococcus sanguinis, Streptococcus sobrinus, Streptococcus tigurinus, Streptococcus vestibularis, Tannerella forsythia, Treponema denticola, Treponema lecithinolyticum, Treponema medium, Treponema pallidum, Treponema socranskii, Treponema vincentii, Ureaplasma urealyticum, Veillonella atypica, Veillonella dispar, *Veillonella parvula,* and *Veillonella rogosae.*

**11.** The method according to any one of claims 1 to 10, wherein Step (c) comprises the steps of:

(c1) creating a calibration curve of relationships, using a part or all of the target nucleic acids and the specific nucleic acid group and the control nucleic acid, between amounts before amplification and indicators of amounts after amplification; and
(c2) calculating a total amount of all target nucleic acids and the specific nucleic acid group in the sample by applying an indicator of a total amount of the amplified all target nucleic acids and the specific nucleic acid group from the sample and an indicator of an amount of the amplified control nucleic acid to the calibration curve created in Step (c1).

**12.** The method according to claim 11, wherein the nucleic acid used in Step (c1) is a part of the target nucleic acids and the specific nucleic acid group.

**13.** The method according to claim 11 or 12, wherein the calibration curve represents a relationship between
a ratio of an indicator of a total amount of a part or all of the target nucleic acids and the specific nucleic acid group to an indicator of an amount of the control nucleic acid after amplification and
a ratio of a total amount of a part or all of the target nucleic acids and the specific nucleic acid group to an amount of the control nucleic acid before amplification.

**14.** The method according to any one of claims 1 to 13, wherein, in Steps (c) and (d), an indicator of an amount of each amplified target nucleic acid, an indicator of a total amount of amplified all target nucleic acids and the specific nucleic acid group, and an indicator of an amount of the amplified control nucleic acid are signal intensities obtained by hybridization with the following probes (x) to (z), respectively, loaded on a DNA chip:

(x) a probe hybridizable respectively with amplified products of two or more types of target nucleic acids;
(y) a probe hybridizable in common with both amplified products of all target nucleic acids and the specific nucleic acid group in the sample; and
(z) a probe hybridizable with amplified products of the control nucleic acid.

**15.** The method according to claim 14, wherein Step (d) comprises the steps of:

(d1) hybridizing two or more types of respective target nucleic acids (each target nucleic acid) with the DNA chip and calculating a relational expression on signal intensities between the probe (x) and the probe (y) hybridizable with each target nucleic acid;
(d2) converting the signal intensity of the probe (x) obtained by hybridizing products amplified from the sample with the DNA chip to a value equivalent to the signal intensity of the probe (y) hybridizable with each target nucleic acid using the relational expression calculated in Step (d1);
(d3) calculating a ratio of the value equivalent to the signal intensity of the probe (y) calculated in Step (d2) to the signal intensity of the probe (y) obtained by hybridizing products amplified from the sample with the DNA chip; and
(d4) calculating an absolute amount of each target nucleic acid in the sample by multiplying the total amount of all nucleic acids and the specific nucleic acid group in the sample calculated in Step (c) by the occupancy calculated in Step (d3).

**16.** The method according to claim 14 or 15, wherein Step (b) comprises the step of enriching nucleic acids after amplification for a single chain hybridizable with probes loaded on the DNA chip.

**17.** The method according to claim 16, wherein the step of enriching for a single chain is carried out by asymmetric PCR or $\lambda$ exonuclease treatment.

**18.** The method according to any one of claims 14 to 17, wherein the probe (x) is any of the following sequences:

(I) a sequence selected from the group consisting of the base sequences as set forth in SEQ ID NO: 3 to 191;
(II) a complementary sequence to (I); or
(III) a sequence substantially identical with the sequence (I) or (II).

**19.** The method according to any one of claims 14 to 18, wherein the DNA chip is a fiber-type DNA chip.

**20.** A kit comprising: one or two pairs of primer sets for co-amplifying all target nucleic acids and the specific nucleic acid group in a sample and a control nucleic acid by PCR method; a control nucleic acid; and a DNA chip on which the following probes (x) to (z) are loaded:

(x) a probe hybridizable respectively with amplified products of two or more types of target nucleic acids;
(y) a probe hybridizable in common with both amplified products of all target nucleic acids and the specific nucleic acid group in the sample; and
(z) a probe hybridizable with amplified products of the control nucleic acid.

**21.** The kit according to claim 20, wherein the probe (x) is any of the following sequences:

(I) a sequence selected from the group consisting of the base sequences as set forth in SEQ ID NO: 3 to 191;
(II) a complementary sequence to (I); or
(III) a sequence substantially identical with the sequence (I) or (II).

**22.** A DNA chip on which the following probes (x) to (z)are loaded:

(x) a probe hybridizable respectively with amplified products of two or more types of target nucleic acids;
(y) a probe hybridizable in common with both amplified products of all target nucleic acids and the specific nucleic acid group in the sample; and
(z) a probe hybridizable with amplified products of the control nucleic acid, wherein the probe (x) is any of the following sequences:

(IV) a sequence selected from the group consisting of the base sequences as set forth in SEQ ID NO: 43 to 191;
(V) a complementary sequence to (IV); or
(VI) a sequence substantially identical with the sequence (IV) or (V).

[Figure 1]

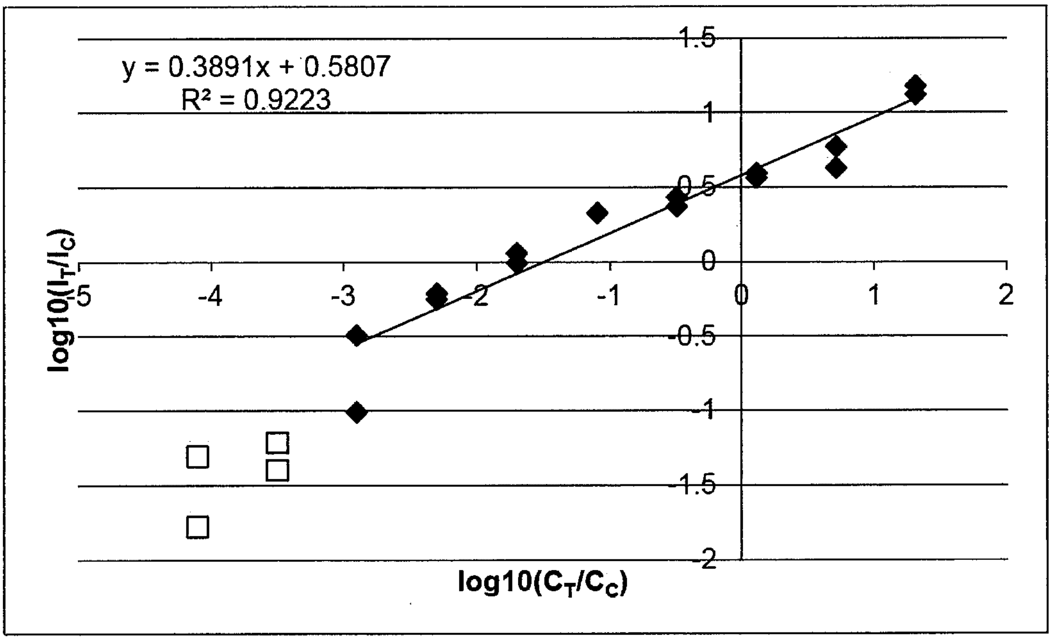


[Figure 2]

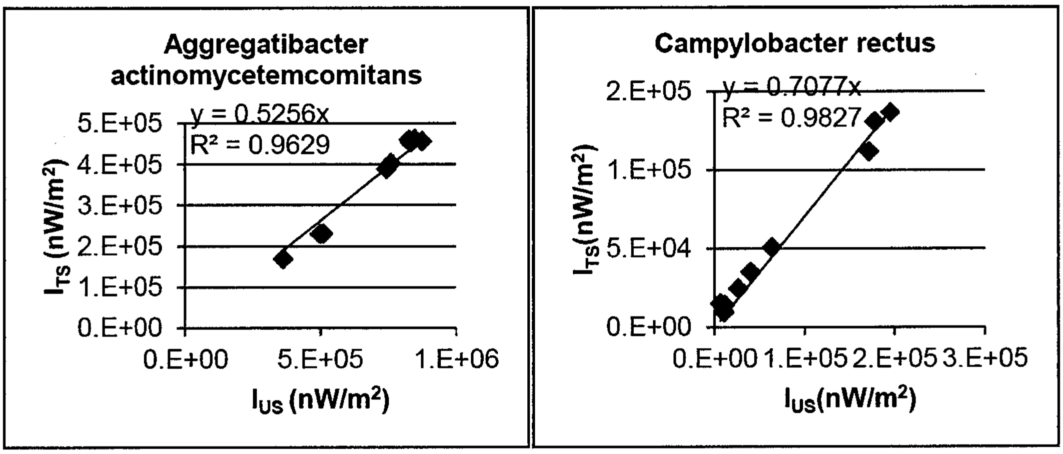

[Figure 3]

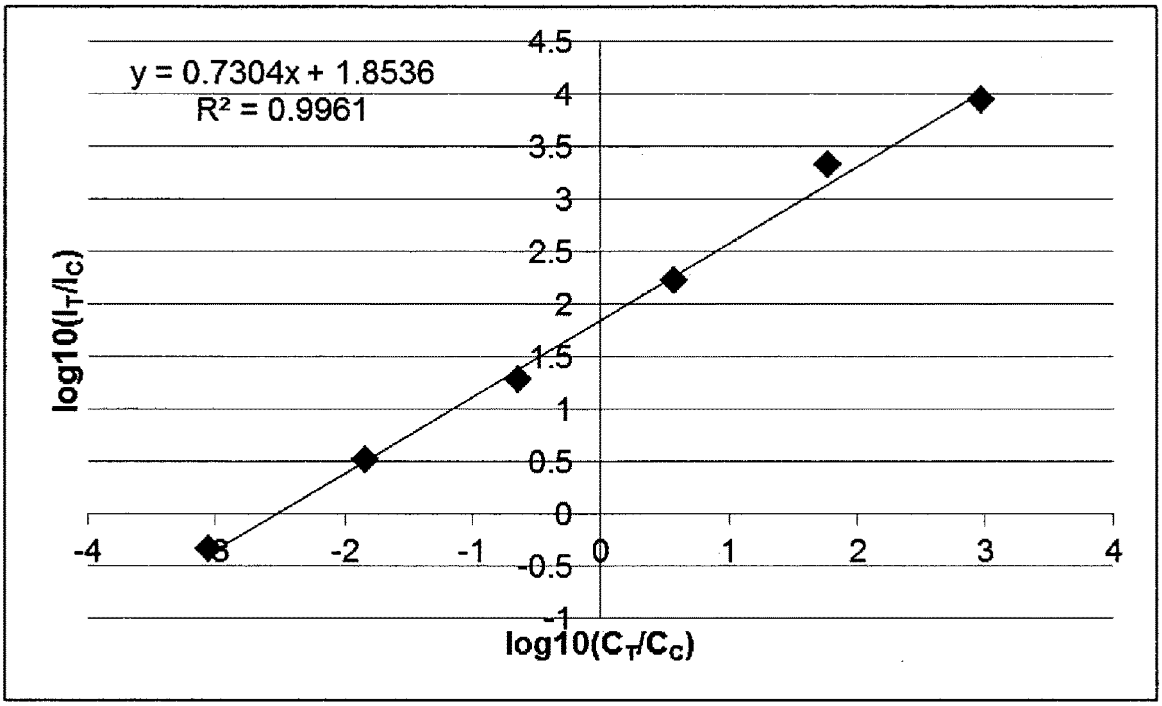
y = 0.7304x + 1.8536
R² = 0.9961
log10(I_T/I_C)
log10(C_T/C_C)
4.5
4
3.5
3
2.5
2
1.5
1
0.5
0
-0.5
-1
-4
-3
-2
-1
0
1
2
3
4

[Figure 4A]

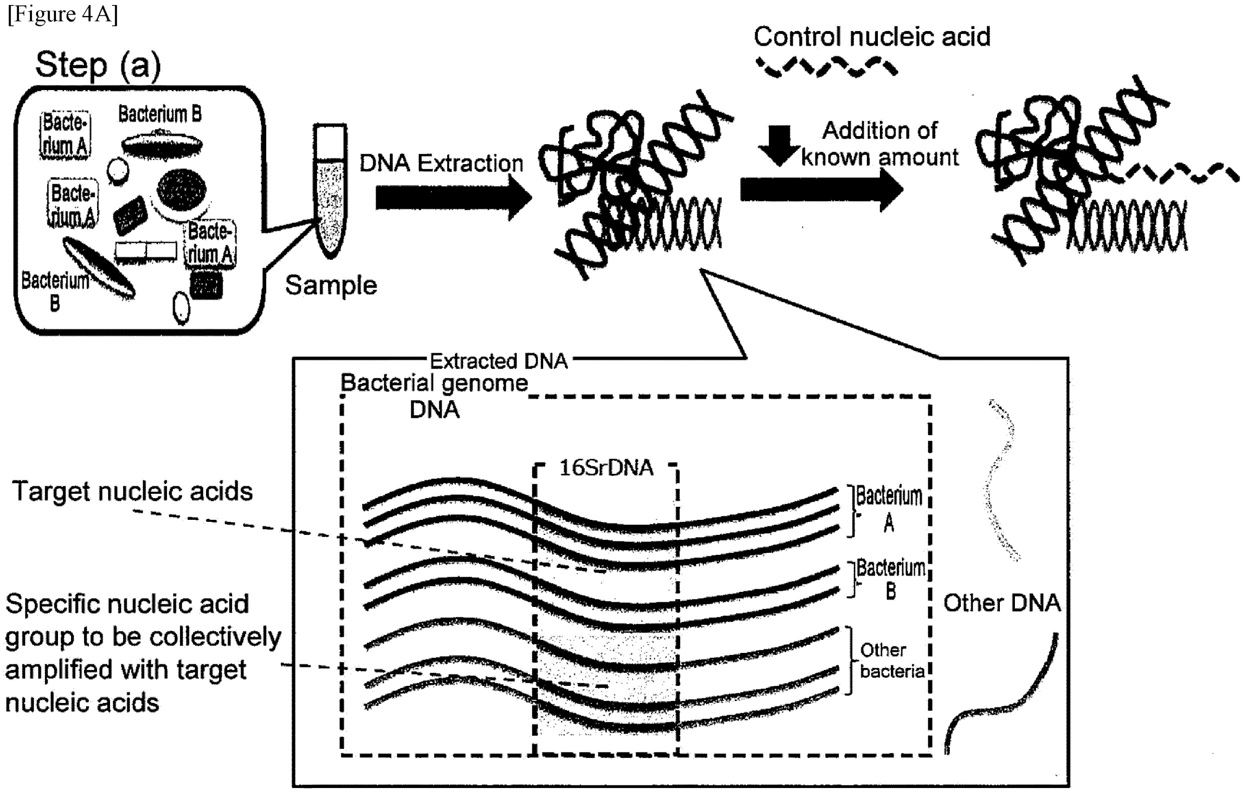
Step (a)
Bacterium A
Bacterium B
Sample
DNA Extraction
Control nucleic acid
Addition of known amount
Extracted DNA
Bacterial genome DNA
16SrDNA
Bacterium A
Bacterium B
Other bacteria
Other DNA
Target nucleic acids
Specific nucleic acid group to be collectively amplified with target nucleic acids

[Figure 4B]

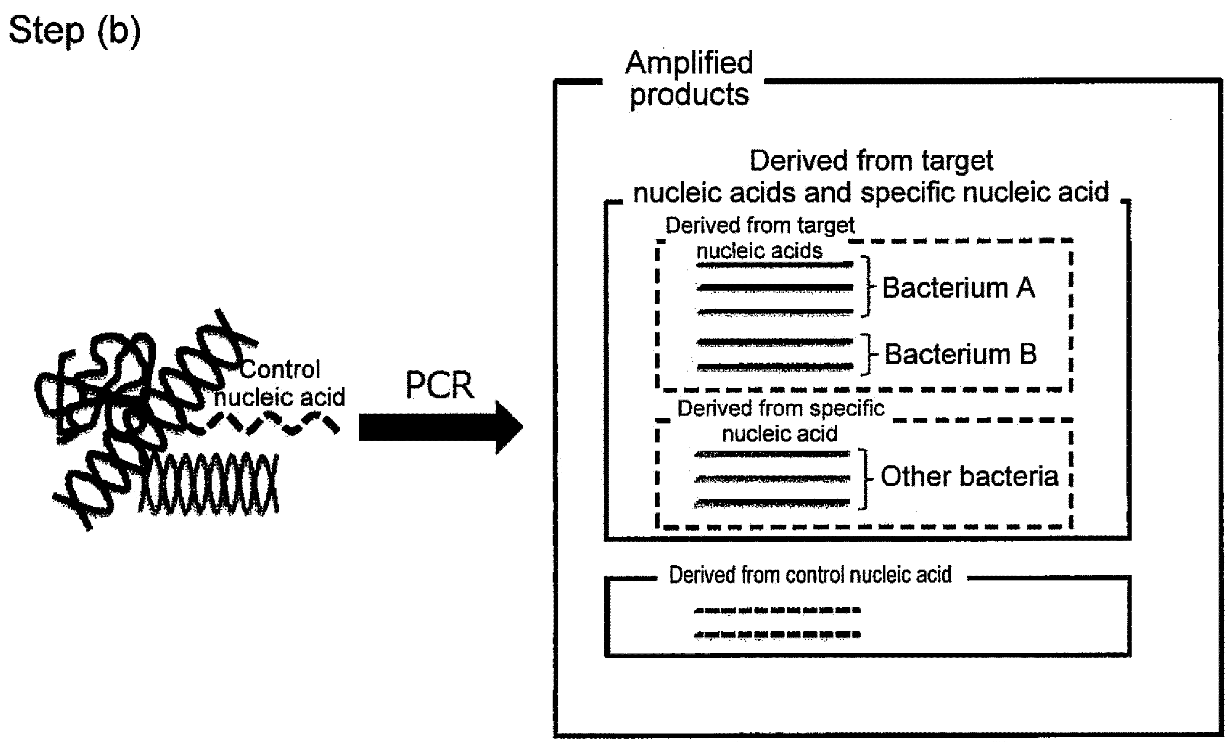
Step (b)
Control
nucleic acid
PCR
Amplified
products
Derived from target
nucleic acids and specific nucleic acid
Derived from target
nucleic acids
Bacterium A
Bacterium B
Derived from specific
nucleic acid
Other bacteria
Derived from control nucleic acid

[Figure 4C]

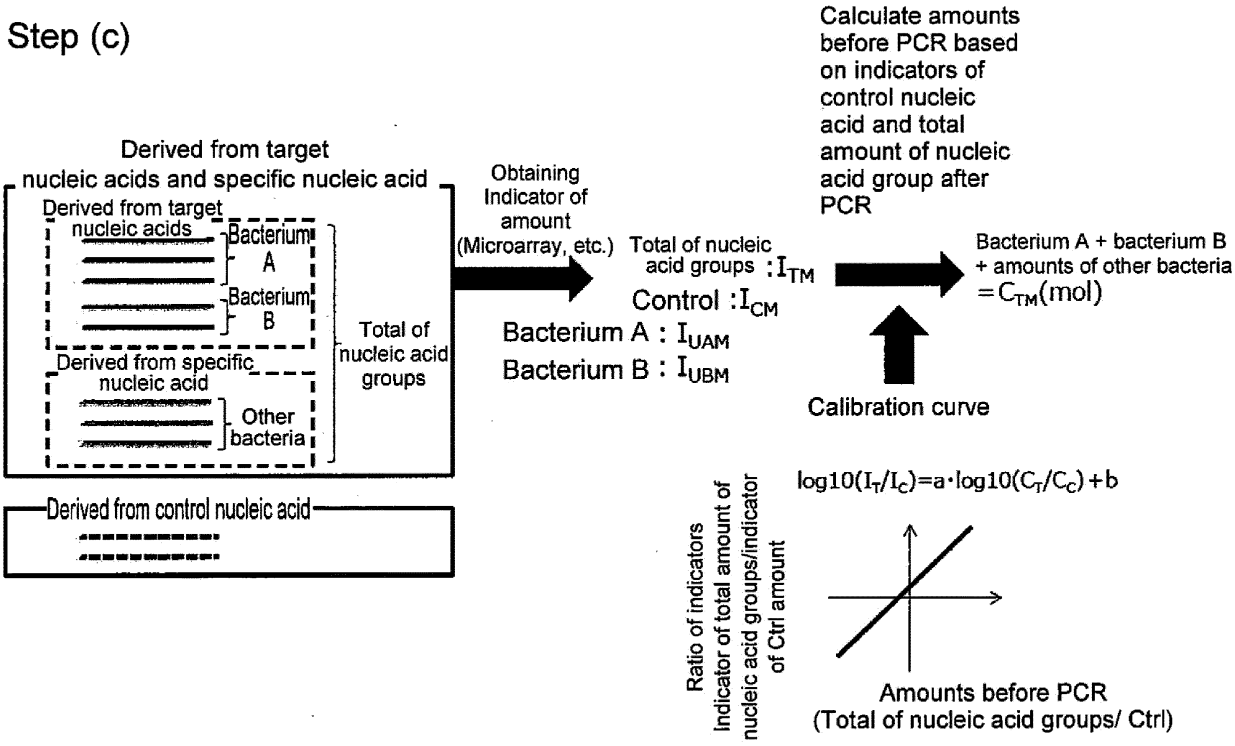
Step (c)
Derived from target nucleic acids and specific nucleic acid
Derived from target nucleic acids
Bacterium A
Bacterium B
Derived from specific nucleic acid
Other bacteria
Total of nucleic acid groups
Derived from control nucleic acid
Obtaining Indicator of amount (Microarray, etc.)
Total of nucleic acid groups : $I_{TM}$
Control : $I_{CM}$
Bacterium A : $I_{UAM}$
Bacterium B : $I_{UBM}$
Calculate amounts before PCR based on indicators of control nucleic acid and total amount of nucleic acid group after PCR
Bacterium A + bacterium B + amounts of other bacteria = $C_{TM}$(mol)
Calibration curve
$\log10(I_T/I_C)=a\cdot\log10(C_T/C_C)+b$
Ratio of indicators Indicator of total amount of nucleic acid groups/indicator of Ctrl amount
Amounts before PCR (Total of nucleic acid groups/ Ctrl)

[Figure 4D]

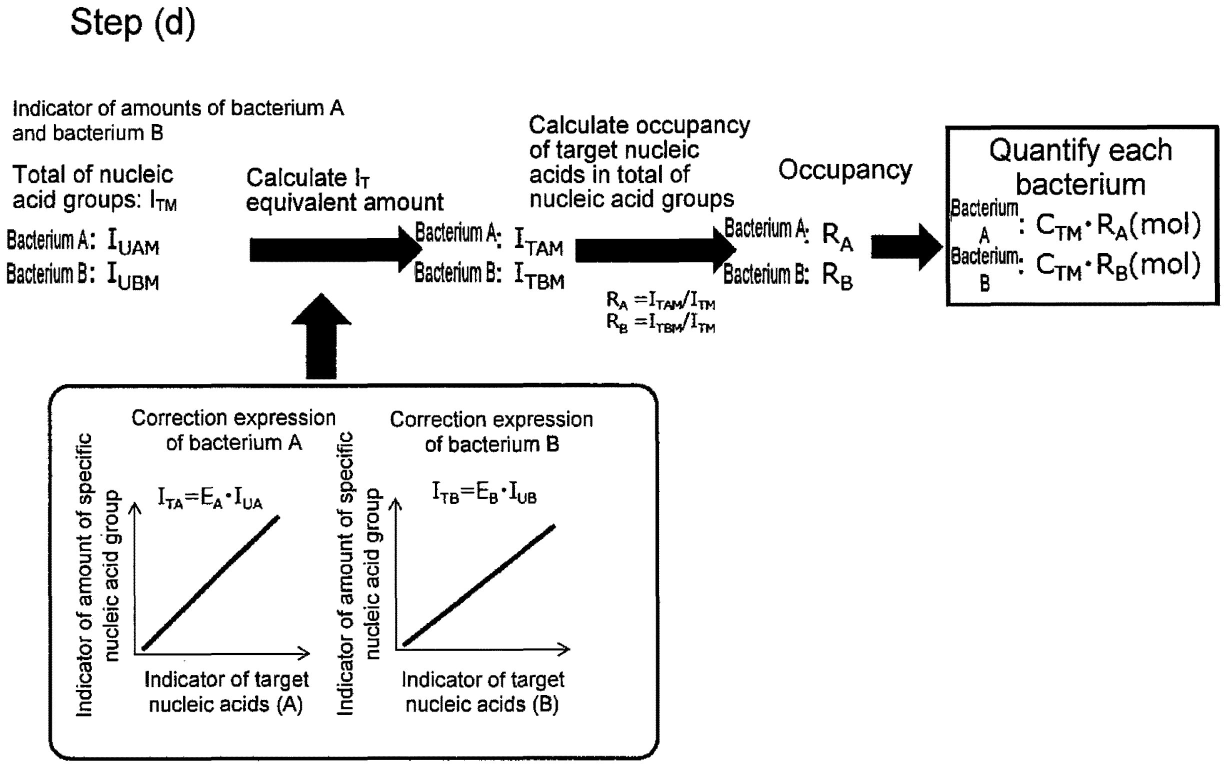
Step (d)
Indicator of amounts of bacterium A and bacterium B
Total of nucleic acid groups: $I_{TM}$
Bacterium A: $I_{UAM}$
Bacterium B: $I_{UBM}$
Calculate $I_T$ equivalent amount
Bacterium A: $I_{TAM}$
Bacterium B: $I_{TBM}$
Calculate occupancy of target nucleic acids in total of nucleic acid groups
$R_A = I_{TAM}/I_{TM}$
$R_B = I_{TBM}/I_{TM}$
Occupancy
Bacterium A: $R_A$
Bacterium B: $R_B$
Quantify each bacterium
Bacterium A: $C_{TM} \cdot R_A$(mol)
Bacterium B: $C_{TM} \cdot R_B$(mol)
Correction expression of bacterium A
$I_{TA}=E_A \cdot I_{UA}$
Indicator of amount of specific nucleic acid group
Indicator of target nucleic acids (A)
Correction expression of bacterium B
$I_{TB}=E_B \cdot I_{UB}$
Indicator of amount of specific nucleic acid group
Indicator of target nucleic acids (B)

[Figure 5]

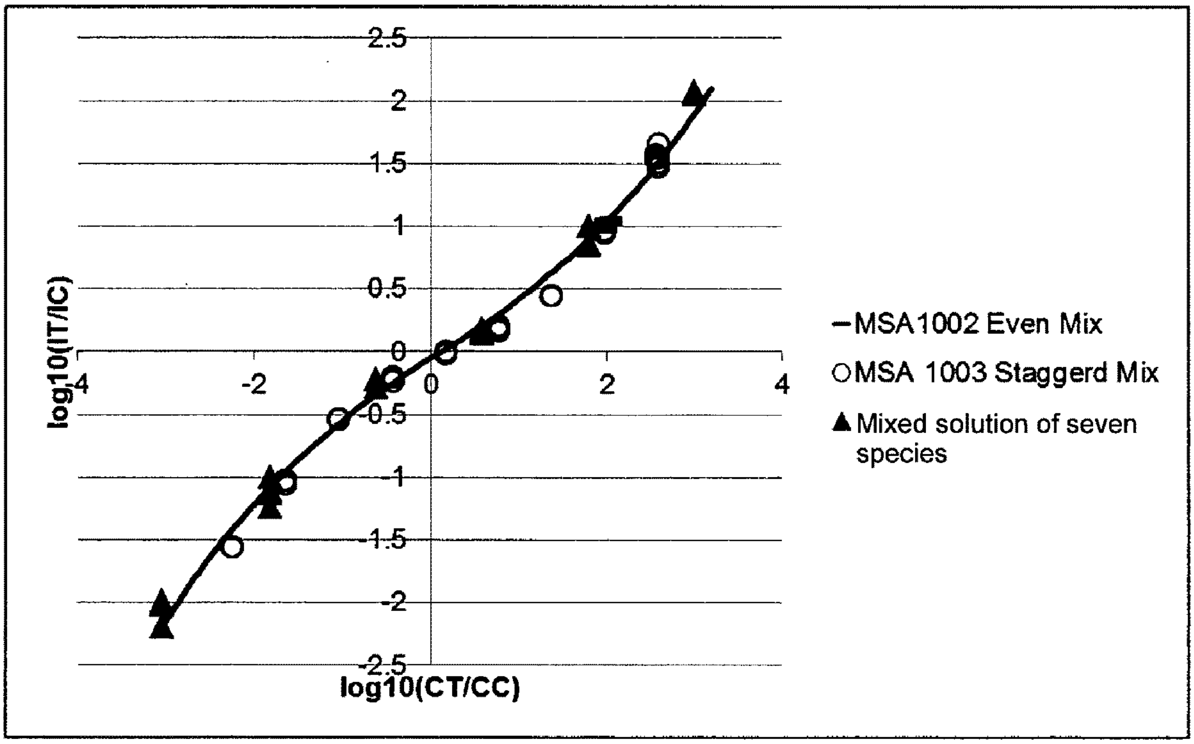
2.5
2
1.5
1
0.5
0
-0.5
-1
-1.5
-2
-2.5
-4
-2
0
2
4
log10(IT/IC)
log10(CT/CC)
—MSA1002 Even Mix
○MSA 1003 Staggerd Mix
▲Mixed solution of seven species

[Figure 6]

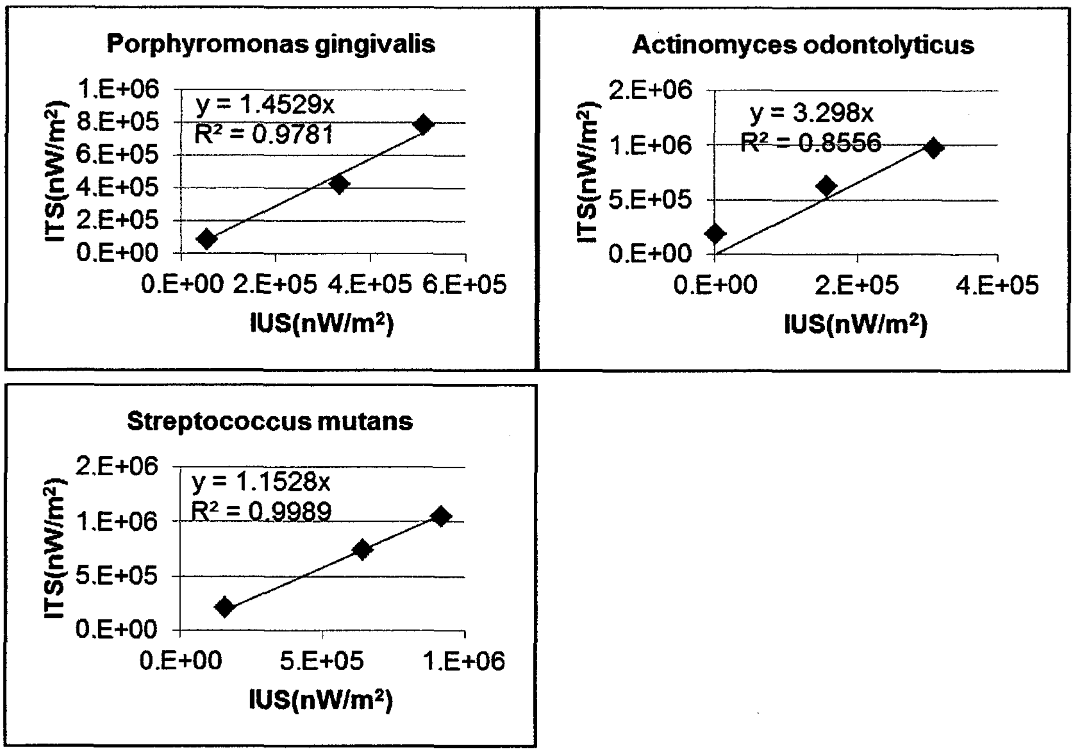


[Figure 7]

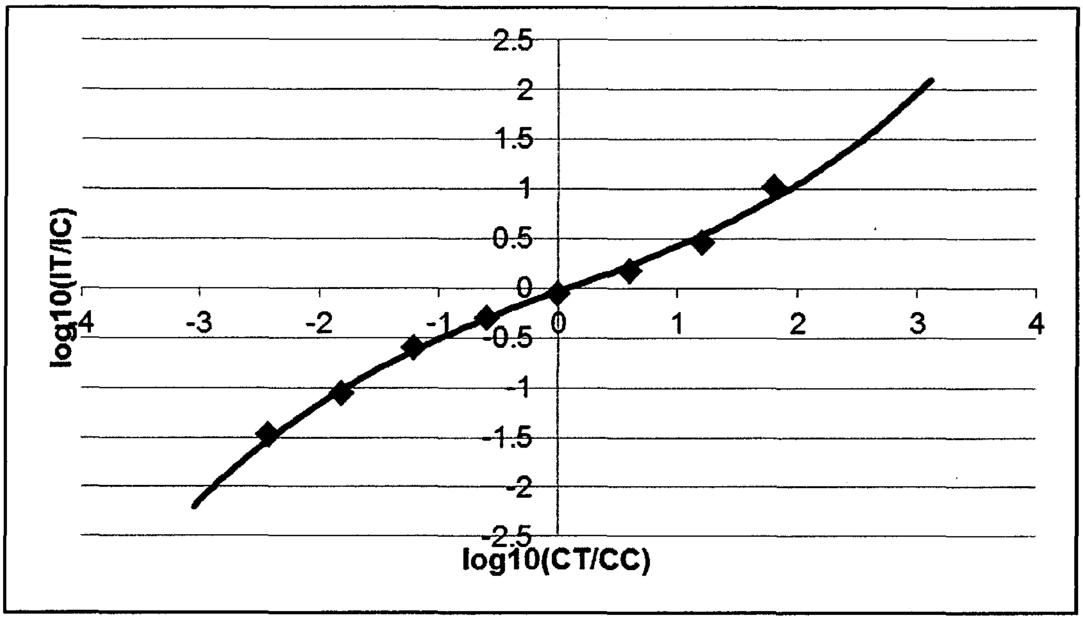

[Figure 8]

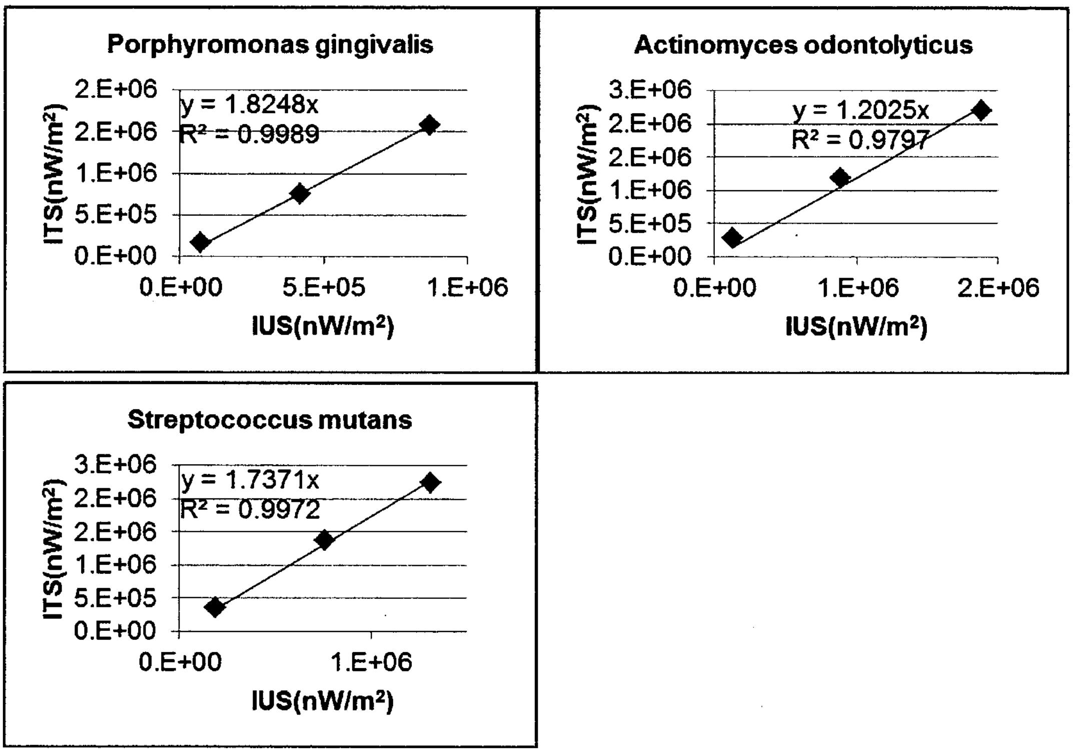


[Figure 9]

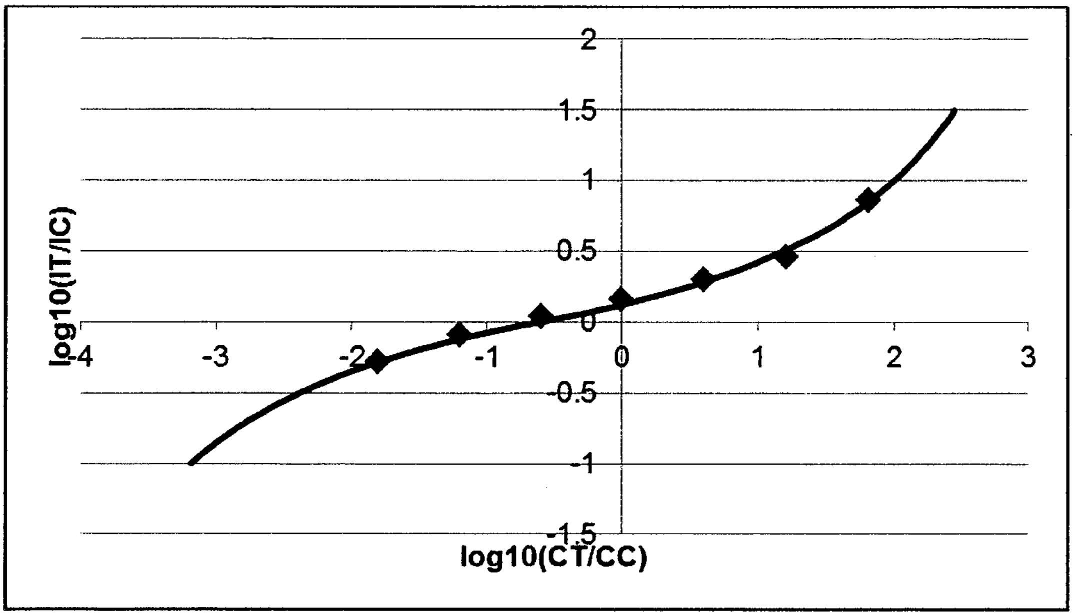

[Figure 10]

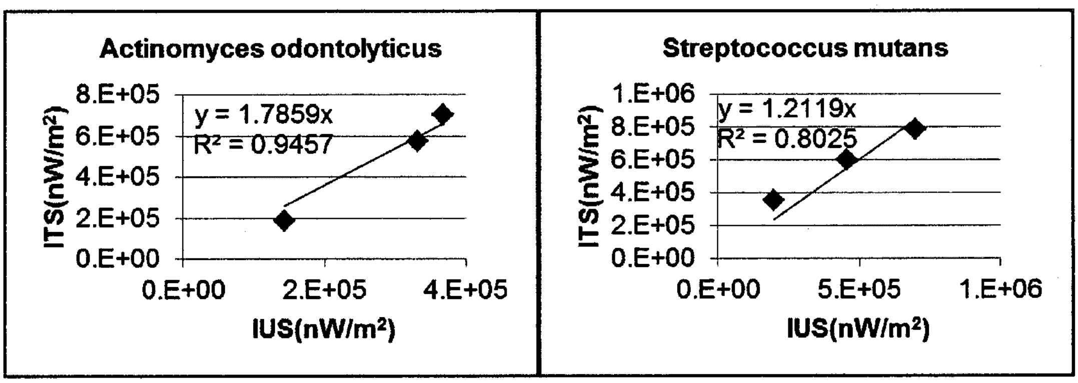
Actinomyces odontolyticus
y = 1.7859x
R² = 0.9457
ITS(nW/m²)
8.E+05
6.E+05
4.E+05
2.E+05
0.E+00
0.E+00
2.E+05
4.E+05
IUS(nW/m²)
Streptococcus mutans
y = 1.2119x
R² = 0.8025
ITS(nW/m²)
1.E+06
8.E+05
6.E+05
4.E+05
2.E+05
0.E+00
0.E+00
5.E+05
1.E+06
IUS(nW/m²)

## INTERNATIONAL SEARCH REPORT

International application No.
PCT/JP2019/020610

A. CLASSIFICATION OF SUBJECT MATTER
Int.Cl. C12Q1/6851(2018.01)i, C12Q1/6813(2018.01)i, C12N15/09(2006.01)n, C12Q1/689(2018.01)n

According to International Patent Classification (IPC) or to both national classification and IPC

B. FIELDS SEARCHED
Minimum documentation searched (classification system followed by classification symbols)
Int.Cl. C12Q1/6851, C12Q1/6813, C12N15/09, C12Q1/689

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 2015-231362 A (MITSUBISHI RAYON CO., LTD.) 24 December 2015, claims, examples, paragraphs [0044]-[0058]<br>(Family: none) | 1-22 |
| A | JP 2013-535951 A (THE TRANSLATIONAL GENOMICS RESEARCH INSTITUTE) 19 September 2013, paragraph [0051]<br>& US 2011/0312504 A1, paragraph [0066] & WO 2011/116313 A1 & EP 2547782 A | 1-22 |
| A | JP 2017-23093 A (MITSUBISHI RAYON CO., LTD.) 02 February 2017, claims, examples<br>(Family: none) | 1-22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13.08.2019 | 20.08.2019 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2019/020610

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-204813 A (NATIONAL INSTITUTE OF ADVANCED INDUSTRIAL SCIENCE AND TECHNOLOGY) 19 November 2015, claims, examples (Family: none) | 1-22 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015204813 A
- JP 2018100475 A
- JP 2019026519 A
- JP 3510882 B
- JP 6299660 B


### Non-patent literature cited in the description


- **TREIMO, J. et al.** *J Appl Microbiol,* 2006, vol. 100, 985-998
- *Oral Dis.,* 2015, vol. 21, 748-54
- *Microbiome,* 2018, vol. 6 (42, https://doi.org/10.1186/s40168-018-0426-3
- *Science,* 1995, vol. 270, 467-470
- *Science,* 1991, vol. 251, 767-773
- Molecular Cloning, A Laboratory Manual. Cold Spring Harbor Laboratory Press, 2012